(19) Europäisches Patentamt
European Patent Office
Office européen des brevets

(11) EP 2 418 196 A1

(12) EUROPEAN PATENT APPLICATION

(43) Date of publication:
**15.02.2012 Bulletin 2012/07**

(21) Application number: **10171196.8**

(22) Date of filing: **29.07.2010**

(51) Int Cl.:
***C07C 237/08*** (2006.01) ***C07D 209/04*** (2006.01)
***C07D 277/04*** (2006.01) ***C07D 401/02*** (2006.01)
***A61K 31/16*** (2006.01) ***A61K 31/505*** (2006.01)
***A61K 31/404*** (2006.01) ***A61K 31/4164*** (2006.01)
***A61P 29/00*** (2006.01) ***A61P 37/00*** (2006.01)
***A61P 25/00*** (2006.01) ***A61P 35/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **IMTM GmbH**
**39120 Magdeburg (DE)**

(72) Inventors:
- **Ansorge, Siegfried, Prof. Dr.**
  **39291 Hohenwarthe (DE)**
- **Bank, Ute, Dr.**
  **39418 Staßfurt (DE)**
- **Heimburg, Antje, Dr.**
  **39110 Magdeburg (DE)**
- **Julius, Heiko**
  **39104 Magdeburg (DE)**
- **Nordhoff, Carsten, Dr.**
  **39118 Magdeburg (DE)**
- **Täger, Michael, Dr.**
  **39326 Heinrichsberg (DE)**

(74) Representative: **Koepe, Gerd L.**
**Koepe & Partner**
**Robert-Koch-Strasse 1**
**80538 München (DE)**

# (54) Dual alanyl-aminopeptidase and dipeptidyl-peptidase IV inhibitors

(57) The invention relates to compounds of the general formula (I)

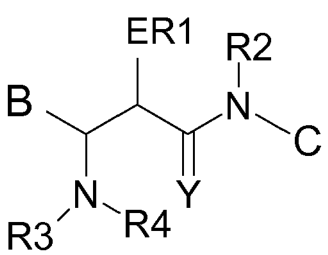

(I)

or the acid addition salts thereof with organic and/or inorganic acids; as well as to the use of the compounds of the general formula (1) in the medical field, specifically for use in the suppression of DNA synthesis and inflammatory cytokine production as well as in the stimulation of anti-inflammatory cytokine production in vitro and in vivo. The invention also relates to pharmaceutically effective compositions comprising at least one compound of the general formula (I), optionally together with one or more pharmaceutically acceptable carrier(s), auxiliary substance(s) and/or adjuvant(s) or cosmetically effective compositions comprising at least one compound of the general formula (I), optionally in combination with one or more cosmetically acceptable carrier(s), auxiliary substance(s) and/or adjuvant(s).

EP 2 418 196 A1

## Description

The invention relates to novel chemical compounds. Moreover, the invention relates to said novel chemical compopunds which are capable of concertedly inhibiting the ectoenzymes dipeptidyl peptidase IV (DPIV) and alanyl aminopeptidase N (APN) ("dual inhibitors") via a direct interaction with the active site and/or via newly defined, functionally relevant binding sites of the enzymes [European Patent Application No. 10 156 805.3 filed on 17 March 2010]. The novel compounds also have an effect on ectopeptidases possessing an enzymatic effect analogous to dipeptidyl peptidase IV (DPIV) ("DPIV-analogous enzymatic effect") and/or have an effect on ectopeptidases having an enzymatic effect analogous to alanyl aminopeptidase N (APN) ("APN-analogous enzymatic effect").

Furthermore, the invention relates to processes of preparing the novel dual inhibitors of DPIV and APN.

The invention also relates to the afore-mentioned novel chemical compounds for a use in the medical field.

Moreover, the invention relates to the afore-mentioned novel chemical compounds for a use for a prophylaxis and a therapy of diseases showing an excessive immune response and having an inflammatory genesis, of neuronal diseases and of diseases causing cerebral damage, of tumor diseases, of skin diseases, of diabetes type I and of SARS.

The enzyme dipeptidyl peptidase IV (DPIV, CD26, EC 3.4.14.5) is a serine protease existing ubiquituously and catalyzing the hydrolysis of peptides specifically after proline and - to a lesser extent - after alanine or - with restrictions - after further amino acids like serine, threonine, valine and glycine at the second position of the N-terminus. Enzymes belonging to the gene family of enzymes having DPIV-analogous enzymatic effect are - *inter alia* -DPII, DP 8, DP 9 and FAP/seprase [T. Chen et al.: Adv. Exp. Med. Biol. 524, 79, 2003]. A substrate specifity analogous to DPIV was also found for attractin (mahagony protein) [J. S. Duke-Cohan et al.: J. Immunol. 156, 1714, 1996]. Said enzyme is also inhibited by DPIV inhibitors.

Dipeptidyl peptidase IV occurs in two forms, as soluble form in blood and other body fluids and as membrane-bound form on cells and in tissues. The membrane-bound form represents more than 99 percent of the total DPIV. The soluble form must be considered to be an artifact due to a proteolytical shedding of the membranous enzyme.

The same holds true also for aminopeptidase N.

The molecular mechanisms as well as the functions of the soluble and membrane-bound DPIV and APN are different. This is due to a preferred access of substrates to the active site at the membrane-bound DPIV via central pores which are localized opposite to the cell membrane.

An analysis of the central pore of DPIV surprisingly shows that membrane DPIV is strongly regulated by a binding site within the central pore approximately 2.17 nm from the active site of this enzyme. Suitable ligands to this site are blocking the access of substrates to the active site and mediate the cellular effects of DPIV, such as a cell cycle arrest at G1/S phase and cytokine production. Consequently, occupation of the central pore binding site, but not the direct inhibition of DPIV via the active site, is a prerequisite for inhibiting the cellular function of DPIV [European patent application No. 10 156 805.3].

A similar situation was found for the APN examining the model of the crystal structure of aminopeptidase N from *Escherichia coli* [K. Ito et al., 2006, J. Biol. Chem. 281, 33664-33676].

Belonging to the group of alanyl aminopeptidases (also existing ubiquituously) is the aminopeptidase N (APN, CD13, EC 3.4.11.2) predominantly appearing as a membrane protein of the type II, and is the cytosolic soluble alanyl aminopeptidase (EC 3.4.11.14, puromycine-sensitive aminopeptidase, aminopeptidase PS, encephaline-degrading aminopeptidase). Alanyl aminopeptidases (including the afore-mentioned two aminopeptidases) act in dependency of a metal, for example in dependency of zinc, and catalyze the hydrolysis of peptide bonds after the N-terminal amino acids of oligopeptides, in the case of APN with a preference of alanine at the N-terminus [A. J. Barrett et al.: Handbook of Proteolytic Enzymes, Academic Press 1998]. All inhibitors of aminopeptidase N also inhibit the cytosolic alanyl aminopeptidase, while specific inhibitors of the cytosolic aminopeptidase exist [M. Komodo et al.: Bioorg. and Med. Chem. 9, 121, 2001].

Equally to DPIV, two access paths to the APN molecule were identified. In addition to the active site, a binding site for substrates and inhibitors within a central pore was recognized by docking approaches, which additional site is located opposite to the N-terminal part of APN adjacent to the membrane. [H. B. Rasmussen et al., 2003, Nat. Struct. Biol. 10, 3-5; Ito et al., 2006, J. Biol. Chem. 281, 33664 - 33676; cf. European Patent Application No. 10 156 805.3 filed on 17 March 2010]. Binding APN inhibitors to this central pore binding site, 1.51 nm from the access site, sterically blocks, as in the case of DPIV, the access of substrates to the active site of APN. Consequently, the functional property of APN is also regulated via these central pore binding sites of this peptidase.

The relative positions of the two access paths of APN and DPIV are shown schematically in Figure 1.

For both groups of enzymes, DPIV as well as APN, important biological functions were proved in different cell systems. This is true - *inter alia* - for the immune system [S. Ansorge et al., 2009, Clin. Chem. Lab. Med. 47, 253-261; U. Lendeckel et al.: Intern. J. Mol. Med. 4, 17, 1999; T. Kähne et al.: Intern. J. Mol. Med. 4, 3, 1999; I. De Meester et al.: Advanc. Exp. Med. Biol. 524, 3, 2002; International Patent Application No. WO 01/89,569; International Patent Application No. WO 02/053,170; International Patent Application No. PCT/EP 03/07,199]; the neuronal system [International Patent Application No. WO 02/053,169 and German Patent Application No. 103 37 074.9]; the fibroblasts [German Patent Application No. 103 30 842.3]; the keratinocytes [International Patent Application No. WO 02/053,170]; the sebaceous gland cells/sebocytes [International Patent Application No. PCT/EP 03/02,356]; tumors as well as for virus-caused infections as, for example infections caused by corona viruses [D. P. Kontoyiannis et al.: Lancet 361, 1558, 2003].

The capability of soluble DPIV in blood of specifically inactivating the incretory hormones GIP and GLP led to the development of a new therapeutic concept for treating glucose metabolic disorders [D. M. Evans: Drugs 5, 577, 2002].

For both groups of enzymes, different inhibitors are known [reviews are found in: D. M. Evans: Drugs 5, 577, 2002; and in: M.-C. Fournie-Zaluski and B. P. Roques: in J. Langner and S. Ansorge: Ectopeptidases, Kluwer Academic/ Plenum Publishers, p. 51, 2002]. Most of them are based on the action on soluble peptidases and not on membrane-bound cellular DPIV and APN.

The isolated inhibition of the alanyl aminopeptidases and of the dipeptidyl peptidase IV as well as the inhibition of enzymes having an analogous substrate specificity, in particular the combined inhibition of enzymes of both groups of enzymes, results into a strong inhibition of the DNA synthesis in immune cells and in other cells, e.g. skin cells and tumor cells, and, hence, into a strong inhibition of the cell proliferation as well as into a change of the cytokine production, particularly into an induction of the immunosuppressive cytokine TGF-$\beta$1 [International Patent Application No. WO 01/89,569; International Patent Application No. WO 02/053,170] as well as into an inhibition of the generation and release of inflammatory cytokines of the type TH1, e.g. interleukine-2 (IL-2), TH2, e. g. interleukine-4 (IL-4) and TH17, e. g. IL-17 [International Patent Application No. WO 02/053,170 and German Patent Application No. 101 02 392.8;S. Ansorge et al., 2009, Clin. Chem. Lab. Med. 47, 253-261].

Moreover, DPIV is capable to inactivate the vasoactive intestinal peptide (VIP), the pituitary adenylate cyclase-activating polypeptide (PACAP) as well as the neuropeptide Y (NPY) which have immunosuppressive and neuroprotective or neurogenetic properties, respectively. VIP has also been shown to activate T regulatory cells (Treg) *in vitro and in vivo* (P. Anderson and E. Gonzalez-Rey, 2010, Mol. Cell. Biol. 30, 2537-2551; E. Gonzalez-Rey et al., 2007, Ann. Rheum. Dis. 66, 70-76; J. Holler et al., 2008, J. Immunol. 181, 6909-6912; J.-R. Zhou et al., 2008, Neurosci. Bull. 24, 155-159). Consequently, inhibition of inactivation of these cytokines protects them from loss of their immunosuppressive and neuroprotective properties and induces an anti-inflammatory and a neuro-protected status, which are prerequisites in treatment of neurodegenerative diseases such as multiple sclerosis, Parkinson's disease and Alzheimer's disease.

Inhibitors of alanyl aminopeptidase effect a strong induction of TGF-$\beta$1 at regulatory T-cells [International Patent Application No. PCT/EP 03/07,199] and an activation of the immunosuppressive phenotyp of regulatory T-cells [German Patent Application No. 102006703942]. In the neuronal system, a decrease or retardation of acute and chronic cerebral damage processes was proved by an inhibition of both enzyme systems [International Patent Application No. WO 02/053,169 and German Patent Application No. 103 37 074.9]. Moreover, it was proved for fibroblasts [German Patent Application No. 103 30 842.3], keratinocytes [International Patent Application No. WO 02/053,170] and sebocytes [International Patent Application No. PCT/EP 03/02,356] that the combined inhibition of alanyl aminopeptidase N and DPIV effects an inhibition of the cell growth and a change of the cytokine production.

This results into the surprising fact that the alanyl aminopeptidases and the dipeptidyl peptidase IV as well as enzymes having an analogous enzymatic effect perform fundamental central biologic functions in different organs and cell systems. Hence, a combined inhibition of both groups of enzymes, particularly via the central pore binding sites, represents a new effective therapeutic principle for the treatment of various - in most cases - chronic inflammatory diseases and tumor diseases.

In accepted animal models, the applicants could show in the meantime that, in particular, the combined administration of inhibitors of both groups of said peptidases results into an inhibition of the growth of different cell systems and into a suppression of an excessive immune response, of chronic inflammatory processes and of cerebral damages, also *in vivo* [International Patent Application No. WO 01/89,569]. The isolated administration of single known inhibitors results into a diminished effect.

The results reported formerly were obtained predominantly by means of known inhibitors of alanyl aminopeptidase N and dipeptidyl peptidase IV alone, being described in the literature and being - in part - commercially available, but in particular by combinations of inhibitors of enzymes of both groups.

In the document EP-A 1 948 627 (WO-A 2007/057,128), novel, predominantly non-peptidic low molecular weight substances were reported which may be employed as prodrugs and may act under physiological and pathological conditions to effective agents or to a mixture of effective agents, which inhibit alanyl aminopeptidase N and enzymes having an analogous substrate specificity, and inhibit dipeptidyl peptidase IV and enzymes having an analogous substrate specificity as well, in a dual manner. The conversion of the prodrugs is conducted by a reduction of -S-S- or -Se-Se-bridges, preferably by cellular thiols (compounds bearing -SH- groups).

In the European patent application No. 09 169 269.9, filed on 2 September 2009, another group of novel multifunctional peptidase inhibitors for medical use was reported.

It was the object of the present invention to provide novel chemical compounds.

It was another object of the present invention to provide novel chemical compounds suitable as dual inhibitors for the above two groups of peptidases, i. e. (ia) dipeptidyl peptidase IV (DPIV) as well as (ib) peptidases having an enzymatic effect analogous to dipeptidyl peptidase IV (DPIV) ("DPIV-analogous enzymatic effect") and/or (iia) alanyl aminopeptidase N (APN) as well as (iib) peptidases having an enzymatic effect analogous to alanyl aminopeptidase N (APN) ("APN-analogous enzymatic effect").

It was a further object of the invention to provide novel chemical compounds suitable for a use in the medical field.

Moreover, it was an object of the invention to provide novel chemical compounds which are suitable for being used for a prophylaxis and a therapy of diseases showing an excessive immune response and having an inflammatory genesis, of neuronal diseases and of diseases causing cerebral damage, of tumor diseases, of skin diseases, of diabetes of the type I and of SARS.

Surprisingly, it was found that novel substances found by the Applicants in their studies of inhibiting ectopeptidases are capable of specifically inhibiting dipeptidy peptidase IV and alanyl aminopeptidase N and, hence, combine the capability of a concerted ("dual") inhibition of both groups of peptidases in one substance. Of course, the novel substances of the present invention may inhibit solely one of the peptidases, i. e. (ia) dipeptidyl peptidase IV (DPIV) as well as (ib) peptidases having an enzymatic effect analogous to dipeptidyl peptidase IV (DPIV) ("DPIV-analogous enzymatic effect") or (iia) alanyl aminopeptidase N (APN) as well as (iib) peptidases having an enzymatic effect analogous to alanyl aminopeptidase N (APN) ("APN-analogous enzymatic effect").

Furthermore, the invention surprisingly found that said novel compounds are capable of interacting not only with the active site of these peptidases, but are also, or alternatively, capable of interacting with the central pore binding sites of these peptidases. As shown for DPIV, this binding site at the ante-chamber of the central pore access located opposite to the membrane of the cell assists the substrate access to the active site of the enzyme. Occupation of this site sterically blocks the access of substrates to the active site and inhibits the function of cellular enzyme. This binding site mediates an autosterical regulation of cellular DPIV and is its most crucial target site to regulate cellular functions, like growth regulation and cytokine production [European Patent Application No. 10 156 805.3 filed on 17 March 2010].

In the meantime, a similar mechanism has been discovered also for alanyl aminopeptidase N. The corresponding data are based on the crystal structure of APN from *Escherichia coli* [K. Ito et al., 2006, J. Biol. Chem. 281, 33664 - 33676].

It should be mentioned that, in contrast to "classical" inhibitors of APN and DPIV which are defined by inhibitory constants such as IC50 values [see, in detail, the patent applications referred to above], the inhibitors/ligands which interact with the central pore binding sites of celluar peptidases, as described here, are mainly characterized by docking approaches. Enzymatic routine assays for these characteristics are not disposable as yet. Therefore these properties are defined mainly by the free energy (-kcal/ mole) of the interaction between the ligand and the respective central pore binding site of DPIV or APN.

With the aim of illustrating the steric inhibition of DPIV as well as of APN, the blocking of the central pore path in both enzymes is shown exemplarly in Figure 2 for an inhibitor substance having no potency of enzymatic inhibition and interaction with the active site. In both examples, the central pore binding site is occupied by a potent inhibitor of the respective enzyme; sitagliptin in the case of DPIV and Bestatin in the case of APN.

Furthermore, it was found surprisingly with the present invention that the novel chemical substances may be used as such for, but also may be used as starting materials for other substances for, a prophylaxis and a therapy of diseases having an excessive immune response (autoimmune diseases, allergies and transplant rejections, sepsis), of other chronic-inflammatory diseases, including arteriosclerosis, of neuronal diseases, and of cerebral damage *(inter alia* multiple sclerosis, Alzheimer's disease and Parkinson's disease), of skin diseases *(inter alia* acne and psoriasis), of tumor diseases and of specific virus infections *(inter alia* SARS) as well as of type I diabetes.

The rationale for the usage of these new chemical entities of peptidase inhibitors/ligands in a prophylaxis and/or a treatment of various inflammatory dieases and of tumor diseases is the following:

**Inflammatory diseases:**

All inflammatory diseases are characterized by an activation of the immune system upon a physical, biological or a chemical challenge. The first step of an immune response is innate and non-specific with respect to the antigen. This is followed by an antigen-specific, adaptive immune resonse. It is well accepted that, also in case of chronic inflammation, the immune response is specific to antigens either from outside or from the own body, which do not exclude non-specific interactions. Besides a non-specific reponse by activation of non-specific cells like granulocytes and macrophages, antigen-specific T and B lymphocytes are the main cellular instruments of a specific immune response. The crucial processes are an activation and a clonal expansion/proliferation of antigen-specific T and B lymphocytes to achieve a sufficiently high cellular potential for a sufficient immune response. The latter is followed by the production of inflammatory cytokines, and finally by anti-inflammatory cytokines to terminate this process.

In case of chronic inflammatory diseases, the termination does not operate adequately. This leads to a permanent proliferation of immune cells and continuing production of inflammatory cytokines followed by destruction of concerned cells and tissues by these pathogenic immune cells.

Remarkably, it is also well known that an activation of the immune system is accompanied by an up-regulation of the expression of APN/CD13 and DPIV/CD26 which are characterized as so-called "activation markers".

The most common chronic inflammatory disases are

- the group of autoimmune disases, such as multiple sclerosis, atherosclerosis, psoriasis, Morbus Parkinson and rheumatoid arthritis, to mention only some prominent of nearly hundred of these diseases;
- the group of allergies such as bronchial asthma and hayfever;
- a rejection of allogenic grafts; and
- other (as yet not fully understood) inflammatory diseases such as Alzheimer's disease and diabetes.

Because of the common mechanism responsible for all inflammatory diseases, it is currently well accepted that one of the most potent approaches to counteract or treat these effects is to suppress the proliferation of activated lymphocytes and to inhibit the production of inflammatory cytokines (e.g. IL-2, IL-17, IL-4) as well as to induce the production of anti-inflammatory cytokines (e.g. TGF-β1, Il-10, N PY, Il-16). Widely accepted immunosuppressive substances in medicine which act via suppression of lymphocyte growth, are cyclosporine A, FK506 and rapamycin. All three compounds exert their pharmacological effects by binding to members of a family of intracellular proteins known as immunophilins, forming complexes that interfere with signaling pathways important for the clonal expansion of lymphocytes [Immunobiology, 2001, C. Janaway et al., "Immunobiology", Garland Publishing , Churchill Livingstone, p 565 - 566].

Inhibitors/ligands of DPIV and APN also fulfill these demands and are a promising novel class of substances for a treatment of inflammatory disorders [S. Ansorge et al., 2009, Clin. Chem. Lab. Med. 47, 253-261; T. Kähne et al, 1999, Int. J. Mol. Med. 1999, 3-15; U. Lendeckel et al., 1999, Int. J. Mol. Med.1999, 17-27; D. M. T. Yu et al, 2010, FEBS J. 2010, 1-19)]. Evidence has been presented that all DPIV and APN inhibitors, even if their molecules have chemical structures widely varying and differing from each other, are capable of suppressing DNA synthesis and meet these requirements to do so, if they show a sufficient interaction with the active sites and/or the central pore binding sites of these peptidases [S. Ansorge et al., 2009, Clin. Chem. Lab. Med. 47, 253-261; European Patent Application No. 10 156 805.3 filed on 17 March 2010].

**Tumor diseases:**

Tumors are characterized by an uncontrolled cell growth and DNA synthesis, as well as by over-activated angio-neogenesis, i.e. generation of small vessels which are necessary for supplying nourishments for growing tumor cells.

Most tumor cells do express DPIV or/and APN on their surface. Remarkably, DPIV and APN inhibitors/ligands are capable to suppress DNA synthesis of tumor cells [D. M. T. Yu et al., 2010, FEBS J. 2010,1-19; N. Petrovic et al., 2004, in: N. Hooper and U. Lendeckel, Aminopeptidases in biology and diseases, Kluwer Academic/Plenum Publishers, New York, p 179 - 200]. Particularly APN inhibitors were shown to be strong antagonists of angiogenesis [R. Pasqualini et al., 2000, Cancer Res.60, 722 - 727; R. Rangel et al., 2007, Proc. Nat. Acad. Sci. 104, 4588 - 4593; N. Petrovic et al., 2004, in: N. Hooper and U. Lendeckel, Aminopeptidases in biology and diseases, Kluwer Academic/Plenum Publishers, New York p 179 - 200].

Consequently, inhibitors/ligands of DPIV and APN fulfill the main requirements for combating tumors in that they suppress DNA synthesis of tumor cells, angiogenesis and metastasis via an interaction of the respective inhibitor or ligand, respectively, with the active sites and/or with the central pore binding sites of these peptidases. It was surprisingly found that this capability is independent of the specific molecular structure of the inhibitor/ligand molecule.

The present invention relates to compounds of the general formula (I),

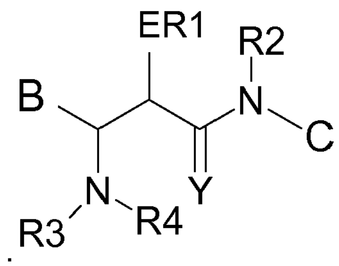

(I)

The meanings of the residues B, E, C and Y in the above general formula (I) are specified and explained in detail below.

Unless not denoted in particular or obvious from the structural formula, all possible stereoisomers of the related embodiments are allowed.

Preferred embodiments of the compounds of the general formula (I) result from subclaim 2 (Table 1).

Compounds of the above general formula (I) may be synthesized by generally known synthetic methods of the organic chemistry described below in detail exemplarily for single compounds or groups of compounds. Such synthetic methods are well known to a person skilled in the field of organic synthesis. These methods are particularly generally known to result into the target compounds in high yields and in high purity. It is also known that the purity of compounds obtained in accordance with these known synthesis methods is not only sufficient, but specifically suitable for making use of such compounds in medical applications, particularly in the administration to patients in need of having such a compound, or two or more of such compounds, administered.

The invention also relates to at least one of the compounds of the above general formula (I) and described below in detail to be used in medicine.

The invention also relates to at least one of the compounds of the general formula (I) mentioned above and described below in detail, for medical use, said use being for the prophylaxis and therapy of diseases with exceeding immune response and inflammatory genesis including arteriosclerosis, neuronal disease, cerebral damages, skin diseases, tumour diseases, virus-caused diseases, and type I diabetes.

The invention relates also to pharmaceutical or cosmetic preparations, comprising at least one of the compounds of the general formula (I) according to one of the claims 1 or 2 and according to the following detailed description, optionally in combination with one or more pharmaceutically or cosmetically effective compounds and further optionally with one or more pharmaceutically or cosmetically acceptable carrier(s), auxiliary compound(s) and/or adjuvant(s).

The invention also relates to pharmaceutical preparations, comprising at least one of the compounds of the general formula (I) according to one of the claims 1 or 2 and as described below in detail, for medical use.

Furthermore, the invention relates to pharmaceutical preparations, comprising at least one of the compounds of the general formula (I) according to one of the claims 1 or 2 and as described below in detail, for medical use, said use being for the prophylaxis and therapy of diseases with exceeding immune response and inflammatory genesis including arteriosclerosis, neuronal disease, cerebral damages, skin diseases, tumour diseases, virus-caused diseases, and type I diabetes.

Finally, the invention also relates to the use of at least one of the compounds of the above general formula (I) for a cosmetic use.

In the following, the invention is described in detail. Reference is made in such a description to preferred embodiments of the invention, which, in an exemplary manner, describe the invention for a better understanding thereof, and even describe the best mode of the invention presently known. However, the invention is not restricted to the examples given for a better understanding thereof.

According to the invention, the new compounds have the general formula (1):

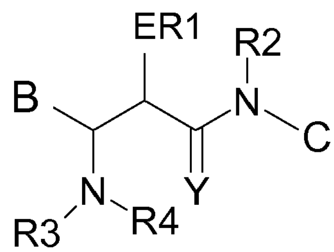

(I)

wherein the residues R1, R2, R3 and R4 may be the same or different and are independently selected from the group consisting of -H; -halogen (i. e. -F, -Cl, -Br, -I); alkyl having 1 to 25 carbon atoms, which alkyl may be straight chain or branched, saturated or once, twice or more times unsaturated (-C=C- double bonds and/or -C≡C- triple bonds), unsubstituted or substituted with any of the residues R1, R2, R3 and/or R4, and/or uninterrupted or interrupted by any of the residues -O-, -NH-, -NR5-, -S-; >C(=O), -C(=O)O-, -O-C(=O)-, -C(=O)NH-, -C(=O)NR5-,- NHC(=O)-, -NR5(C=O)-, >C(=S), -C(=S)O-, -O-C(=S)-, -C(=S)NH-, -C(=S)NR5-, -NHC(=S)-, -NR5(C=S)-, -PH-, -PR5-, >P(=O)$H_2$, >P(=O)H, >P(=O)R5, >P(=O)(OH), >P(=O)OR5; cycloalkyl having 3 to 9 ring members, which cycloalkyl may be saturated or once, twice or more times unsaturated (-C=C- double bonds and/or -C=C- triple bonds), unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; aryl having 3 to 9 ring members, which aryl may be unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; which cycloalkyl and/or aryl groups may form non-condensed ring systems or ring systems comprising one, two or more condensed rings selected from cycloalkyl, heretocycloalkyl, aryl or heteroaryl rings; -OH, -OR5, -$NH_2$, -NHR5, -NR5R6, -C(=O)H, -C(=O)R5, -C(=O)OH, -C(=O)OR5, -C(=O)$NH_2$, -C(=O)NHR5, -C(=O)NR5R6, -NH-C(=O)H, -NR5(C=O)H, -NH-C(=O)R5, -NR5(C=O)R5, -C(=S)OH, -C(=S)OR5, -C(=S)$NH_2$, -C(=S)NHR5, -C(=S)NR5R6, -O-C(=O)H, -OC(=O)R5, -NH(C=O)R5, -NR5(C=O)R6, -C(=O)(NHOH), -C(C=O)(NR5OH) -C(C=O)(NR5OR6) -C(C=0)NHOR5, -$PH_2$, -PHR5, -PR5R6, -P(=O)$H_2$, -P(=O)R5H, -P(=O)R5R6, -P(=O)$(OH)_2$, -P(=O)R5OH -P(=O)OR5OR6;
wherein R5 and R6 may be the same or different and may be selected from the group of residues defined above by R1, R2, R3 and R4;
E may represent a group selected from -O-, -S-, -NH- or -NR7-, wherein R7 is a group which may be selected from the group of residues defined above by R1,R2, R3 and R4;
Y may represent a group selected from -O-, -NH-, -NR8-, -S-, -$CH_2$-, -CHR8-and -CR8R9-, wherein R8 and R9 may be the same or different and may be selected from the group of residues defined above by R1, R2, R3 and R4;
B may represent a group having the general formula (II)

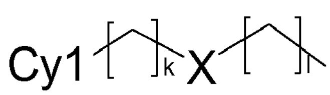

or

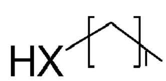

(IIa) (IIb)

wherein

Cy1 may represent condensed or non condensed, aromatic or non aromatic homo- or heterocyclic systems having 3 to 9 ring members, and, in the case of a condensed system, 3 to 9 ring members in each partial ring which, in the case of non aromatic moieties, may be saturated or once, twice or more times unsaturated (-C=C- double bonds and/or -C≡C- triple bonds), and Cy1 may be unsubstituted or substituted with any of the residues R1, R2, R3 and/or R4, and/or may comprise one or several hetero-atoms within the ring structure, which heretoatoms may be selected from the group consisting of -O-, unsubstituted or alkyl-substituted -N<, - S- and -P<; aryl having 3 to 9 ring members, and, in the case of a condensed system, 3 to 9 ring members in each partial ring, which aryl may be unsubstituted

or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; which cycloalkyl and/or aryl groups may form non-condensed ring systems or ring systems comprising one, two or more condensed rings selected from cycloalkyl, heretocycloalkyl, aryl or heteroaryl rings;

X may represent a single bond, -O-, -S-, -NH-, -NR10-, -$CH_2$-, -CHR10-, -CR10R11-, >C(=O), >C(=S), >C(=NH), >C(=NR10), -C(=O)O-, -C(=S)O-, -C(=NH)NH-, -C(=O)NH-, -C(=O)NR10-, -O(C=O)-, -NH(C=O)-, -NR10(C=O)-, -O(C=S)-, -NH(C=S)-, or -NR10(C=S)-, wherein R10 and R11 may be the same or different and may be selected from the group of residues defined above by R1, R2, R3 and R4;

k and l may be the same or different and represent zero (0) or may be integers in the range of from 1 to 5;

C may represent a group having the general formula (III)

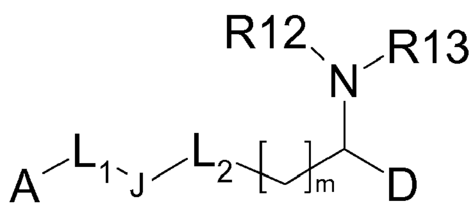

(III)

wherein

m may be the same or different and represent zero (0) or may be integers in the range of from 1 to 5;

the sequence A - L1 - J - L2 as a whole may be a single bond, or

A may be absent or may be selected from the group consisting of residues defined as for R1 above, with the proviso that the carbon chain may have 1 to 10 carbon atoms; and

J may be absent or may be selected from the group consisting of alkylene having 1 to 10 carbon atoms, which alkylene may be straight chain or branched, saturated or once, twice or more times unsaturated (-C=C- double bonds and/or -C=C- triple bonds), unsubstituted or substituted with any of the residues R1, R2, R3 and/or R4, and/or uninterrupted or interrupted by any of the residues -O-, -NH-, -NR5-, -S-; >C(=O), -C(=O)O-, -O-C(=O)-, -C(=O)NH-, -C(=O)NR5-, -NHC(=O)-, -NR5(C=O)-, >C(=S), -C(=S)O-, -O-C(=S)-, -C(=S)NH-, -C(=S)NR5-, -NHC(=S)-, -NR5(C=O)-, -PH-, -PR5-, >P(=O)$H_2$, >P(=O)H, >P(=O)R5, >P(=O)(OH), >P(=O)OR5; cycloalkylene having 3 to 9 ring members, which cycloalkylene may be saturated or once, twice or more times unsaturated (-C=C- double bonds and/or -C≡C- triple bonds), unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; arylene having 3 to 9 ring members, which arylene may be unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; which cycloalkylene and/or arylene groups may form non-condensed ring systems or ring systems comprising one, two or more condensed rings selected from cycloalkyl, heretocycloalkyl, aryl or heteroaryl rings; -NH-, -NR5-, -C(=O)-, -C(=O)O-, -C(=O)R5-, -C(=O)NH-, -C(=O)NR5-, -NH-C(=O)-, -NR5-C(=O)-, -C(=S)O-, -C(=S)R5-, -C(=S)NH-, -C(=S)NHR5-, -C(=S)NR5- -NH-C(=O)-, -NR5-C(=O)-, -C(=O)(NHO)-, -C(=O)(NR5O)- -PH-, -PR5-, -P(=O)H-, -P(=O)R5-, -P(=O)(OH)-, -P(=O)OR5-; wherein R5 and R6 may be the same or different and may be selected from the group of residues defined above by R1, R2, R3 and R4;

L1 and L2 may be the same or different and may represent a single bond or may represent moieties each independently selected from the group consisting of -$CH_2$-, -O-, >C=O, -NH-, -NR12-, -S-, >C=S, -$SO_2$-,

-C(=O)-O-, -C(=S)-O-, -C(=O)-S-, -C(=S)-S-, -C(=O)NH-, -C(=O)NR14-, -C(=S)NH-, -C(=S)NR14-, -C(=NH)-,

-C(=NH)-NH-, -C(=NH)-NR14-, -C(=NR1)-NR14-, wherein R12, R13 and R14 may be the same or different and may be selected from the group of residues defined above by R1, R2, R3 and R4; and

D represents any of the structures (IVa) or (IVb)

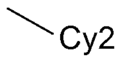 (IVa) 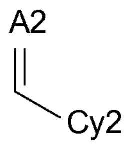 (IVb)

wherein

Cy2 is homo- or heterocyclic, non-aromatic or aromatic, uncondensed or once or twice condensed, annelated structural element and binds directly to the rest of the structure, which, in the case of heteroaromatic residues, may contain the groups -N=, -NH-, -NR1-, -S-, -O-, -S(=O)-, $-S(=O)_2-$, -P=, -PH-, -PR15-, -P(=O)-, -OP(=O)- and -P(=O)O- as the ring members, where carbon or a heteroatom moiety may be the connecting unit to structural part C (IVa) and =A2 (IVb), respectively, wherein, in the case of non-aromatic moieties Cy2, the ring structures making up Cy2 may be saturated, may be partially unsaturated, may be unsubstituted or substituted once, twice or more times at any chemically possible position by any of the substituents defined above as substituents for cycloaliphatic and aromatic residues, and Cy2 may comprise 3 to 9 ring members, and, in the case of a condensed system, 3 to 9 ring members in each partial ring; and

A2 may represent a group selected from =C, =CH, =CR16, -O-, -S-, -NH- and -NR16-, wherein R15 and R16 may be the same or different and may be selected from the group of residues defined above by R1, R2, R3 and R4.

The above novel compounds of the general formula (I) may be present, or may be synthesized, or may be used in whatever field, particularly in the medical field, as neutral molecules represented by the above general formula (I). Alternatively, the novel compounds of the general formula (I) may be present, or may be synthesized, or may be used in whatever field, particularly in the medical field, as acid addition salts with physiologically or pharmaceutically acceptable inorganic or organic acids. The acids for such acid addition salts of the compounds of the general formula (I) of the invention are not specifically restricted, but are suitably selected from the group consisting of hydrochloric acid, trifluoroacetic acid, tartaric acid, succinic acid, formic acid and/or citric acid, thus resulting into acid addition salts of the compounds of the general formula (I) selected from hydrochlorides, trifluoroacetates, tartrates, succinates, formiates and/or citrates.

It was surprisingly found that the compounds of the above formula (I) themselves have inhibitory effects with regard to the enzymes mentioned in detail below. Moreover the said compounds react to other compounds under defined conditions, and hence are precursors of such other compounds. These other compounds, in turn, are also inhibitors/ ligands of the enzyme dipeptidyl peptidase IV (DPIV) and of peptidases with analogous enzymatic effect and are also inhibitors/ligands of the enzyme alanyl aminopeptidase N (APN) and of peptidases with analogous enzymatic effect.

The term "comprise" as used in the present specification and claims, for example in claim 15 or claim 17, has the meaning that said composition of the invention may comprise (i) at least one compound of the general formula (I) or may comprise (ii) two or more compounds of the general formula (I), or that (iii) further components (more specifically defined below) may also be comprised by the composition.

The term "comprise" as used in the present specification and claims may, however, also include cases where the composition of the invention mainly consists of (i) at least one compound of the general formula (I) or mainly consists of (ii) two or more compounds of the general formula (I), optionally together with any necessary component a skilled person may include into such a composition in order to achieve the object of the invention, or may even include cases where the composition of the invention exclusively consists of (i) at least one compound of the general formula (I) or exclusively consists of (ii) two or more compounds of the general formula (I), optionally together with any necessary component a skilled person may include into such a composition in order to achieve the object of the invention.

In other words: The term "comprise" or "comprises" or "comprising" may have, in the present specification and claims, the meanings of describing an exhaustive or, alternatively, a non-exhaustive enumeration of elements.

Using the term "dipeptidyl peptidase IV" (DPIV, CD26, EC 3.4.14.5) in the following description and in the claims, the serine protease is recognized which catalyzes the hydrolysis of peptide bonds specifically after proline and

to a lesser degree alanine and - with restrictions - after other amino acids like serine, threonine, valine, and glycine respectively at the second position of the N-terminus of peptides.

Using the term "peptidases with dipeptidyl peptidase IV-analogous enzymatic effect", peptidases are recognized in the present description and in the claims which catalyze the hydrolysis of peptides specifically after proline or alanine at the second position of the N-terminus. Examples of peptidases with dipeptidyl peptidase IV-analogous enzymatic effect are, without restricting the invention to those, DPII, DP 8, DP 9 and FAP/seprase [T. Chen et al., a. a. O.] and attractin (mahagony protein) [J. S. Duke-Cohan et al., a. a. O.].

Using the term "alanyl aminopeptidase N" (APN, CD13, EC 3.4.11.2) in the present description and in the claims the protease is recognized, which operates metal- (zinc-) dependent and catalyzes the hydrolysis of peptide bonds specifically after N-terminal amino acids of peptides and preferably alanine at the N-terminus.

Using the term "peptidases with alanyl aminopeptidase N-analogous enzymatic effect" peptidases are recognized in the present description and in the claims, which - like APN - operate metal-dependent and catalyze the hydrolysis of peptide bonds specifically after N-terminal amino acids of peptides and preferably after alanine at the N-terminus. Examples of peptidases with alanyl aminopeptidase N-analogous enzymatic effect are, without restricting the invention thereto, the cytosolic soluble alanyl aminopeptidase (EC 3.4.11.14, puromycine-sensitive aminopeptidase, aminopeptidase PS, encephaline-degrading aminopeptidase) [A. J. Barret et al., a. a. O.]

Using the term "inhibitor" and "ligand" in the present description and in the claims, such compounds of natural origin, synthetic origin or natural origin with synthetic modification are recognized which have a regulatory, particularly inhibitory effect on an enzyme or a group of enzymes. Such a regulatory or particularly inhibitory effect may be an effect mainly or partially on the central pore binding site of the enzyme or of the enzymes.

This does not exclude that precursors *per se* are able, before being transformed into drugs with a defined pharmacological (for example inhibitory) effect, to develop a pharmacological effect (for example to inhibit one of the two, or both, afore-mentioned enzymes).

Using the term "alkyl residue" in the present description and in the claims, a monovalent straight chain ("unbranched") or branched residue made of carbon atoms linked by single bonds to each other with hydrogen atoms bound to the carbon atoms is recognized. Hence, alkyl residues are, according to the present invention, saturated monovalent hydrocarbon residues. Preferably the alkyl residues in the compounds of the general formula (1) comprise 1 to 18 carbon atoms and are thus selected from the residues methyl, ethyl, n-propyl, i-propyl and the numerous different straight chain and branched isomers of the residues butyl, pentyl, hexyl, heptyl, octyl, nonyl, decyl, undecyl, dodecyl, tridecyl, tetradecyl, pentadecyl, hexadecyl, heptadecyl and octadecyl. Particularly preferred are straight chain and branched alkyl residues having 1 to 12 carbon atoms. Straight chain and branched alkyl residues having 1 to 6 carbon atoms are even more preferred. In the present specification and claims, alkyl residues having 1 to 6 carbon atoms sometimes are also referred to as "lower alkyl" residues. Most preferred alkyl residues are the residues methyl, ethyl, n-propyl, i-propyl, n-butyl, i-butyl, sec-butyl and tert-butyl.

Accordingly in the present description and in the claims, for the terms "alkenyl residue" and "alkinyl residue", monovalent straight chain ("unbranched") or branched residues of carbon atoms linked to each other by single bonds and at least one double bond or triple bond, respectively at an arbitrary, but defined position in the molecule, with hydrogen atoms being bound to the remaining bonds of the carbon atoms are recognized, said molecules having at least 2 carbon atoms and up to 18 carbon atoms. Preferably the alkenyl residues or alkinyl residues in the compounds of the general formula (1) comprise 2 to 18 carbon atoms and are thus selected from the residues ethenyl/ethinyl, n-propenyl/propinyl, i-propenyl/propinyl and the numerous different straight chain and branched isomers of the residues butenyl/butinyl, pentenyl/pentinyl, hexenyl/hexinyl, heptenyl/heptinyl, octenyl/octinyl, nonenyl/noninyl, decenyl/decinyl, undecenyl/undecinyl, dodecenyl/dodecinyl, tridecenyl/tridecinyl, tetradecenyl/tetradecinyl, pentadecenyl/pentydecinyl, hexadecenyl/hexadecinyl, heptadecenyl/heptadecinyl and octadecenyl/octadecinyl. Particularly preferred are straight chain and branched alkenyl/alkinyl residues having 1 to 12 carbon atoms. Straight chain and branched alkenyl/alkinyl residues having 1 to 6 carbon atoms are even more preferred. In the present specification and claims, alkenyl/alkinyl residues having 1 to 6 carbon atoms sometimes are also referred to as "lower alkenyl" or "lower alkinyl" residues. Most preferred alkenyl/alkinyl residues are the residues ethenyl, vinyl, ethinyl, n-propenyl, allyl, n-propinyl, i-propenyl, i-propinyl, n-butenyl, n-butinyl, i-butenyl, i-butinyl, sec-butenyl, sec-butinyl, tert-butenyl and tert-butinyl. Alkenyl and alkinyl residues according to the present invention may also contain more than one multiple C-C bond. Such multiple C-C bonds may be isolated C-C multiple bonds (i. e. more than one C-C single bond is between two C-C multiple bonds) or may be conjugated C-C multiple bonds. A common example for a conjugated C-C multiple bond may be found in a 1.3-butadien-3-yl residue. However, carbon-carbon multiple bond-containing residues are not restricted to said residues specifically mentioned above.

In the present descriptions and in the claims the term "alkylene residue" is recognized to be a divalent straight chain ("unbranched") or branched residue of carbon atoms linked to each other by single bonds with hydrogen atoms bound to the remaining bonds of the carbon atoms not bound to another carbon atom. Hence, alkylene residues are according to the present invention saturated divalent hydrocarbon residues. Preferably, alkylene residues in the compounds of the general formulae (1) and (2) comprise 1 to 18 carbon atoms and are selected from the residues methylene, ethylene, n-propylene, 2,2-propylene, 1,2-propylene and numerous different straight chain and branched isomers of the residues butylene, pentylene, hexylene, heptylene, octylene, nonylene, decylene, undecylene, dodecylene, tridecylene, tetradecylene, pentadecylene, hexadecylene, heptadecylene and octadecylene. Particularly preferred are straight chain and branched alkylene residues having 1 to 12 carbon atoms. Straight chain and branched alkylene residues having 1 to 6 carbon atoms are more preferred. In the present specification and claims, alkylene residues having 1 to 6 carbon atoms sometimes are also referred to as "lower alkylene" residues. Most preferred are the residues methylene, ethylene, n-propylene, 2,2-propylene, 1,2-propylene and the numerous different butylenes position isomers.

In the alkyl-residues and/or the alkylene-residues which, according to the invention, may be part of the compounds of the general formula (1), the chains of carbon atoms might be interrupted by O-atoms, N-atoms, S-atoms or P-atoms. Hence, in the course of the chain, there might exist instead of one or more $-CH_2$-group(s) one or more group (s) of the group -O-, -NH-, -S- and -P-, whereas usually not two of the groups -O-, -NH-, -S- and/or -P- groups follow each other in the chain. Said one or more group(s) -O-, -NH-, -S- or -P- can be inserted at arbitrary positions in the molecule. Preferably, if a hetero group of that ilk is present, a group of that ilk is present in the molecule.

Straight chain as well as branched alkyl- or alkylene-residues might be substituted, according to the invention in the compounds of the general formula (1) in a further embodiment, with one or more substituents, preferably with one substituent. Even more preferably, the substituent is selected from those residues defined above for R1, R2, R3 and R4. The substituent(s) can be located at arbitrary positions of the backbone, formed by the carbon atoms and can preferably, without restricting the invention hereto, be selected from the group consisting of halogen atoms like fluorine, chlorine, bromine and iodine, particularly preferred chlorine and bromine, alkyl groups having 1 to 6 carbon atoms each, particularly preferred alkyl groups having 1 to 4 carbon atoms, alkoxy groups having 1 to 6 carbon atoms in the alkyl residue, preferably having 1 to 3 carbon atoms in the alkyl residue, unsubstituted or - with one or two alkyl residue(s) containing 1 to 6 carbon atoms independently from each other, preferably 1 to 3 carbon atoms - substituted amino groups, carbonyl groups and carboxyl groups. The latter can also be present in form of salts or esters with alcohols having 1 to 6 carbon atoms in the alkyl residue; hence the term "carboxyl-groups" includes groups of the general structure -COO- $M^+$ (with M = monovalent metal atom such as an alkali metal-atom or an accordant equivalent of a polyvalent metal atom such as half an equivalent of a divalent metal atom like an alkaline earth metal atom) or of the general structure $-COOR_x$ (with $R_x$ = alkyl groups having 1 to 6 carbon atoms). The substituting alkyl groups are selected from alkyl groups mentioned above in detail and are particularly preferred methyl groups, ethyl groups, n-propyl groups, i-propyl groups, n-butyl groups, i-butyl groups, sec-butyl groups or tert-butyl groups.

Alkoxy groups are alkyl groups in the above-defined sense which are bound via an O-atom to the backbone formed by the carbon atoms. They are preferably selected from the group consisting of the residues methoxy, ethoxy, n-propoxy, i-propoxy; n-butoxy, i-butoxy, sec-butoxy and tert-butoxy.

Amino groups are groups of the general structure $-NR_x R_y$ in which the residues $R_x$ and $R_y$ might independently from each other designate: hydrogen or alkyl groups (according to the afore-mentioned definition) having 1 to 6 carbon atoms particularly preferred having 1 to 3 carbon atoms in which the residues $R_x$ and $R_y$ might be identical to or different from each other. Such amino groups being particularly preferred as substituents are $-NH_2$, $-NH(CH_3)$, $-N(CH_3)_2$, $-NH(C_2H_5)$ and $-N(C_2H_5)_2$. The term "amino groups" contains also groups of the above-defined structure which are present as quaternary ammonium ions, either because of salt formation with organic acids or inorganic acids (e.g. residues of the structure $R_x R_y R_z N^+ Q^-$, in which $R_x$, $R_y$ and $R_z$ might be identical or different, preferably identical, and $R_x$ and $R_y$ might have the above-defined meanings, and at least one of the residues is hydrogen from the quaternation with the organic or inorganic acid, and Q is an acid residue from an acid of the organic or inorganic acid) or because of salt formation with suitable quaternation reagents which are known to a person skilled in the field such as (without restriction hereto) with alkyl halogenids.

In the present description and in the claims the term "cycloalkyl" is used for unsubstituted or substituted monovalent residues of $-CH_2$ groups linked to each other in form of closed rings. According to the invention said rings might contain preferably 3 to 8 atoms forming the ring and might either contain exclusively carbon atoms ("carbocyclic cycloalkyl residues") or contain one or more hetero atom(s) ("heterocyclyl residues") which heteroatoms is/are selected from -O-, -S- and $-NR_x$- in which $R_x$ is hydrogen or a alkyl residue (as defined above) having 1 to 6 carbon atoms. In case hetero atoms are inserted into the rings, said hetero atoms can be - in case of more than one hetero atom - identical or different. Preferably in case hetero atoms are present one hetero atom is inserted into the ring. Particularly preferred among purely carbocyclic rings are the residues cyclopentyl, cyclopentenyl, cyclopentadienyl, cyclohexyl, cyclohexenyl, cyclohexadienyl, cycloheptyl, cycloheptenyl, cycloheptadienyl and cycloheptatrienyl. Examples for heteroatom-containing cycloalkyl residues ("heterocyclyl residues"), are the residues tetrahydrofuranyl, pyrrolidinyl, pyrazolidinyl, imidazolidinyl, piperidinyl, piperazinyl and morpholinyl.

Possible substituents at the carbocyclic or heterocyclic cyloalkyl residues might be preferably, without restricting the invention hereto, selected from the afore-mentioned group of substituents for linear alkyl-groups. Particularly preferred substituents for cycloalkyl-groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy and -tert-butoxy, $-NH_2$, $-NH(CH_3)$, $-N(CH_3)_2$, $-NH(C_2H_5)$ and $-N(C_2H_5)_2$, -carbonyl and -carboxyl.

In the present description and in the claims the term "cycloalkylene" is used for unsubstituted or substituted divalent residues of $-CH_2$ groups linked to closed rings. According to the invention these can preferably contain three to eight atoms in the ring and can consist either exclusively of carbon atoms or contain one or more hetero atom(s) which is/are selected from -O-, -S- and $-NR_x-$, in which $R_x$ is hydrogen or a alkyl-residue (as defined above) having 1 to 6 carbon atoms. Particularly preferred among the purely carbocyclic rings are the residues cyclopentylen, cyclopentenylen, cyclopentadienylen, cyclohexylen, cyclohexenylen, cyclohexadienylen, cycloheptylen, cycloheptenylen, cycloheptadienylen and cycloheptatrienylen. Also, the heterocyclic groups defined above with regard to the cycloalkyl residues can appear in compounds of the general formula (I) as groups "B" in form of divalent residues and particularly preferred are such cyclic divalent residues in which one group -O- or $-NR_x-$ is inserted into the ring. In those cases, both valences are localized at arbitrary carbon atoms in the ring. Preferably one hetero atom or two hetero atom(s) is/are inserted into the ring and in particularly preferred embodiments of such groups, the divalent residues are derived from tetrahydrofuran, pyrrolidin, pyrazolidin, imidazolidin, piperidin, piperazin and morpholin.

Possible substituents at these carbocyclic or heterocyclic cycloalkylene residues can be preferably, without restricting the invention hereto, selected from the afore-mentioned group of substituents for linear alkyl-groups. Particularly preferred substituents for cycloalkylene-groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy and -tert-butoxy, $-NH_2$, $-NH(CH_3)$, $-N(CH_3)_2$, $-NH(C_2H_5)$ and $-N(C_2H_5)_2$, -carbonyl and -carboxyl.

Using the term "aryl residue" in the present description and in the claims, a monovalent hydrocarbon residue is recognized, which is derived from a cyclic molecule with aromatic character ($4n + 2$ $\pi$-electrons delocalized in ring-shaped orbitals) which might be unsubstituted or substituted. The ring structure of such an aryl residue can be a five-, six- or seven-membered ring structure with one ring or a structure formed by two or more ("annelated") rings bound to each other where the annelated rings have identical or different numbers of ring members, particularly of C-atoms. In case of systems consisting of at least two rings condensated to each other, benzo-condensated rings are particularly preferred, i.e. a ring system in which at least one of the rings is an aromatic six-membered ring exclusively containing C-atoms (e. g. a phenyl ring). Typical but not limiting examples of aryl rings are cyclopentadienyl-residues ($C_5H_5^-$) (being a five-membered aryl ring), phenyl-residues (being a six-membered aryl ring), cycloheptatrienyl-residues ($C_7H_7^+$) (being an seven-membered aryl ring) naphthyl-residues (being a ring system comprising two annelated six-membered rings) as well as monovalent residues being derived from anthracen and phenanthren (being three annelated six-membered rings). According to the invention most preferred aryl-residues are phenyl- and naphthyl-residues.

Possible substituents of carbocyclic aryl-residues can be selected preferably from the groups of substituents mentioned above for linear alkyl-groups, without restricting the invention to these substituents. Particularly preferred substituents for aryl-groups are substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy and -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituent(s) of this group, which might be identical to or different from each other, can be bound to one aryl residue according to the present invention. The substituted position(s) at the aryl ring (system) can be chosen arbitrarily.

A comparable definition as in case of the aryl residues applies to the present description and the claims with regard to the definition of the term "arylene residue": In this regard, a divalent residue is recognized the elementary composition of which, the selection of which and the substituent(s) of which are comparable to the afore-mentioned definitions of the aryl residues, with the exception that it is a divalent residue the insertion of which can be carried out at two arbitrary carbon atoms.

In the present description and in the claims, by the term "heteroaryl residue" an aryl residue is recognized (in accordance with the afore-mentioned definition) the ring structure of which contains one or more hetero atom(s) preferably from the group O, N or S, without losing the aromatic character of the molecule. The ring structure of such a heteroaryl residue may either be a five-membered, a six-membered or a seven-membered ring structure with one ring or may be a structure formed by two or more ("annelated") rings bound to each other, wherein the annelated rings might have an identical or a different number of ring members. The hetero atom(s) can occur in one ring alone or in more than one ring of the ring system, or a hetero atom may even exist at the "bridge" between two allelated rings.

The heteroaryl residues preferably consist of one or two rings. In case of systems consisting of more than one ring, e. g. two rings condensed to each other, benzo-condensed rings are especially preferred, i.e. ring systems in which at least one of the rings is an aromatic carbocyclic (i.e. containing only carbon atoms) six-membered ring. Particularly preferred heteroaryl residues are selected from furanyl, thiophenyl, pyridyl, indolyl, cumaronyl, thionaphthenyl, chinolinyl (benzopyridyl), chinazolinyl (bezopyrimidinyl) and chinoxylinyl (benzopyrazinyl).

Heteroaryl residues can be unsubstituted or substituted according to the invention. Possible substituents at these heteroaryl residues can be preferably selected from the afore-mentioned group of substituents for linear alkyl groups without restricting the invention to these substituents. Particularly preferred substituents for heteroaryl-groups

are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy, -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituents of that group, which might be identical to or different from each other, might be bound to one heteroaryl residue according to the present invention. The substituted position (s) at the heteroaryl ring (-system) can be selected arbitrarily.

A comparable definition as in the case of the heteroaryl residues applies to the present description and the claims with regard to the definition of the term "heteroarylene residue": In this regard a divalent residue is recognized the general composition of which and the selection of which and the substituents of which are comparable to the afore-mentioned definition of "heteroaryl residues", with the exception that it is a divalent residue the insertion of which can be carried out at two arbitrary carbon atoms of the ring or the ring system, respectively, or at a nitrogen atom as well.

In the context of the present description and in the claims the terms "aralkyl residue", "heteroarylalkyl residue", "heterocycloalkyl residue", "arylamidoalkyl residue" and "heteroarylamidoalkyl residue", mean alkyl residues (or - more specifically - alkylene residues) according to the afore-mentioned general and specific definition which are substituted at one of their bonds with an aryl-residue (according to the afore-mentioned general and specific definition), heteroaryl residue (according to the afore-mentioned general and specific definition), heterocyclyl residue (according to the afore-mentioned general and specific definition of the cycloalkyl residues substituted with hetero atoms), arylamido residues (according to the following general and specific definition) or heteroarylamido residues (according to the following general and specific definition). These residues can be unsubstituted or substituted.

In preferred embodiments of the invention aralkyl residues are residues of that group, in which the aryl residue is a phenyl residue, substituted phenyl residue, naphthyl residue or substituted naphthyl residue and the alkyl(ene) group is straight-chained or branched and may have 1 to 6 carbon atoms. In a very particular and advantageous way, the residues benzyl, phenethyl, naphthylmethyl and naphthylethyl can be used as aralkyl residues, of which benzyl residues are particularly preferred.

Possible substituents at the aryl groups of the aralkyl residues can be preferably selected from the afore-mentioned group of substituents for linear alkyl groups without restricting the invention to those substituents. Particularly preferred substituents for aryl groups of the aralkyl residues are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy, -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One ore more substituents of that group which might be identical or different from each other can be bound to one aryl group of an aralkyl residue according to the present invention. The substituted position(s) at the aryl ring (-system) can be chosen arbitrarily.

In preferred embodiments of the invention the heteroarylalkyl residues are such residues in which the heteroaryl residue of the heteroarylalkyl residue according to the invention is substituted and the alkylene group is a straight chain or branched alkylene group and may have 1 to 6 carbon atoms. The ring structure of such a heteroaryl residue can be a ring structure with one ring or a structure formed by two or more than two ("annelated") rings bound to each other wherein the annelated rings might have an identical or different number of ring members. The hetero atom(s) can occur in one or more ring(s) of the ring system. The heteroaryl residues of the heteroarylalkyl residue consist preferably of one or two rings. In case of heteroarylalkyl systems composed of at least two rings condensated to each other, benzo-condensated rings are especially preferred, i.e. ring systems in which at least one of the rings is an aromatic carbocyclic six-membered ring. Particularly preferred heteroarylalkyl residues are selected from furanylmethyl and -ethyl, thiophenylmethyl and -ethyl, pyridylmethyl and -ethyl, indolylmethyl and -ethyl, cumaronylmethyl and -ethyl, thionaphthenylmethyl and -ethyl, chinolinyl-(bezopyridyl-)methyl and -ethyl, chinazolinyl-(benzopyrimidinyl-) and chinoxylinyl-(benzopyrazinyl-)methyl and -ethyl.

Possible substituents at these heteroaryl-groups of heteroarylalkyl-residues can be preferably selected from the afore-mentioned group of substituents for linear alkyl groups without restricting the invention to thereto. Particularly preferred substituents for heteroaryl groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituent(s) of that group which can be identical to or different from each other can be bound to a heteroarylalkyl residue according to the present invention. The substituted position(s) at the heteroaryl ring (-system) can be chosen arbitrarily.

In preferred embodiments of the invention heterocycloalkyl residues are cycloalkyl residues according to the afore-mentioned general and specific definition, which contain one or more hetero atom(s) which is/are selected from -O- -S- and $-NR_x-$, in which $R_x$ is hydrogen or an alkyl residue having 1 to 6 carbon atoms (as defined above) and the alkyl(ene) groups of the heterocycloalkyl residues are straight-chained or branched and may have 1 to 6 carbon atoms. In case of at least two hetero atoms inserted into the ring(s), these can be identical or different. Preferably one hetero atom is incorporated in the ring. Preferred examples for hetero atoms containing cycloalkyl residues which are also referred to as heterocycloalkyl residues are, in further embodiments of the invention, the residues tetrahydrofuranyl, pyrrolinidyl, pyrazolidinyl, imidazolidinyl, piperidinyl, piperazinyl and morpholinyl.

Possible substituents at these heterocycloalkyl residues can preferably be selected from the afore-mentioned group of substituents for linear alkyl groups, without restricting the invention to those substituents. Particularly preferred substituents for heteroaryl groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituent(s) of that group, which might be identical to or different from each other, can be bound to one heterocycloalkyl residue according to the present invention. The substituted position(s) at the heterocacloalkyl ring (-system) can be chosen arbitrarily.

Using the terms "arylamidoalkyl-residue" and "heteroarylamidoalkyl-residue" in the present description and in the claims, alkyl residues (more precisely: alkylene residues) according to the afore-mentioned general and specific definition are recognized which are substituted at one of their bonds by an arylamido residue or heteroarylamido residue of the general formula $Ar-NR_x-C(=O)-$ or the general formula $Ar-C(=O)-NR_x-$ in which $R_x$ is hydrogen or an alkyl having 1 to 6 carbon atoms and Ar is an arbitrary aryl residue or heteroaryl residue according to the afore-mentioned general or specific definition. These aryl or heteroaryl residues can be unsubstituted or substituted. Preferred examples for an arylamidoalkyl residue - without restricting the invention - are 2-, 3- or 4-benzoic acid-amino-n-butyl residues or 2-nitro-3-, -4-, -5- or -6-benzoic acid-amido-n-butyl residues; preferred but not limiting examples for heteroarylamidoalkyl residues are 2-, 4-, 5- or 6-pyridin-3-carbonic acid-amido-n-butyl residues.

Possible substituents at these arylamidoalkyl residues and heteroarylamidoalkyl residues can preferably be selected from the afore-mentioned group of substituents for linear alkyl groups, without restricting the invention to those substituents. Particularly preferred substituents for aryl groups or heteroaryl groups of the arylamidoalkyl residues and heteroarylamidoalkyl residues are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy, -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$,- carbonyl and -carboxyl. One or more substituent(s) of that group which can be identical to or different from each other can be bound to an aryl or heteroaryl group of the arylamidoalkyl residues or heteroarylamidoalkyl residues according to the present invention. The substituted position(s) at the aromatic ring (-system) can be chosen arbitrarily.

A comparable definition as for the aralkyl residues, heteroarylalkyl residues, heterocycloalkyl residues, arylamidoalkyl residues and heteroarylamidoalkyl residues applies in the context of the present description and the claims with regard to the definition of the terms "aralkylene residue", "heteroarylalkylene residue", "heterocycloalkylene residue", "arylamidoalkylene residue" and "heteroarylamidoalkylene residue": These are understood to be divalent residues which general composition and the selection thereof and the substituent(s) thereof are comparable to the afore-mentioned definition of "aralkyl residue", "heteroarylalkyl residue", "heterocycloalkyl residue", "arylamidoalkyl residue" and "heteroarylamidoalky residue", with the exception that it is in either case a divalent residue the insertion of which can be carried out at two arbitrary carbon atoms of the ring or the ring system of the alkylene group respectively, or also at a nitrogen atom of the heteroaryl or heterocyclyl ring system.

According to the invention, the compounds of the general formula (I) are present in form of neutral molecules and are according to the invention used as neutral molecules. Alternatively, the compounds of the general formula (I) can also be present in form of their acid addition salts with inorganic and/or organic acids. Because of the presence of basic atoms (i. e. bearing free electron pairs; mostly of alkaline nitrogen atoms) in the molecule, such acid addition salts are formed by the addition of one or more molecules of H-acid compounds (Brönstedt acids) preferably one molecule of an H-acid compound and provide an improved solubility of the molecules on polar media like for example in water. The latter characteristic is of particular impact for such compounds which develop pharmacological effect.

In preferred embodiments of the invention acid addition salts are salts of pharmaceutically acceptable acids are advantageously chosen (but without limiting the present invention) from the group consisting of hydrochlorides, trifluoroacetates, tartrates, succinates, formiates and/or citrates of the compounds of the general formula (I).

There were prepared, in particularly preferred embodiments of the invention, a number of exemplary (non-restricting) compounds of the general formula (I) which are shown and summarized in the subsequent Table 1. The compounds were prepared in accordance with well-established synthesis methods of the organic chemistry. Specific synthesis examples are described below in the experimental section of the description. However, the synthesis routes described are exemplary and given for a better understanding of the invention, only, and do not restrict the invention.

**Table1: Examples for specific compounds of the general formula (1)**

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C18.001** | 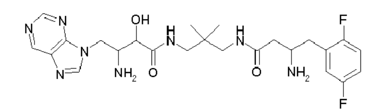 | 0.2470 | 0.9100 | 16.0000 | 1.0000 | 29.8 | | 156.37 | -10.12 | -6.46 |
| **C18_A01** | 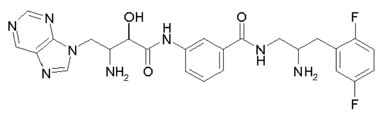 | | | | | | | | -11.32 | -9.01 |
| **C18_A02** | 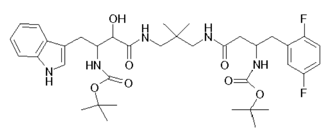 | | | | | | | | -8.99 | -6.79 |
| **C18_A03** | 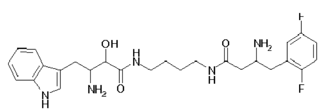 | | | | | | | | -10.28 | -7.48 |
| **C18_A04** | 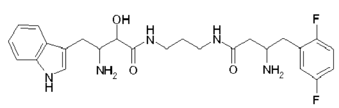 | | | | | | | | -10.37 | -7.22 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| C18_A05 | 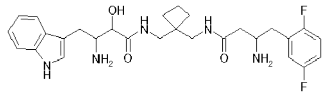 | | | | | | | | -10.53 | -7.98 |
| C18_A06 | 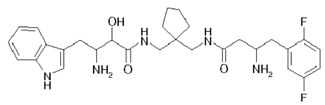 | | | | | | | | -10.15 | -7.94 |
| C18_A07 | 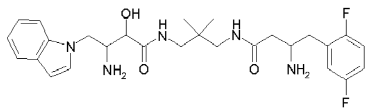 | | | | | | | | -10.04 | -8.53 |
| C18_A08 | 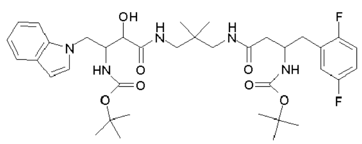 | | | | | | | | -8.63 | -9.04 |
| C18_A09 | 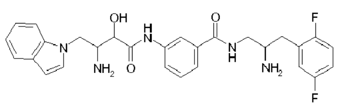 | | | | | | | | -11.32 | -8.22 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| C19.001 | 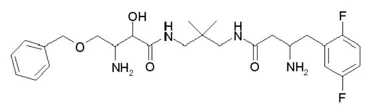 | 0.0560 | 0.0076 | 1.2500 | 0.0310 | 28.1 | 90.70 | 77.43 | -9.80 | -8.56 |
| C19_A01 | 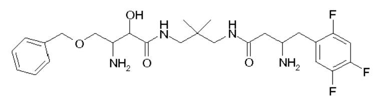 | | | | | | | | -10.30 | -8.57 |
| C19_A02 | 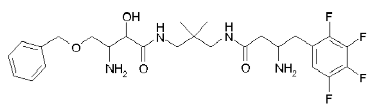 | | | | | | | | -9.75 | -8.56 |
| C19_A03 | 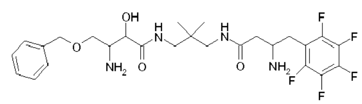 | | | | | | | | -10.15 | -8.07 |
| C19_A04 | 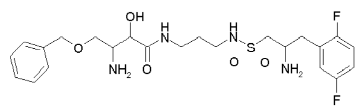 | | | | | | | | -11.63 | -9.09 |
| C19_A05 | 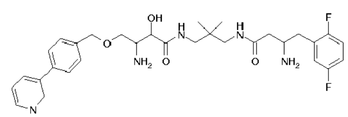 | | | | | | | | -11.05 | -9.04 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| C19_A06 | 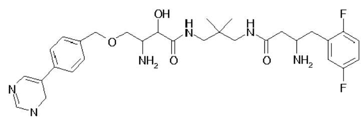 | | | | | | | | -10.91 | -7.91 |
| C19_A07 | 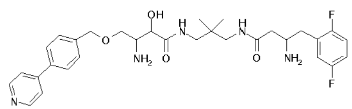 | | | | | | | | -11.05 | -8.82 |
| C19_A08 | 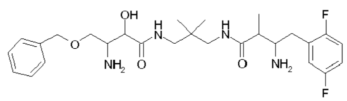 | | | | | | | | -9.94 | -7.83 |
| C19_A09 | 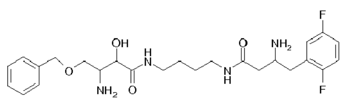 | | | | | | | | -9.80 | -7.12 |
| C19_A10 | 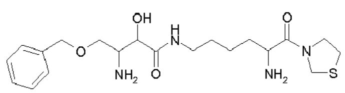 | | | | | | | | -10.60 | -8.93 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| C19_A11 | 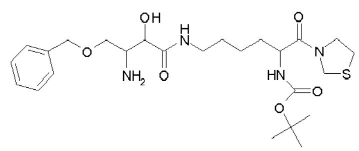 | | | | | | | | -11.01 | -8.44 |
| C19_B01 | 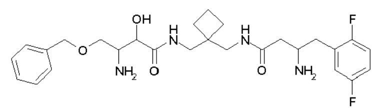 | | | | | | | | -9.81 | -7.42 |
| C19_B02 | 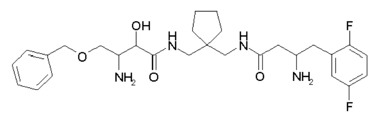 | | | | | | | | -10.67 | -8.98 |
| C19_B03 | 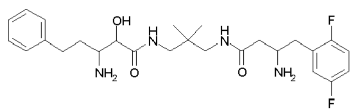 | | | | | | | | -10.71 | -8.27 |
| C19_B04 | 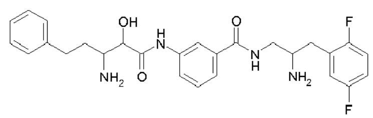 | | | | | | | | -10.91 | -8.37 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C19_B05** | 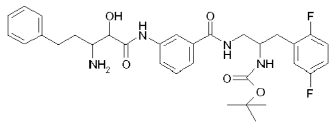 | | | | | | | | -10.39 | -8.90 |
| **C19_B06** | 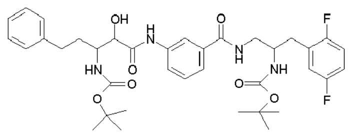 | | | | | | | | -10.80 | -8.04 |
| **C19_B07** | 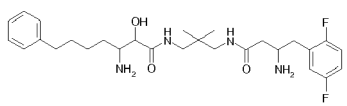 | | | | | | | | -9.87 | -8.22 |
| **C19.002 (mixture)** | 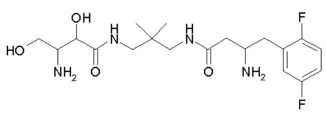 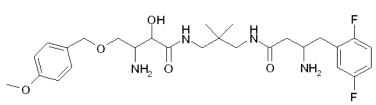 | 0.0470 | 0.1200 | 1.3000 | 0.3100 | 174.0 | | 200.00 | -9.79 | |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C19.003** | 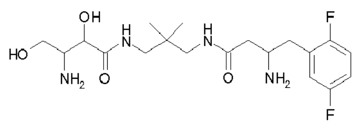 | 0.3300 | 0.9200 | 3.8000 | 0.3700 | 81.2 | | 81.43 | -9.31 | |
| **C19.004** | 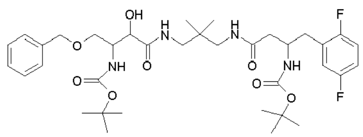 | no inhibition | no inhibition | no inhibition | no inhibition | 9.7 | 19.75 | 18.33 | -8.19 | -0.48 |
| **C19_C01** | 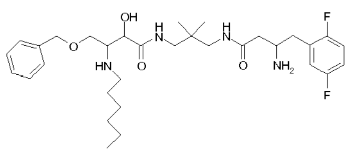 | | | | | | | | -11.12 | -7.38 |
| **C19_C02** | 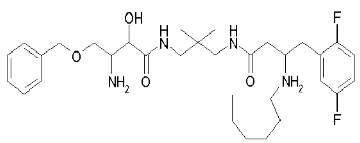 | | | | | | | | -11.05 | -7.13 |
| **C19_C03** | 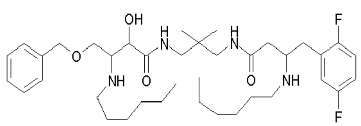 | | | | | | | | -9.83 | -6.49 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C19.005** | 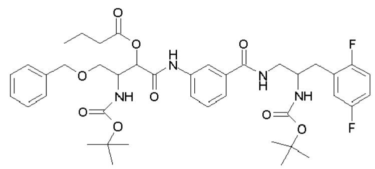 | no inhibition | no inhibition | no inhibition | 5.7000 | 11.5 | 3.65 | 10.67 | -7.33 | |
| **C19.006** | 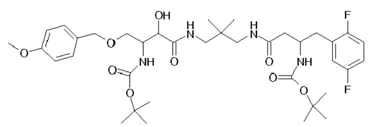 | no inhibition | no inhibition | no inhibition | 10.2000 | 6.2 | 24.13 | 18.53 | -8.43 | |
| **C19.007** | 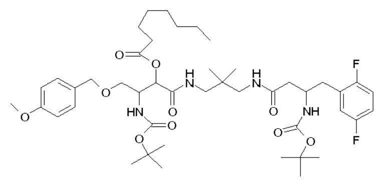 | no inhibition | no inhibition | no inhibition | no inhibition | 78.6 | 120.00 | 56.83 | -7.29 | |
| **C19.008** | 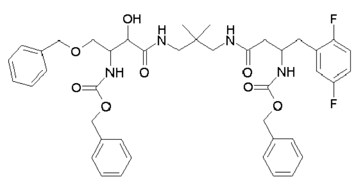 | no inhibition | no inhibition | no inhibition | 5.8000 | 5.7 | 6.77 | 8.60 | -8.69 | -6.29 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C19.009** | 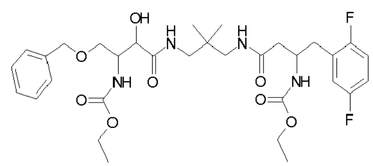 | no inhibitition | 5.9000 | no inhibitition | 3.4000 | 33.9 | 100.00 | 59.13 | -7.72 | |
| **C19.010** | 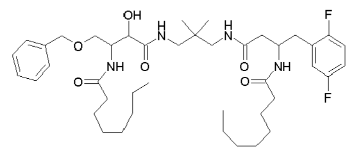 | no inhibitition | no inhibitition | no inhibitition | 3.5000 | 7.6 | 48.10 | 12.20 | -8.18 | |
| **C19.011** | 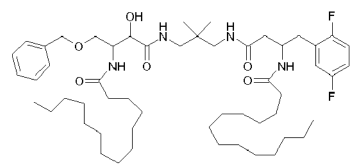 | no inhibitition | no inhibitition | no inhibitiion | no inhibitition | 167.8 | 110.00 | 154.67 | n.d. | |
| **C19.012** | 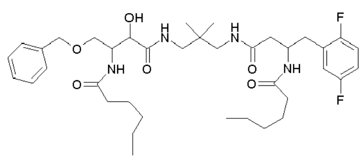 | no inhibitition | no inhibitition | no inhibitition | 0.5100 | 12.2 | 17.47 | 28.58 | -8.49 | |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol1) | $\Delta E_{CPBS}$(APN) /kcal/mol12) |
|---|---|---|---|---|---|---|---|---|---|---|
| C19.013 | 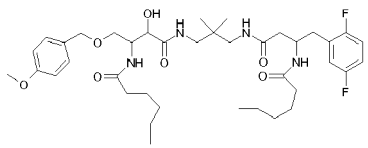 | no inhibitition | no inhibitition | no inhibitition | no inhibitition | 6.5 | 15.30 | 17.40 | -8.25 | |
| C19.014 | 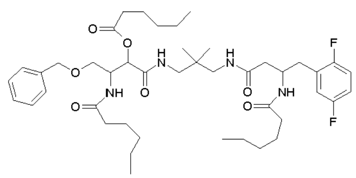 | no inhibitition | no inhibitition | no inhibitition | no inhibitition | 19.8 | 2.97 | 148.90 | -5.89 | |
| C19.015 | 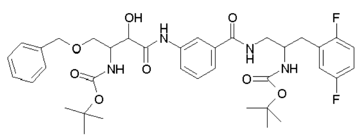 | no inhibitition | no inhibitition | no inhibitition | no inhibitition | 8.0 | 15.90 | 5.75 | -10.12 | |
| C19.016 | 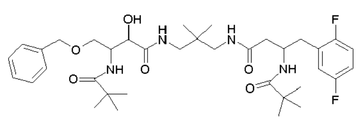 | no inhibitition | no inhibitition | no inhibitition | no inhibitition | 15.7 | 37.90 | 49.20 | -8.55 | |
| C19.017 | 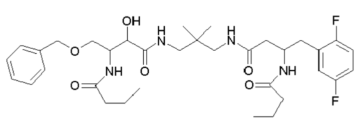 | no inhibitition | no inhibitition | no inhibitition | no inhibitition | 30.5 | 64.63 | 172.68 | -7.41 | |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C19.018** | 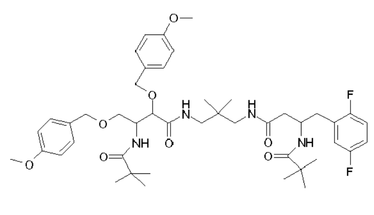 | no inhibitition | no inhibitition | no inhibitition | no inhibitition | 3.3 | 9.90 | 9.10 | -8.33 | -3.48 |
| **C19.019** | 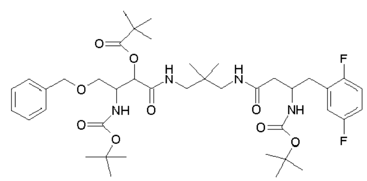 | no inhibitition | no inhibitition | no inhibitition | no inhibitition | 122.4 | 130.68 | 70.64 | -6.71 | |
| **C19.020** | 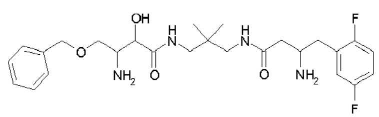 | no inhibitition | 0.1300 | 1.2000 | 0.0280 | 47.9 | 92.77 | 123.84 | -10.14 | |
| **C19.021** | 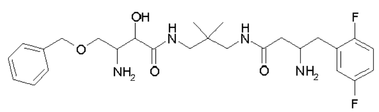 | No inhibi tion | 0.260 | 40210.0000 | 0.1400 | 58.8 | 68.60 | 105.65 | -9.66 | |
| **C19.022** | 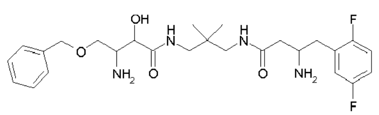 | No inhibition | 0.430 | 1.2000 | 0.3700 | 161.8 | 104.20 | 171.35 | -9.64 | |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C19.023** | 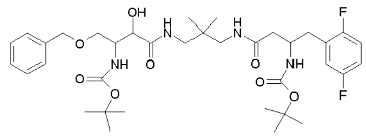 | No inhibition | no inhibition | no inhibition | no inhibition | 11.5 | 40.97 | 15.90 | n.d. | |
| **C19.024** | 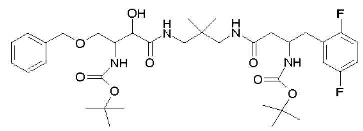 | No inhibition | no inhibition | no inhibition | no inhibition | 11.4 | 21.10 | 13.28 | n.d. | |
| **C19.025** | 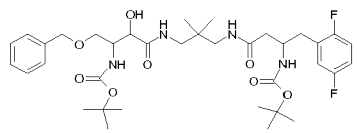 | No inhibition | no inhibition | no inhibition | no inhibition | 12.3 | 17.33 | 15.38 | n.d. | |
| **C19.026** | 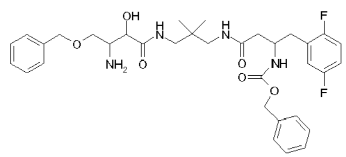 | No inhibition | 1.0000 | 7.3000 | 0.0500 | 12.2 | 8.90 | 22.73 | -10.76 | |
| **C19.027** | 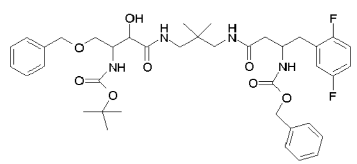 | No inhibition | no inhibition | no inhibition | no inhibition | 5.2 | 42.05 | 8.53 | -9.32 | |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C19.028** | 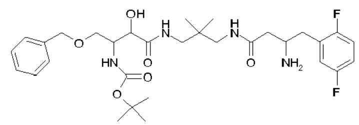 | 0.1700 | no inhibition | 1.4000 | no inhibition | 9.0 | 31.40 | 49.40 | -10.63 | |
| **C19.029** | 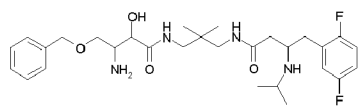 | 14.5000 | 0.5900 | 24.3000 | 0.3300 | 29.4 | 34.80 | 92.65 | -13.64 | |
| **C19.030** | 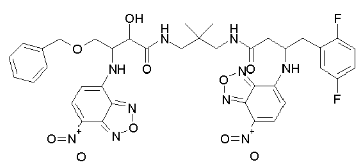 | No inhibition | no inhibition | no inhibition | no inhibition | 18.2 | 7.55 | 29.73 | -9.23 | |
| **C19.031** | 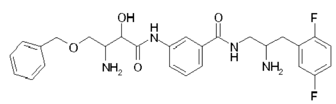 | 2.3000 | 0.0120 | 13.3000 | 0.1800 | 58.2 | 20.10 | 119.23 | -11.32 | |
| **C19_D01** | 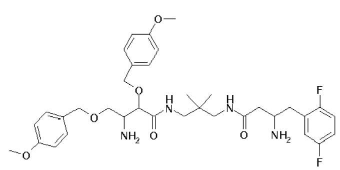 | | | | | | | | -11.87 | -8.11 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| C19_D02 | 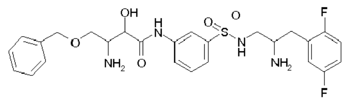 | | | | | | | | -13.64 | -8.46 |
| C20_A01 | 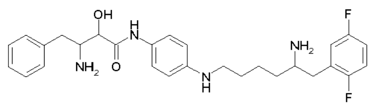 | | | | | | | | -9.03 | -8.58 |
| C20_A02 | 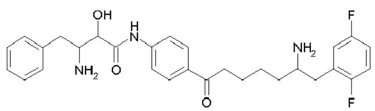 | | | | | | | | -10.08 | -8.50 |
| C20_A03 | 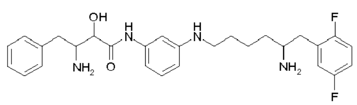 | | | | | | | | -10.53 | -8.45 |
| C20_A04 | 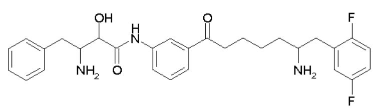 | | | | | | | | -10.67 | -9.62 |
| C20_A05 | 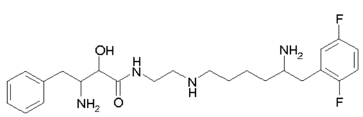 | | | | | | | | -11.67 | -7.63 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| C20_A06 | 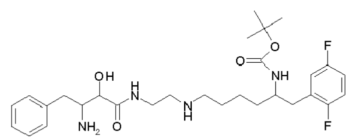 | | | | | | | | -11.34 | 7.64 |
| C20_A07 | 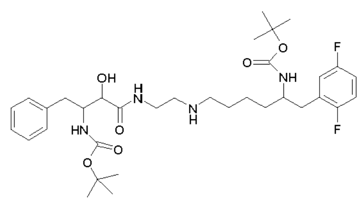 | | | | | | | | -9.16 | -6.84 |
| C21.001 | 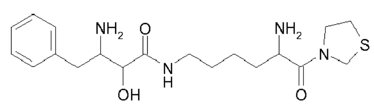 | 0.11 | 0.178 | 0.02 | 0.066 | 90.1 | 77.00 | 65.50 | -10.32 | |
| C21_A01 | 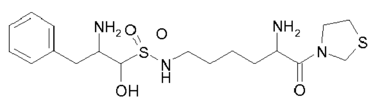 | | | | | | | | -15.72 | -7.37 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C21.002** | 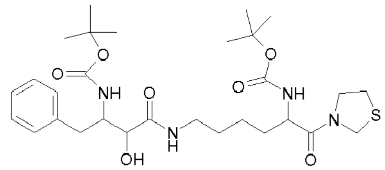 | no inhibition | no inhibition | no inhibition | no inhibition | 55.1 | 49.30 | 61.60 | -11.32 | |
| **C21.003** | 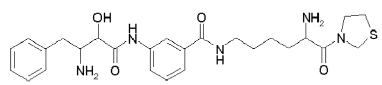 | 0.07 | 0.62 | 0.054 | 0.13 | 156.7 | 62.00 | 200.00 | -11.32 | |
| **C21.004** | 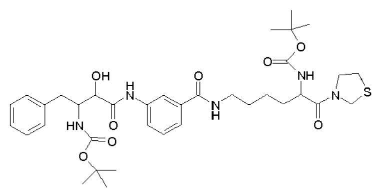 | no inhibition | no inhibition | no inhibition | no inhibition | 12.0 | 13.90 | 19.40 | n.d. | |
| **C21.005** | 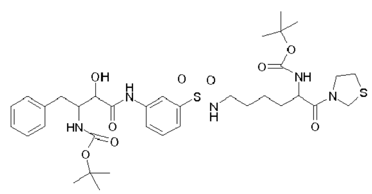 | no inhibition | no inhibition | no inhibition | no inhibition | 11.4 | 15.30 | | n.d. | |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C21.006** | 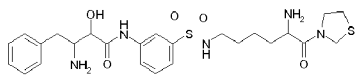 | 0.05 | 0.89 | 0.11 | 0.12 | 156.7 | 66.15 | | -16.45 | |
| **C21_B1** | 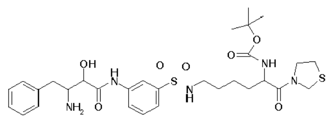 | | | | | | | | -8.62 | -9.76 |
| **C21_B2** | 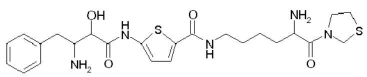 | | | | | | | | -10.28 | -9.29 |
| **C21_B3** | 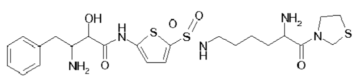 | | | | | | | | -11.77 | -10.35 |
| **C22.001** | 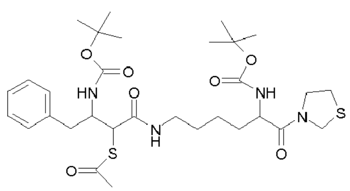 | no inhibition | no inhibition | no inhibition | 0.57 | 136.3 | 200.00 | 57.10 | n.d. | |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol1) | $\Delta E_{CPBS}$(APN) /kcal/mol12) |
|---|---|---|---|---|---|---|---|---|---|---|
| C22.002 | 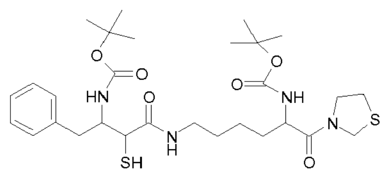 | no inhibiion | no inhibition | no inhibition | 1 | 54.1 | 85.20 | 200.00 | n.d. | |
| C22.003 | 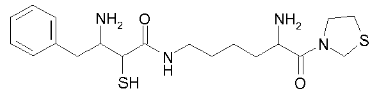 | 0.032 | 3.79 | 0.34 | 3.5 | 30.2 | 38.90 | 75.20 | -10.21 | |
| C22_B01 | 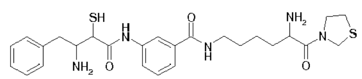 | | | | | | | | -10.00 | -10.67 |
| C22_B02 | 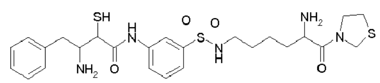 | | | | | | | | -11.63 | -10.90 |
| C26.001 | 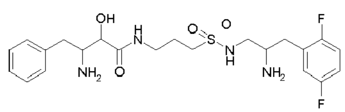 | 0.58 | 0.07 | 7.37 | 0.09 | 200.0 | 135.43 | | -10.91 | |
| C26_A01 | 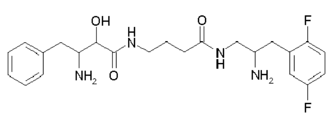 | | | | | | | | -10.50 | -7.77 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol1) | $\Delta E_{CPBS}$(APN) /kcal/mol12) |
|---|---|---|---|---|---|---|---|---|---|---|
| **C26_B01** | 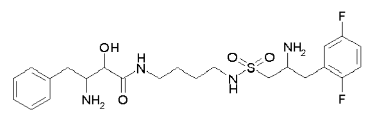 | | | | | | | | -10.50 | -9.77 |
| **C26_B02** | 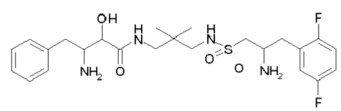 | | | | | | | | -14.14 | -9.74 |
| **C26_B03** | 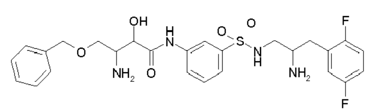 | | | | | | | | -14.08 | -9.92 |
| **C26.002** | 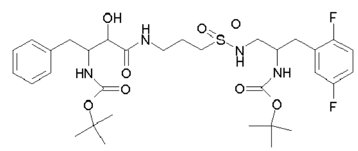 | 6.52 | no inhibition | no inhibition | no inhibition | 200.0 | 18.07 | | -8.57 | |
| **C26_C01** | 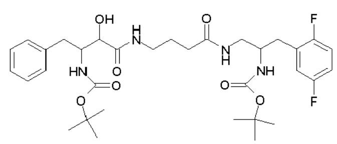 | | | | | | | | -9.68 | -6.74 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol1) | $\Delta E_{CPBS}$(APN) /kcal/mol12) |
|---|---|---|---|---|---|---|---|---|---|---|
| **C26.003** | 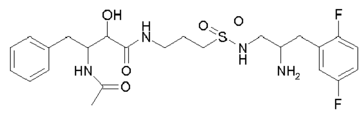 | 0.36 | 0.19 | no inhibition | 0.25 | 70.0 | 167.33 | | -10.37 | |
| **C26_C02** | 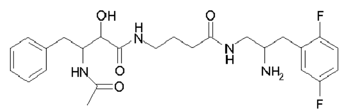 | | | | | | | | -10.91 | -6.22 |
| **C26.004** | 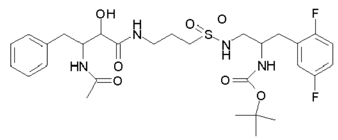 | no inhibition | no inhibition | no inhibition | no inhibition | 70.0 | 105.80 | | -9.57 | |
| **C26.005** | 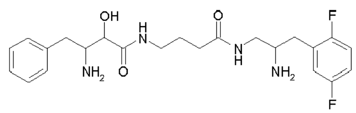 | no inhibition | 0.09 | not determined | 0.13 | 27.3 | 28.90 | | -10.50 | -7.45 |
| **C26.006** | 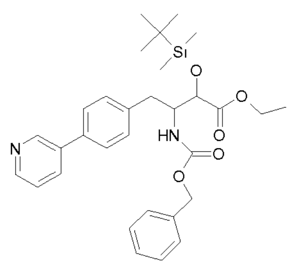 | no inhibition | no inhibition | not determined | no inhibition | 3.8 | 1.90 | 7.90 | -9.68 | -7.33 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| C26.007 | 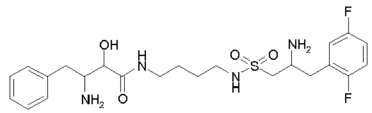 | no inhibition | no inhibition | | | 3.5 | | 7.3 | -10.50 | -5.13 |
| C26.008 | 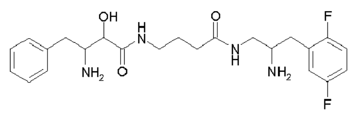 | 0.90 | 0.01 | not determined | not determined | | | | -10.50 | |
| C26.009 | 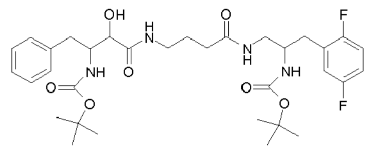 | no inhibition | no inhibition | not determined | not determined | | | | -9.68 | |
| C-122 | 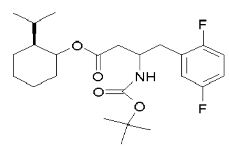 | no inhibition | no inhibition | no inhibition | no inhibition | 5.83 | 69.4 | 65.2 | -9.21 | -5.63 |

(continued)

| Compound | Structure | meas. IC50 (DPIV) /μM | meas. IC50(APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$(APN) /kcal/mol[12)] |
|---|---|---|---|---|---|---|---|---|---|---|
| **C-125** | 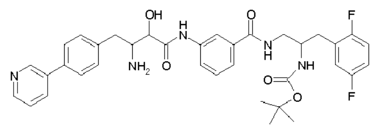 | no inhibition | 0.094 | not determined | 0.005 | 12.90 | 4.4 | 13.73 | -16.37 | -8.79 |

1) Free Energy of the interaction between the ligand and the respective central pore binding site

2) In case of missing data for the APN central pore binding site experimental values characterize the interaction.

### 1. Enzymatic measurements

### 1.1. Measurement of enzymatic activity and inhibition of DPIV and related enzymes

The inhibition of the enzymatic activity of DPIV and related enzymes was measured by using purified, recombinant human DPIV (purified from transfected Sf9 cells, final enzyme concentration approx. 1 nM) as well as purified, recombinant DP8 and DP9 (purchased from Biomol). The assay was performed in 0.05M TRIS/HCl puffer pH 7.5, supplemented with 0.05% Triton (v/v), 0.05% BSA (w/v), 2mM $MgCl_2$.

The enzymatic activity of DPIV was assessed by the hydrolysis of the fluorogenic substrates H-Gly-Pro-4-amino-7-methyl coumarine (abbreviation of fluorogenic group AMC; purchased from Bachem) or $(Ala-Pro)_2$-Rhodamine 110 (abbreviation R110). The final substrate concentrations were 50μM or 1μM, respectively.

The assay was performed in white microtitre plates for fluorescence measurements. The test items, substrate and enzyme were diluted in assay buffer. The highest test item concentration used was 25μM. For the calculation of IC50 values, at least 16 log2-dilutions of each test item were analyzed. As controls, the DPIV activity in the absence of test items as well as the spontaneous hydrolysis of the substrate was determined.

The release of the fluorescent hydrolysis product AMC was measured at an excitation wavelength of 380 nm and an emission wavelength of 460 nm by using a microtitre fluorescence reader immediately after substrate addition as well as after 30, 60 and 120min. The hydrolysis of the substrate $(Ala-Pro)_2$-Rhodamine 110 was determined at an exitation wavelength of 488 nm and emission wavelength of 530 nm.

### 1.2. Measurement of enzymatic activity and inhibition of aminopeptidase

The inhibition of the enzymatic activity of aminopeptidase N (APN) was measured by using purified, recombinant human enzyme (purified from transfected ECV cells, final enzyme concentration approx. 1 nM). The cytosolic aminopeptidase (cAAP) was purified from Jurkat cells, which do not express APN. The assay was performed in 0.05M TRIS/HCl puffer pH 7.5, supplemented with 0.05% Triton (v/v), 0.05% BSA (w/v), 2mM $MgCl_2$.

The aminopeptidase acitivities wer assessed by the hydrolysis of the fluorogenic substrates H-Ala-4-amino-7-methyl coumarine (abbreviation of fluorogenic group AMC; purchased from Bachem) or $(Ala-)_2$-Rhodamine 110 (abbreviation R110). The final substrate concentrations were again 50 and 1μM, respectively.

The assay was performed in white microtitre plates for fluorescence measurements. The test items, substrate and enzyme were diluted in assay buffer. The highest test item concentration used was 25μM. For the calculation of IC50 values, at least 16 log2-dilutions of each test item were analyzed. As controls, the DPIV activity in the absence of test items as well as the spontaneous hydrolysis of the substrate was determined.

The release of the fluorescent hydrolysis products AMC and R110 was measured as described above for DPIV substrates.

### 2. Evaluation of test item effects on DNA synthesis in different cell types

### 2.1. Inhibition of proliferation of human peripheral blood mononuclear cells and T lymphocytes

Peripheral blood mononuclear cells (PBMC) from healthy human volunteers were freshly isolated by density gradient centrifugation. T lymphocytes (T cells) were isolated from PBMC fractions via nylon wool adherence. Both cell populations were cultured in serum free lymphocyte medium (AIMV medium, Invitrogen) and stimulated by addition of 1μg/ml Phytohemagglutinine for 48 hours in 96 well flat-bottom microtiter plates. Test compounds were added at concentration ranges from 0.1 up to 250μM over the total assay period.

DNA synthesis of proliferating PBMC was assessed by incorporation of nucleotide analogue bromdesoxyuridine (BrdU) and subsequent detection of BrdU by ELISA technique (Cell Proliferation Biotrak™ ELISA system, GE Health Care) according to the protocol of the manufacturer. DNA synthesis of proliferating T cells was determined by incorporation of radio labelled tritium thymidine and subsequent radio detection.

Data output was based on raw data and calculation of relative proliferation response in relation to PHA-activated T cells in the absence of test compound (100% control). The IC50 value of proliferation suppression was assessed by graphical evaluation.

### 2.2. Inhibition of the DNA synthesis and proliferation of Normal Human Epidermal Keratinocytes (NHEK)

NHEK cells (primary cells from 30 years old caucasian female) were purchased from PromoCell. For these assay the adherent cells were cultured in serum-free Keratinocyte Growth medium 2 supplemented with epidermal growth factor (EGF) and bovine pituitary extract for 48hours in flat-bottom microtiter plates. EGF stimulates the DNA

synthesis and proliferation of these cells. Test items were added in concentrations from 0.1 up to 100μM.

The DNA synthesis was assessed by incorporation of bromdesoxyuridine (BrdU) and subsequent detection of BrdU by ELISA technique. Based on these raw data the relative proliferation response in relation to cells cultured in the absence of test compound (100% control) was calculated. IC50 values of proliferation suppression were assessed by computer-assisted graphical evaluation.

### 2.3. Inhibition of the DNA synthesis and proliferation of immortalized human sebocytes

The immortalized human sebocyte line SZ95 was provided by Prof. C Zouboulis [CC Zouboulis, H Seltmann, H Neitzel, CE Orfanos, Establishment and characterization of an immortalized human sebaceous gland cell line (SZ95). J. Invest. Dermatol. 113(6):1011 -1020 (1999)]. These adherent growing cells were cultured in serum-free Sebomed Complete (Biochrom). Recombinant EGF was added in order to stimulate DNA synthesis and proliferation of the SZ95 cells. Test items were added in concentrations from 0.1 up to 100 μM.

The DNA synthesis of proliferating cells was detected by incorporation of radiolabelled tritium thymidine (3HT) and counting at a beta counter. Based on these raw data the relative proliferation response in relation to cells cultured in the absence of test compound (100% control) was calculated. IC50 values of proliferation suppression were assessed by computer-assisted graphical evaluation.

### 3. Computational analyses

All enzymatic data is related to the DPIV crystal structure, solved by H. B. Rasmussen et al., 2003.

The in detail study of the protein surface round the active site pocket revealed a shallow dell within the central pore (tunnel) between the active site pocket and the central access. Several docking runs with known DPIV inhibitors, that include the region round both, the active site pocket and the shallow dell discussed here, resulted in binding constants for the shallow dell region within one or two orders of magnitude of those obtained for the binding of the respective inhibitor within the active site pocket. Restriction of the region considered in the docking procedures and application on a wide range of compounds proved that this shallow dell is a possible alternative binding site. This binding site is named central pore binding site.

For geometric studies, the active site pocket is represented by *GLU205, GLU206,* and *ARG358,* the central pore binding site *GLU361, HIS363* and *GLU408.*

Two critical distances are included in this model. The first distance for characterizing the position of small molecules within the central pore binding site towards the active site pocket is that between the carboxyl carbon of *ARG206* and the centre of coordinates of the inhibitor/ligand ($r_{as}$). The second one is that between the centre access point of the DPIV central pore and the centre of mass of the inhibitor/ligand ($r_{cpbs}$) that characterizes the position of the inhibitor/ligand towards the entrance of the central pore. The *van der Waals* surface $A_{vdW}$ of the inhibitor/ligand represents the obstruction of the DPIV central access pore. Furthermore, short polar contacts between the inhibitors/ligands and at least two of the residues that represent the central pore binding site are crucial for stable thermodynamic interactions.

### 4. Molecular docking and quantum chemical calculations

The docking studies were carried out with the Autodock 4 package [www.Scripps.edu] on a 4 CPU computer system under Windows XP.

For the docking procedures, we used the A-monomer of the dimeric DPIV crystal structure. The water molecules within the region of the monomer considered here were removed before. For all dockings, 256 generic algorithm runs were carried out.

The ligand/inhibitor molecules were pre-optimized using the force field procedure implemented in the Chem-Sketch software package [www.acdlabs.com]. LogP values were calculated with the method implemented as optional add -on in the ChemSketch software package [www.acdlabs.com].

Molecular volumes and surfaces were obtained by using the COSMO method [A. Klamt et al., Journal of the Chemical Society, Perkin Transaction 2, 799 (1993)], that is implemented in *MOPAC2002* [http://www.cache.fujitsu.com/mopac/index.shtml].

### 5. Multilinear Regression Analysis as model of T cell proliferation inhibition

We developed a three descriptor linear regression analysis of the T cell proliferation suppression according the equation:

$$\mathrm{Ln[calc.\ DNA\text{-}Supp.]} = c_0 + c_1[\mathrm{Ln}[K_{CPBS}] + c_2[r^2_{AS} - r^2_{CPBS}] + c_3[N_A]$$

The following abbreviations were used:

DNA-Supp. is the $IC_{50}$ of the suppression of T-cell DNA-synthesis;
$K_{CPBS}$ is the apparent affinity constant of the central pore binding site;
AS means active site;
CPBS stands for central pore binding site;
$r^2_{AS}$-$r^2_{CPBS}$ is the difference between the squared distance of the central pore binding site and the active site and the squared distance between the central pore binding site and the access site.

The active site is defined by the coordinates of the carboxyl carbon atom of the Glu206 residue, the central pore binding site by the coordinate centre of the ligand bound to the central pore binding site near His363 and the access site by the approximate central point of the access of the central pore near Glu464.

The most expedient distance related parameter for the model discussed here is the difference of the two squared distances considered here, $r_{as}^2$ - $r_{CPBS}^2$. Their representation in squared shape seems necessary to avoid the occurrence of equal differences for different positions, as it would be probable for simple differences of distances. $N_A$ is the number of amino groups. $c_0$, $c_1$, $c_2$ and $c_3$ are the linear coefficients determined by the regression calculation according to the least square method.

Due to its thermodynamic and kinetic background, the regression equation introduced above uses the natural logarithms of DNA-Supp and $K_{CPBS}$. Their values are given in μM). The calibration data set contains an overall number of 41 structurally diverse chemical compounds with DNA-Supp values that range from 3.23 to 200. The correlation coefficient R is 0.8213, the standard deviation (logarithmic) is 0.7647.

The basic principles of the interaction between the novel compounds of the general formula (I) according to the present invention and the exemplary enzymes dipeptidylpeptidase IV (DPIV) and aminopeptidase N (APN) are shown in the accompanying Figures, wherein

Figure 1 is a sketch showing the essential distances (in Angström) in the monomer molecules of DPIV and APN; and

Figures 2a to 2d are drawings showing the binding of ligands to the central pore binding sites of DPIV and APN.

Figure 2 specifically shows a binding of ligands at the central pore binding sites of DPIV and APN. The Figures show the following:

a): DPIV with Sitagliptin (dark space filling presentation) in the active site;
b): DPIV with Sitagliptin (dark space filling presentation) in the active site and C26.001 binding in the central pore binding site (light grey space filling presentation); blocking the access to the active site;
c): APN with Bestatin (dark space filling presentation) in the active site;
d): APN with Bestatin (dark space filling presentation) in the active site and C26.001 binding in the central pore binding site (light grey space filling presentation); blocking the access to the active site.

In accordance with the present invention, the compounds of the above general formula (I) in general, or the afore-mentioned compounds in accordance with the Table 1, are compounds for a use in the medical field. The term "for a use in the medical field" is understood in the present specification and claims in the broadest sense as for a use of one compound of the above general formula (I), or of two or more of the compounds of the general formula (I), in general or of the afore-mentioned compounds in accordance with the Table 1, for any use in the medical field, e. g. as effectors on substances having a medical effect in a body, preferably a mammalian body, more preferably on the body of a human, as substances having a medical effect themselves due to their action on a body, preferably a mammalian body, even more preferred a human body in need of such a substance against a disease or pathological condition, as components of a medicament or of a pharmaceutically effective preparation exerting an effect on the body, preferably the body of a mammal, more preferably on the body of a human in need of such a medicament or pharmaceutically effective preparation against a disease or pathological condition or as components of a diagnostic preparation exerting an effect on, or seeking for an effect in, the body, preferably the body of a mammal, more preferably on the body of a human to which such a diagnostically effective preparation against a disease or pathological condition is administered or applied. Examples (which do not restrict the invention) of a use in the medical field are a treatment of a disease or

pathological condition, a prophylaxis of becoming affected by a disease or pathological condition, the improvement of a body in its status of recovering from a disease or pathological condition, a prevention of a body from becoming affected another time by a disease or pathologic condition of which the body had suffered earlier, a diagnostic procedure for testing a body for its status of being protected against, of becoming affected by or of being in recovery from, a disease or pathological condition, to name only a few examples.

All compounds of the above general formula (I) in general, or all the afore-mentioned compounds in accordance with the Table 1, were found to be suitable for a use in the medical field. For a suitability of any one or more of the compounds of the general formula (I) in general or of the afore-mentioned compounds in accordance with the Table 1, for being used in the medical field, the specific mechanism by which the respective compound(s) work(s) may be a mechanism proposed by the present specification. However, the mechanism of action described in the specification is not to be interpreted as a restriction of the invention, but has only exemplary character and represents the best mode of the invention at the time the invention was made, but may be another (optionally related) mechanism than the one described above.

Specifically, the compounds of the above general formula (I) in general, or the afore-mentioned compounds in accordance with the Table 1, are characterized by their capability of enzymatically inhibiting DPIV and APN, by suppressing the DNA synthesis (proliferation) of T lymphocytes, keratinocytes and sebocytes and by binding to the central pore binding sites and blocking the access of substrates to the active sites of cellular APN and DPIV. This was defined by docking approaches using the crystal structures of both peptidases and of the compounds of the general formula (I) in general, or of the afore-mentioned compounds in accordance with the Table 1.

More specifically, the compounds of the above general formula (I) in general, or the afore-mentioned compounds in accordance with the Table 1, are for use as dual inhibitors or central pore binding ligands of dipeptidyl peptidase IV and of peptidases with DPIV-analogous enzymatic effect and of alanyl aminopeptidase N (APN) and of peptidases with APN-analogous enzymatic effect. Even more specifically, the compounds of the above general formula (I) are for use in the suppression of DNA synthesis and inflammatory cytokine production as well as in the stimulation of anti-inflammatory cytokine production in vitro and in vivo.

It was surprisingly found by the present inventors that the compounds of the general formula (I) in general, or the afore-mentioned compounds in accordance with the Table 1, in accordance with the general principles of an inhibition of ectopeptidases selected from dipeptidyl peptidase IV and of peptidases with analogous enzymatic effect and of alanyl aminopeptidase N (APN) and of peptidases with analogous enzymatic effect are suitable for use for the prophylaxis and therapy of autoimmune diseases, of diseases with exceeding immune response and/or inflammatory genesis, including arteriosclerosis, neuronal diseases, cerebral damages, skin diseases, tumour diseases, transplant rejection, Graft-versus-Host Diseases (GvHD) and virus- or bacteria-caused diseases.

Moreover, in preferred embodiments of the invention, the compounds of the general formula (I) in general, or the afore-mentioned compounds in accordance with the Table 1, are suitable for use for the prophylaxis and therapy of diseases or conditions selected from multiple sclerosis, morbus Crohn, colitis ulcerosa, diabetes mellitus Typ 1, rheumatoide arthritis, arteriosclerosis, arterial inflammation, stent-restenosis and other autoimmune diseases as well as inflammatory diseases.

In further preferred embodiments of the invention, compounds of the general formula (I) in general, or the afore-mentioned compounds in accordance with the Table 1, are for use in the prevention and therapy of tumours as well as of metastases.

Further preferred embodiments of the invention are directed to compounds of the general formula (I) in general, or to the afore-mentioned compounds in accordance with the Table 1, for use in the prevention and treatment of diseases or conditions selected from skin- and mucosa-related diseases, psoriasis, acne as well as dermatological diseases with hyper-proliferation and modified conditions of differentiation of fibroblasts, benign fibrosing and sclerosing skin diseases and malign fibroblastic conditions of hyper-proliferation.

In further preferred embodiments of the invention, the compounds of the general formula (I) in general, or the afore-mentioned compounds in accordance with the Table 1, are for use in the prevention and treatment of diseases and conditions of asthma bronchiale and of other allergic diseases as well as of chronic obstructive pulmonary disease (COPD).

The present invention comprises as further preferred embodiments compounds of the general formula (I) in general, or the afore-mentioned compounds in accordance with the Table 1, for use in the treatment and prevention of diseases and conditions selected from acute neuronal diseases, ischemia-caused cerebral damages after an ischemia- or haemorrhagic apoplexia, cranio-cerebral injury, cardiac arrest, heart attack or as a consequence of cardio surgical intervention, of chronic neuronal diseases for example of Morbus Alzheimer, of the Pick-disease, a progressive supra-nuclear palsy, the corticobasal degeneration, the frontotemporal dementia, of Morbus Parkinson, especially parkinsonism coupled to chromosome number 17, of Morbus Huntington, of prion-caused conditions or diseases and amyotrophic lateral sclerosis.

Furthermore, the invention comprises in further preferred embodiments compounds of the general formula (I)

in general, or the afore-mentioned compounds in accordance with the Table 1, for use in the treatment and prevention/prophylaxis of a rejection of allogene or xenogene transplanted organs, of tissues and cells such as bone marrow, kidney-, heart-, liver- pancreas-, skin- or stem cells, and stents, of joint implants (knee joint implants, hip joint implants), bone implants, cardiac pace makers or other implants, vessel balloons, as well as Graft-versus-Host Diseases (GvHD).

Moreover, further preferred embodiments of the invention comprise compounds of the general formula (I) in general, or the afore-mentioned compounds in accordance with the Table 1, for use in the prevention/prophylaxis and treatment of diseases or conditions selected from inflammatory infectious diseases such as malaria, severe acute respiratory syndrome (SARS), and of sepsis and sepsis-like conditions.

Compounds of the general formula (I) in general, or the afore-mentioned compounds in accordance with the Table 1 according to the invention, may be used, as synthesized or after a suitable purification known to a person skiled in the art of applying chemical compounds in the medical field, alone as one compound or as two or even more compounds. The compounds of the general formula (I) in general, or the afore-mentioned compounds in accordance with the Table 1, may be used alone or, alternatively, may be used in combination with one or more pharmaceutically acceptable carrier(s), auxiliary substance(s) and/or adjuvant(s). One pharmaceutically acceptable carrier and/or auxiliary substance and/or adjuvant may be used, or two or even more pharmaceutically acceptable carriers and/or auxiliary substances and/or adjuvants may be used in accordance with the invention. Such pharmaceutically acceptable carriers, auxiliary substances and/or adjuvants are generally known in the medical field and need no detailed description here. In addition, reference may be made to standard textbooks dealing with carriers, auxiliary substances and/or adjuvants suitable for a use in the medical field, and one of these is "Remington, The Science and Practice of Pharmacy; Lippincott, Williams & Wilking (editors), 2000".

In another embodiment, the invention also relates to pharmaceutical preparations comprising at least one of the compounds of the general formula (I) according to the above detailed definition and description in general, or at least one of the afore-mentioned compounds in accordance with the Table 1. In the present invention and claims, the term "pharmaceutical preparation" is considered to mean a preparation containing one or two or even more substances having any pharmaceutical effect on a body, specifically on a mammalian body and more preferably on the body of a human, which preparation may be administered, on whatever route, to said body, if in need of such a substance or such a preparation with the aim of exerting a pharmaceutical effect. Pharmaceutical preparations of the present invention may comprise one compound of the general formula (I), or may comprise two or even more compounds of the general formula (I), as pharmaceutically effective substances. Preferred are pharmaceutical preparations comprising one compound of the general formula (I) in general, or one of the afore-mentioned compounds in accordance with the Table 1 according to the invention.

In a further embodiment of the invention which may result into specific pharmaceutical effects, a pharmaceutical preparation of the invention may comprise, in addition to at least one compound of the general formula (I) in general, or in addition to at least one of the afore-mentioned compounds in accordance with the Table 1, at least one, preferably one, further pharmaceutically effective compound. Such further pharmaceutically effective compound(s) may have a pharmaceutical effect (or may have several pharmaceutical effects) on the same field as the present compounds of the general formula (I) defined above or may have one (or several) pharmaceutical effect(s) on one field or on several fields different from the field where the above compounds of the above general formula (I) exert their pharmaceutical effect.

In the pharmaceutical preparations of the present invention, the compound or the compounds of the general formula (I) in general, or the afore-mentioned compound/compounds in accordance with the Table 1, may be used alone or, alternatively, may be used in combination with one or more pharmaceutically acceptable carrier(s), auxiliary substance(s) and/or adjuvant(s). One pharmaceutically acceptable carrier and/or auxiliary substance and/or adjuvant may be used in such pharmaceutical preparations of the invention, or two or even more pharmaceutically acceptable carriers and/or auxiliary substances and/or adjuvants may be used in accordance with the invention. Such pharmaceutically acceptable carriers, auxiliary substances and/or adjuvants to be used in the pharmaceutical preparations of the invention are generally known in the medical field and need no detailed description here. In addition, reference may be made to standard textbooks dealing with carriers, auxiliary substances and/or adjuvants suitable for a use in the medical or pharmaceutical field, and one of these is "Remington, The Science and Practice of Pharmacy; Lippincott, Williams & Wilking (editors), 2000".

The pharmaceutical preparations of the present invention comprising at least one compound of the above general formula (I) in general, or comprising at least one of the afore-mentioned compounds in accordance with the Table 1, may, for example, be preparations which are for an administration on a topical route in the form of for example crèmes, ointments, pastes, gels, solutions, sprays, liposomes and nanosomes, shake mixtures, "pegylated" formulations, degradable (e.g. degradable under physiological conditions) depot matrices, hydrocolloid-bandages, plasters, micro-sponges, prepolymers and similar carrier substrates, jet-injection or other dermatological principles/vehicles including instillative application. Alternatively, the pharmaceutical preparations may be for a systemic administration on either of oral, transdermal, intravenous, subcutane, intracutane, intramuscular, intrathecal routes, which may occur in suitable formulations or suitable galenic forms as, for example, in the form of tablets, dragees, lozenges, capsules, aerosols,

sprays, solutions, emulsions and suspensions.

In accordance with the present invention and in preferred embodiments thereof, the amounts of at least one of the compounds of the general formula (I) in general, or of at least one the afore-mentioned compounds in accordance with the Table 1, in the pharmaceutical preparations of the invention may be widely selected, without imposing any restriction to the skilled practitioner. I will be particularly be possible that, in accordance with usual parameters as, for example parameters depending on the person to be treated, the disease status of said person, the severeness of the disease or condition, and other usual parameters, the amounts to be administered may easily be determined by a skilled person in the medical field by conducting only a few orienting experiments. Specifically, the amounts may be (without restricting the invention to those amounts) in the range of 0.01 to 1000 mg with regard to at least one of the compounds of the general formula (I) in general, or to at least one of the afore-mentioned compounds in accordance with the Table 1, per application unit, preferably in the range of 0.1 to 100 mg per application unit.

In another embodiment, the invention also relates to cosmetic preparations comprising at least one of the compounds of the general formula (I) according to the above detailed definition and description in general, or at least one of the afore-mentioned compounds in accordance with the Table 1. In the present invention and claims, the term "cosmetic preparation" is considered to mean a preparation containing one or two or even more substances having any cosmetic effect on a body, specifically on a mammalian body and more preferably on the body of a human, which preparation may be applied, on whatever route, to said body, if the application of such a substance or such a preparation is desired with the aim of exerting a cosmetic effect. Cosmetic preparations of the present invention may comprise one compound of the general formula (I), or may comprise two or even more compounds of the general formula (I), as cosmetically effective substances. Preferred are cosmetic preparations comprising one compound of the general formula (I) in general, or one of the afore-mentioned compounds in accordance with the Table 1 according to the invention.

In a further embodiment of the invention which may result into specific cosmetic effects, a cosmetic preparation of the invention may comprise, in addition to at least one compound of the general formula (I) in general, or in addition to at least one of the afore-mentioned compounds in accordance with the Table 1, at least one, preferably one, further cosmetically effective compound(s). Such further cosmetically effective compound(s) may have a cosmetic effect (or may have several cosmetic effects) on the same field as the present compounds of the general formula (I) defined above or may have one (or several) cosmetic effect(s) on one field or on several fields different from the field where the above compounds of the above general formula (I) exert their cosmetic effect.

In the cosmetic preparations of the present invention, the compound or the compounds of the general formula (I) in general, or the afore-mentioned compound/compounds in accordance with the Table 1, may be used alone or, alternatively, may be used in combination with one or more cosmetically acceptable carrier(s), auxiliary substance(s) and/or adjuvant(s). One cosmetically acceptable carrier and/or auxiliary substance and/or adjuvant may be used in such cosmetic preparations of the invention, or two or even more cosmetically acceptable carriers and/or auxiliary substances and/or adjuvants may be used in accordance with the invention. Such cosmetically acceptable carriers, auxiliary substances and/or adjuvants to be used in the cosmetic preparations of the invention are generally known in the cosmetic field and need no detailed description here. In addition, reference may be made to standard textbooks dealing with carriers, auxiliary substances and/or adjuvants suitable for a use in the cosmetuc field, and one of these is "G.A. Nowak, Die kosmetischen Präparate, Band 2: Die kosmetischen Präparate - Rezepturen, Rohstoffe, wissenschaftliche Grundlagen, Verlag für Chemische Industrie H. Ziolkowsky KG, Augsburg".

The cosmetic preparations of the present invention comprising at least one compound of the above general formula (I) in general, or comprising at least one of the afore-mentioned compounds in accordance with the Table 1, may, for example, be preparations which are for an application on a topical route in the form of for example crèmes, ointments, pastes, gels, solutions, sprays, liposomes and nanosomes, shake mixtures, "pegylated" formulations, degradable (e.g. degradable under physiological conditions) depot-matrices, hydrocolloid-bandages, plasters, microsponges, prepolymers and similar carrier substrates, jet-injection or other dermatological principles/vehicles including instillative application. Alternatively, the cosmetic preparations may be for a systemic administration on either of oral, transdermal, intravenous, subcutane, intracutane, intramuscular, intrathecal routes, which may occur in suitable formulations or suitable galenic forms as, for example, in the form of tablets, dragees, lozenges, capsules, aerosols, sprays, solutions, emulsions and suspensions. In the cosmetic field, preparations of the invention are preferred which are for an application on any topical route.

In accordance with the present invention and in preferred embodiments thereof, the amounts of at least one of the compounds of the general formula (I) in general, or of at least one the afore-mentioned compounds in accordance with the Table 1, in the cosmetic preparations of the invention may be widely selected, without imposing any restriction to the skilled practitioner. I will be particularly be possible that, in accordance with usual parameters as, for example parameters depending on the person to be treated, the skin status of said person, and other usual parameters, the amounts to be applied (or even administered) may easily be determined by a skilled person in the cosmetic field by conducting only a few orienting experiments. Specifically, the amounts may be (without restricting the invention to those amounts) in the range of 0.01 to 1000 mg with regard to at least one of the compounds of the general formula (I) in

general, or to at least one of the afore-mentioned compounds in accordance with the Table 1, per application unit, preferably in the range of 0.1 to 100 mg per application unit.

Below, the invention is further explained by means of examples. These examples refer to preferred embodiments of the invention, which are given mainly to exemplify and explain the invention for a better understanding. The Examples, however, should not be construed to restrict the invention.

## Examples

### Example 1

### Preparation of compounds of the general formula (I)

When preparing the compounds of the general formula (I), the synthesis routes of the following Schemes 1 to 17 were selected; the reaction conditions for selected steps are indicated below the schemes:

Scheme 1:

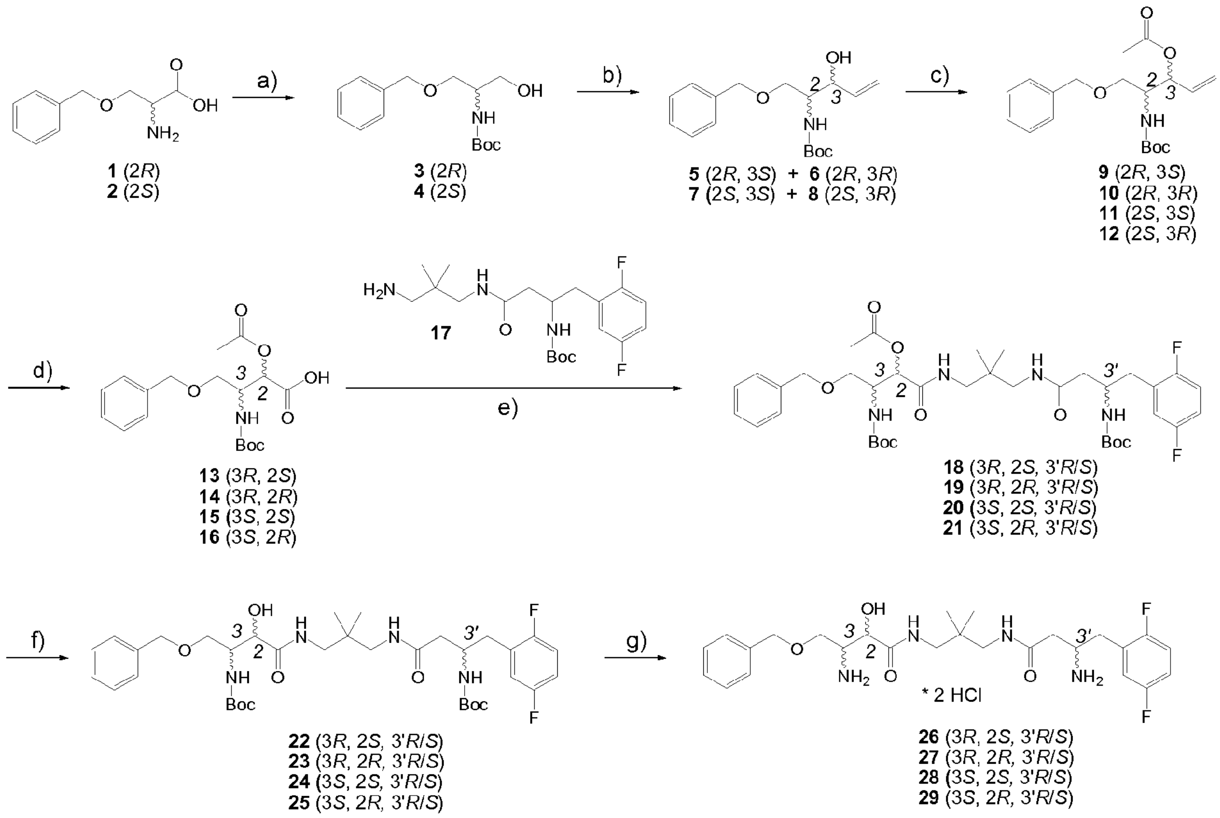


**1** (2*R*)
**2** (2*S*)

**3** (2*R*)
**4** (2*S*)

**5** (2*R*, 3*S*) + **6** (2*R*, 3*R*)
**7** (2*S*, 3*S*) + **8** (2*S*, 3*R*)

**9** (2*R*, 3*S*)
**10** (2*R*, 3*R*)
**11** (2*S*, 3*S*)
**12** (2*S*, 3*R*)

**17**

**13** (3*R*, 2*S*)
**14** (3*R*, 2*R*)
**15** (3*S*, 2*S*)
**16** (3*S*, 2*R*)

**18** (3*R*, 2*S*, 3'*R*/*S*)
**19** (3*R*, 2*R*, 3'*R*/*S*)
**20** (3*S*, 2*S*, 3'*R*/*S*)
**21** (3*S*, 2*R*, 3'*R*/*S*)

**22** (3*R*, 2*S*, 3'*R*/*S*)
**23** (3*R*, 2*R*, 3'*R*/*S*)
**24** (3*S*, 2*S*, 3'*R*/*S*)
**25** (3*S*, 2*R*, 3'*R*/*S*)

**26** (3*R*, 2*S*, 3'*R*/*S*)
**27** (3*R*, 2*R*, 3'*R*/*S*)
**28** (3*S*, 2*S*, 3'*R*/*S*)
**29** (3*S*, 2*R*, 3'*R*/*S*)

Reagents and conditions: a) 1. $LiAlH_4$, THF, rfl.; 2. NaOH/$H_2O$, $Boc_2O$, DCM, RT. b) 1. $(COCl)_2$, DMSO, DCM, -78˚C -> -10˚C; 2. Vinyl-magnesium bromide, DCM, THF, RT. c) $Ac_2O$, pyridine, DCM, 0˚C -> RT. d) $NaIO_4$ cat. $RuCl_3$, CH3CN, EtOAc, $H_2O$. e) DCC, HOBt, DCM, 17,0˚C -> RT. f) LiOH, MeOH, $H_2O$. g) aq. HCl (37 %), EtOH.

Scheme 2:

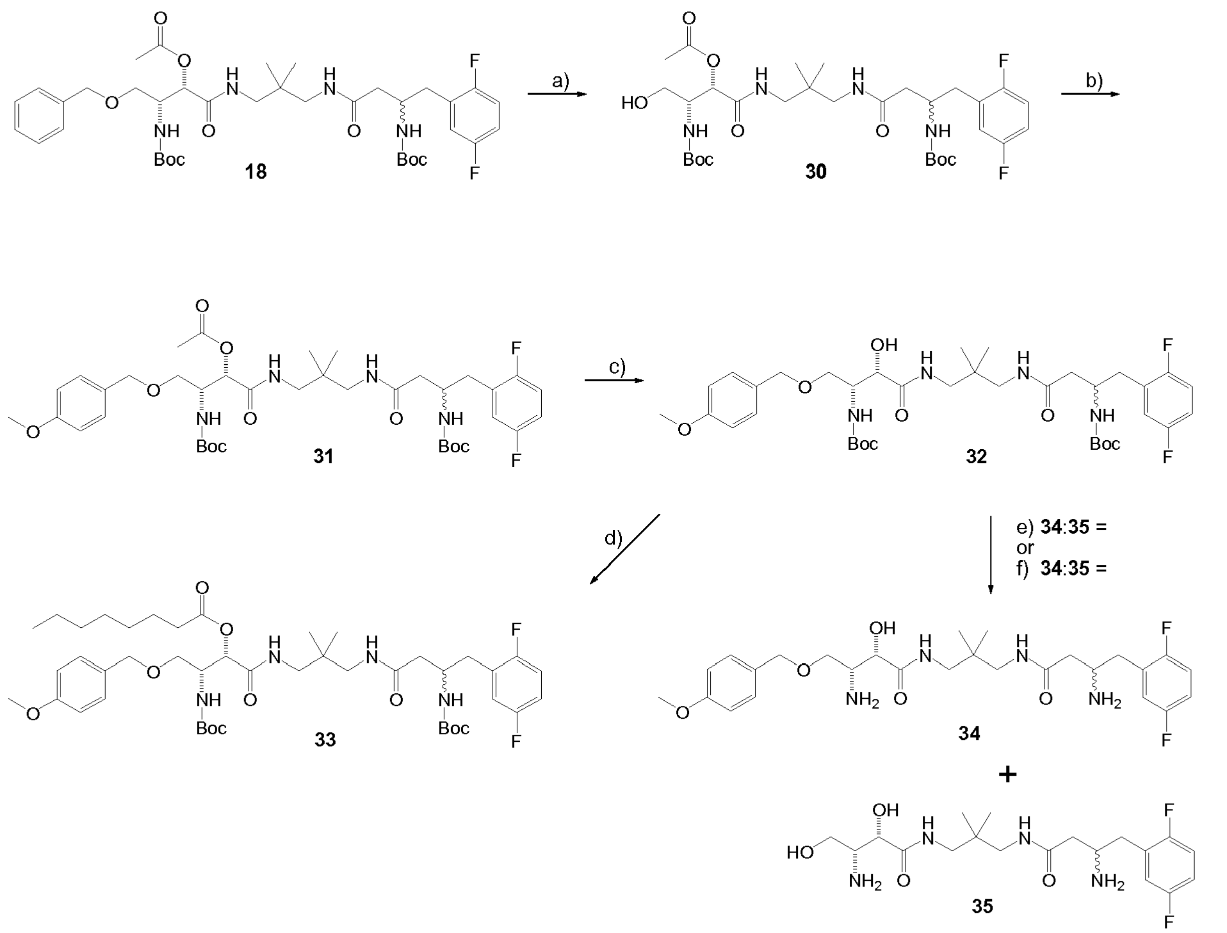


Reagents and conditions: a) $H_2$, Pd/C, MeOH. b) NaH, NaI, PMB-Cl, THF. c) LiOH, MeOH, $H_2O$. d) n-$C_7H_{13}COCl$, TEA, DMAP, DCM, RT. e) f)

Scheme 3:

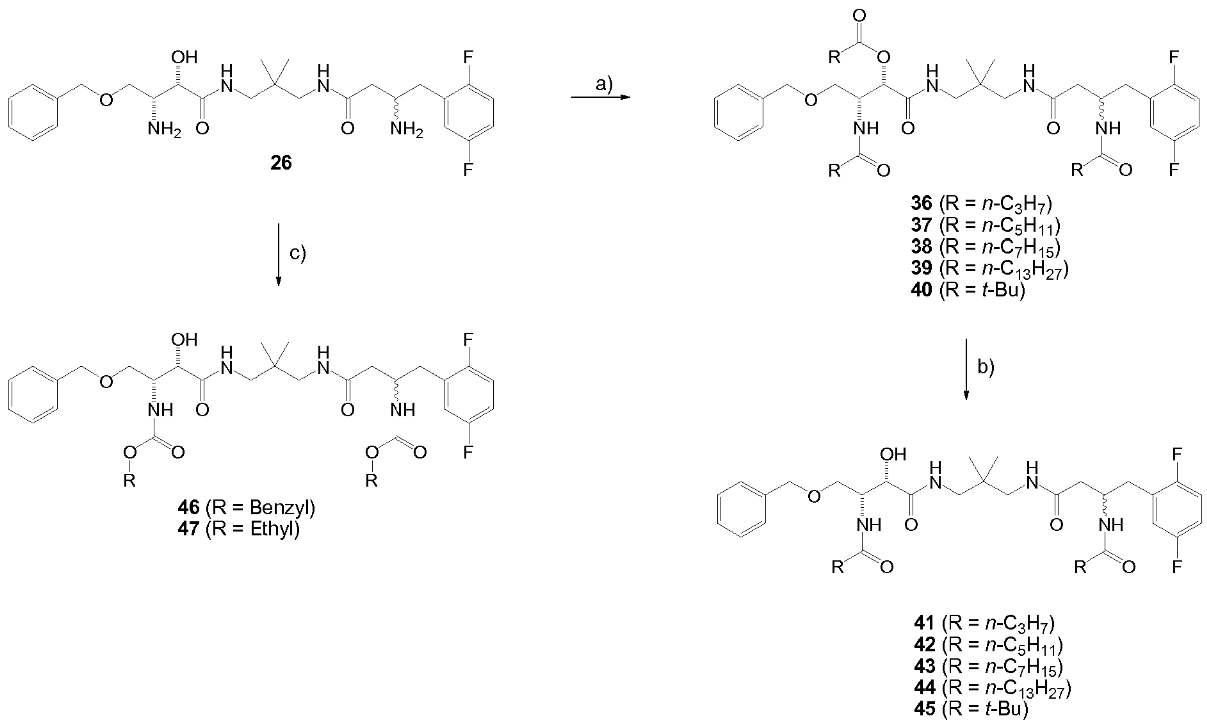


26

**36** (R = $n$-$C_3H_7$)
**37** (R = $n$-$C_5H_{11}$)
**38** (R = $n$-$C_7H_{15}$)
**39** (R = $n$-$C_{13}H_{27}$)
**40** (R = $t$-Bu)

**46** (R = Benzyl)
**47** (R = Ethyl)

**41** (R = $n$-$C_3H_7$)
**42** (R = $n$-$C_5H_{11}$)
**43** (R = $n$-$C_7H_{15}$)
**44** (R = $n$-$C_{13}H_{27}$)
**45** (R = $t$-Bu)

Reagents and conditions: a) RCOCl, TEA, DMAP, DCM. b) LiOH, MeOH, $H_2O$. c) ROCOCl, $NaHCO_3$ $H_2O$, 1,4-dioxane.

Scheme 4:

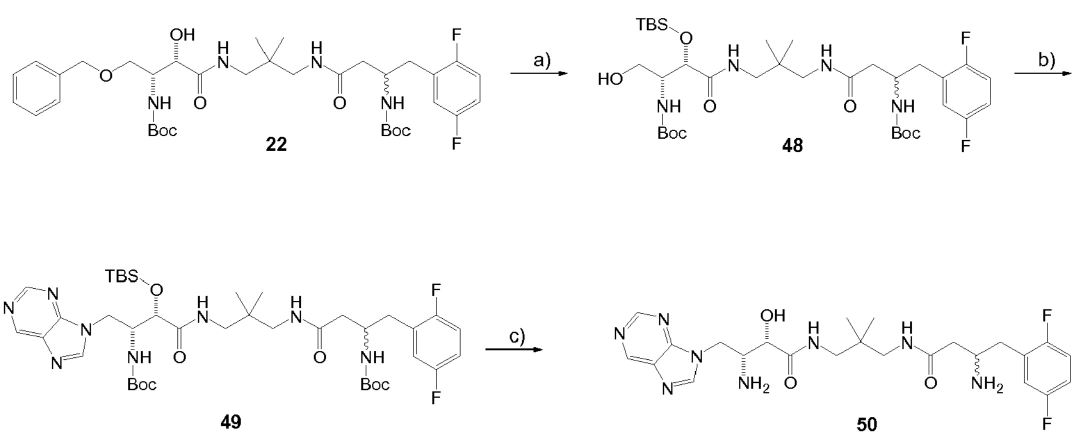


**22** **48** **49** **50**

Reagents and conditions:a) 1. TBS-Cl, imidazole, DMF. 2. $H_2$, Pd/C, methanol b) Purine, $PPh_3$, DIAD, THF. c) aq. HCl (37 %), EtOH.

Scheme 5:

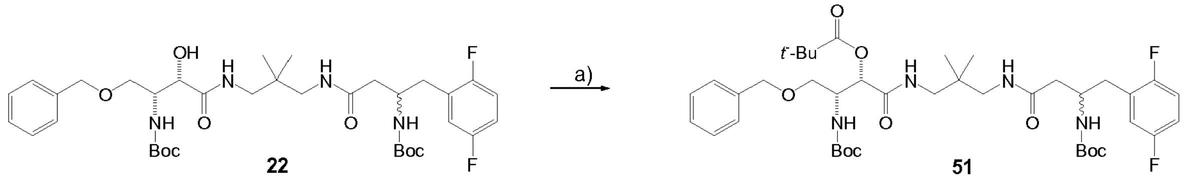


22 51

Reagents and conditions: a) t-Bu-COCl, TEA, DMAP, DCM.

Scheme 6:

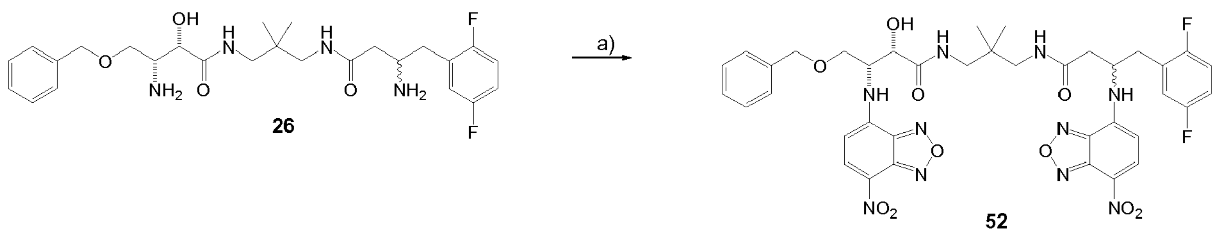


26 52

Reagents and conditions: a) NBD-F, EtOH, rfl.

Scheme 7:

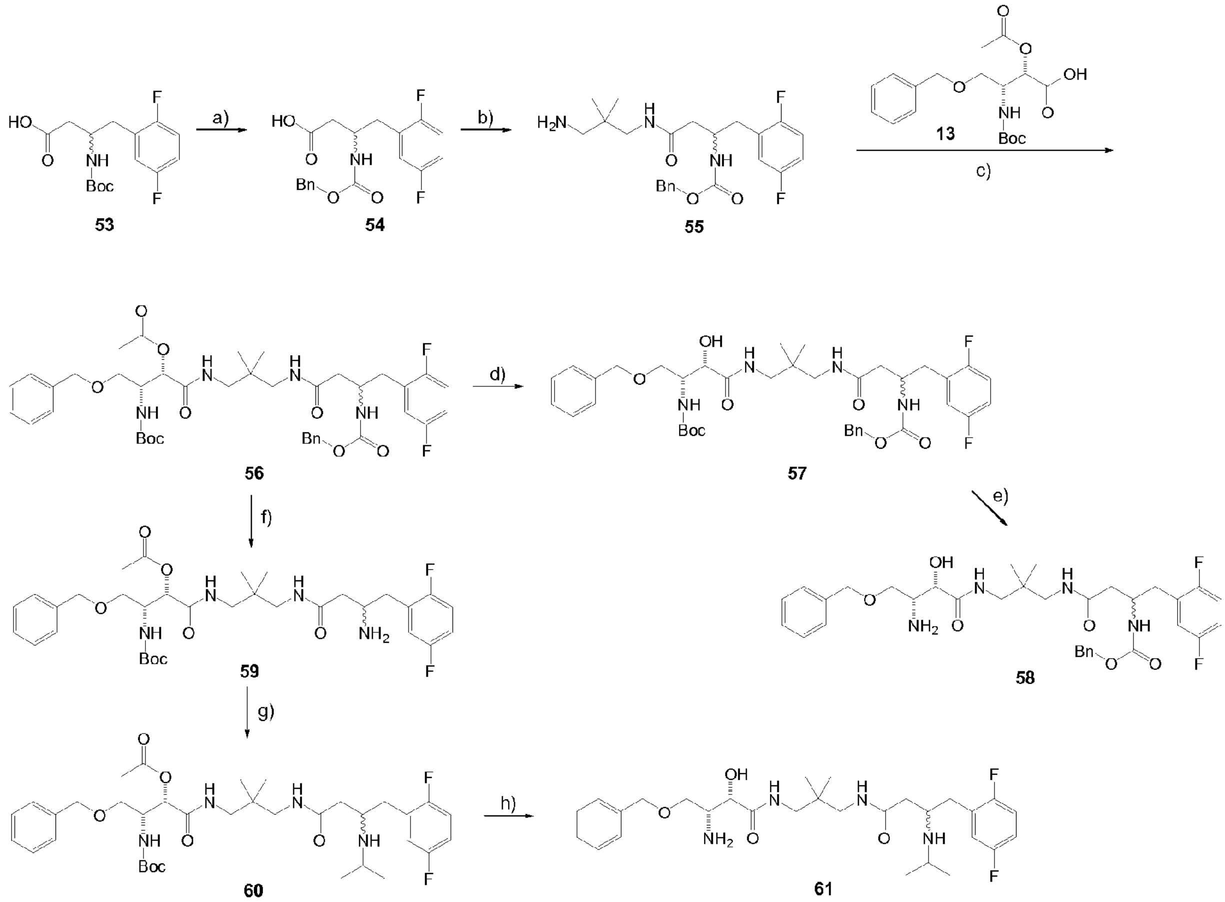


53 54 55 13 56 57 58 59 60 61

Reagents and conditions: a) 1. TFA, DCM. 2. BnOCOCl, $NaHCO_3$ $H_2O$, 1,4-dioxane. b) DCC, HOBt, 1,3-diamino-2,2-dimethylpropane, DCM, 0˚C -> RT. c) DCC, HOBt, **13**, DCM, 0˚C -> RT. d) LiOH, MeOH, $H_2O$. e) aq. HCl (37 %), EtOH. f) $H_2$, Pd/C, MeOH. g) acetone, MS 3 A, $NaCNBH_3$, THF. h) 1. LiOH, MeOH, $H_2O$. 2. aq. HCl (37 %), EtOH.

Scheme 8:

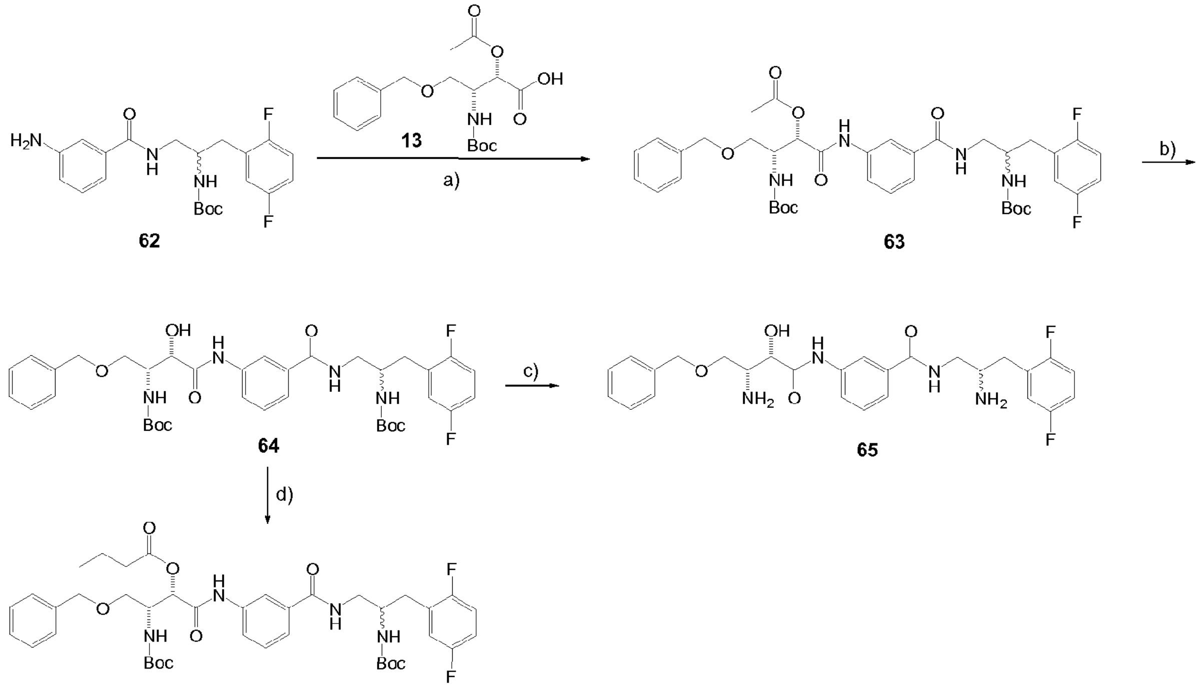


62 63 64 65 66

Reagents and conditions: a) DCC, HOBt, **13**, DCM, 0°C -> RT. b) LiOH, MeOH, $H_2O$. c)) aq. HCl (37 %), EtOH. d) *n*-$C_3H_7$-COCl, TEA, DMAP, DCM.

Scheme 9:

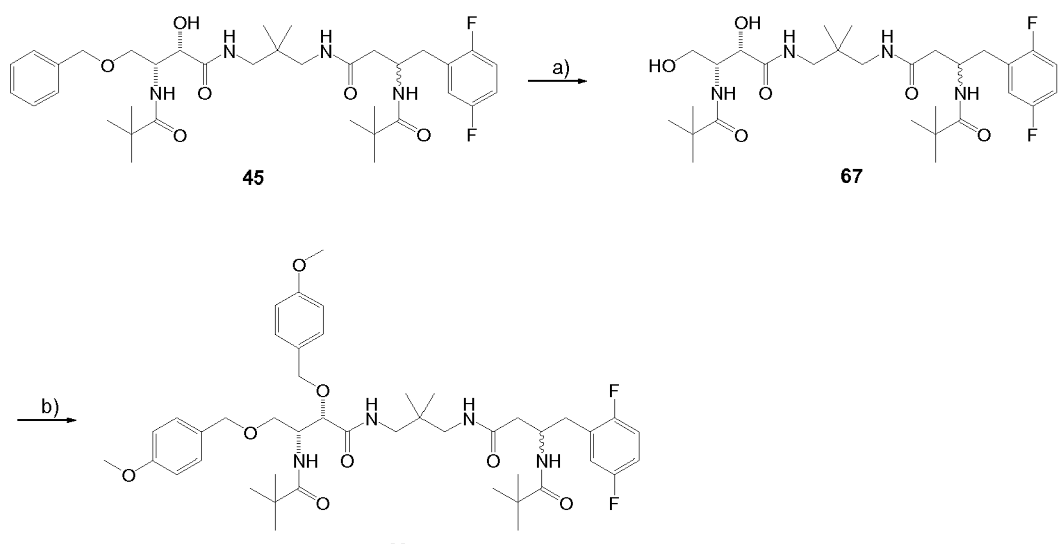


45 67 68

Reagents and conditions: a) $H_2$, Pd/C, MeOH. b) NaH, NaI, PMB-Cl, THF.

Scheme 10:

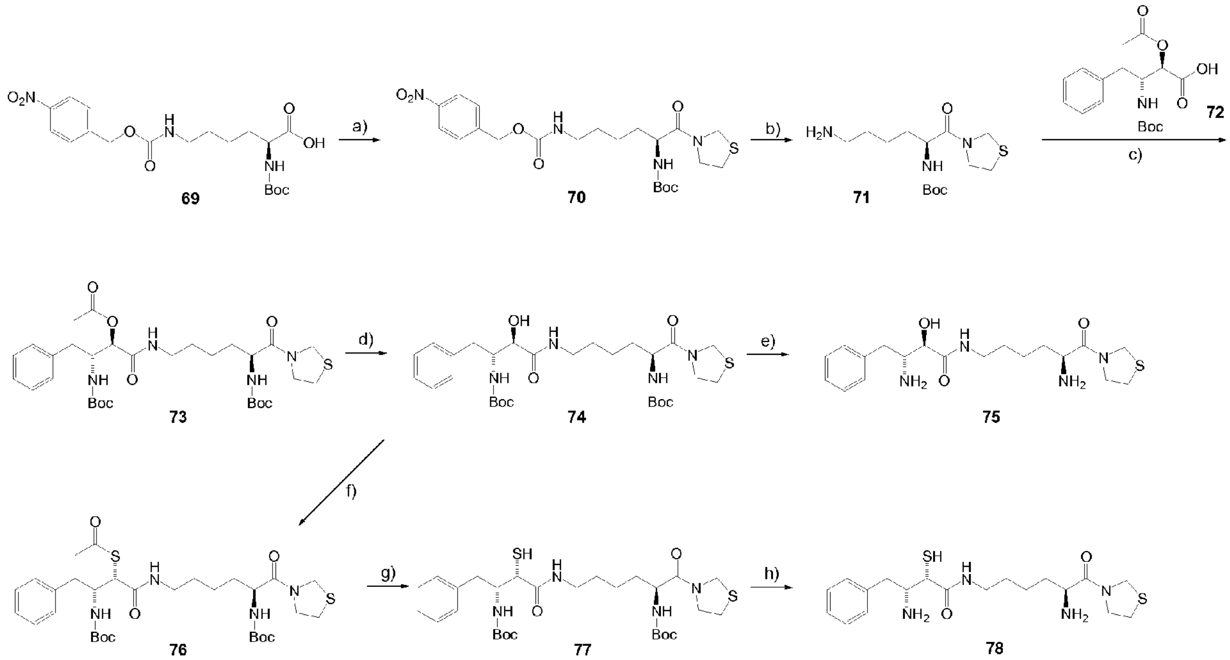


Reagents and conditions: a) EDC, DIPEA, thiazolidine, DCM, 0˚C -> RT. b) $H_2$, Pd/C, MeOH. c) DCC, HOBt, **72**, DCM, 0˚C -> RT. d) LiOH, MeOH, $H_2O$. e) aq. HCl (37 %), EtOH. f) 1. MsCl, TEA, DCM. 2. KSAc, DMF, 60˚C. g) LiOH, MeOH, $H_2O$. h) aq. HCl (37 %), EtOH.

Scheme 11:

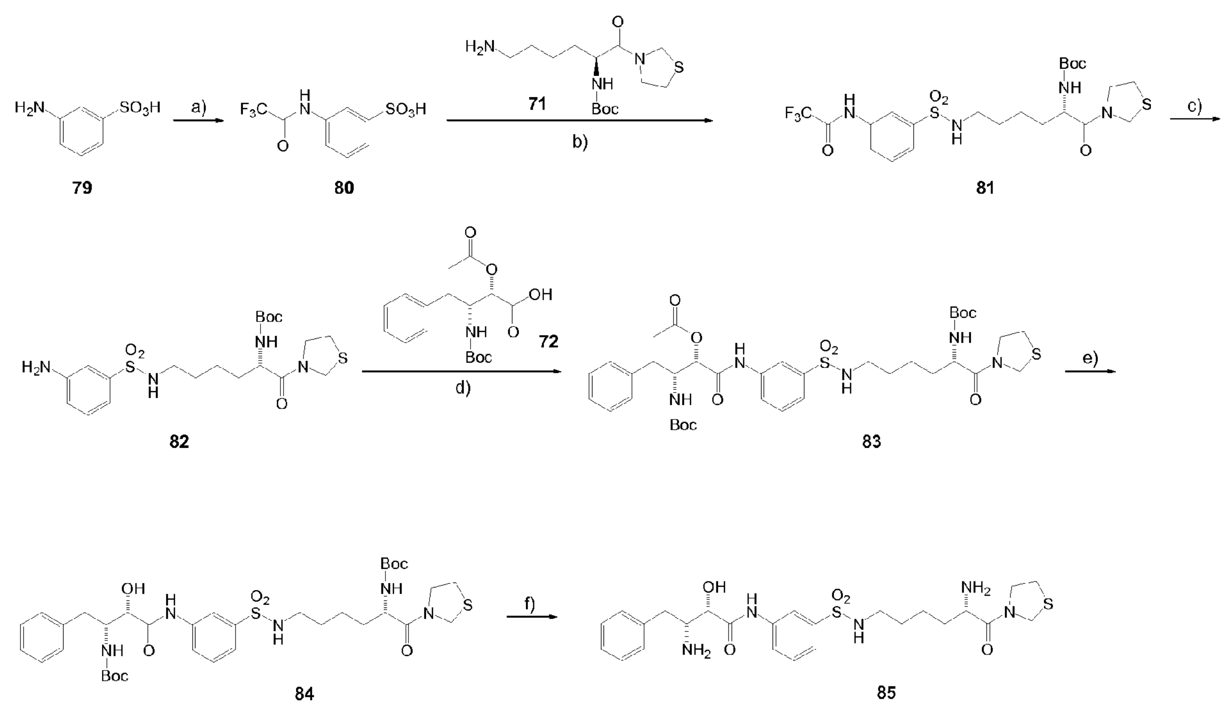

Reagents and conditions: a) TFAA, pyridine, DCM. b) 1. $SOCl_2$, DMF. 2. TEA, **71**, DCM. c) LiOH, MeOH, $H_2O$. d) DCC, HOBt, **72**, DCM, 0˚C -> RT. e) LiOH, MeOH, $H_2O$. f) aq. HCl (37 %), EtOH.

Scheme 12:

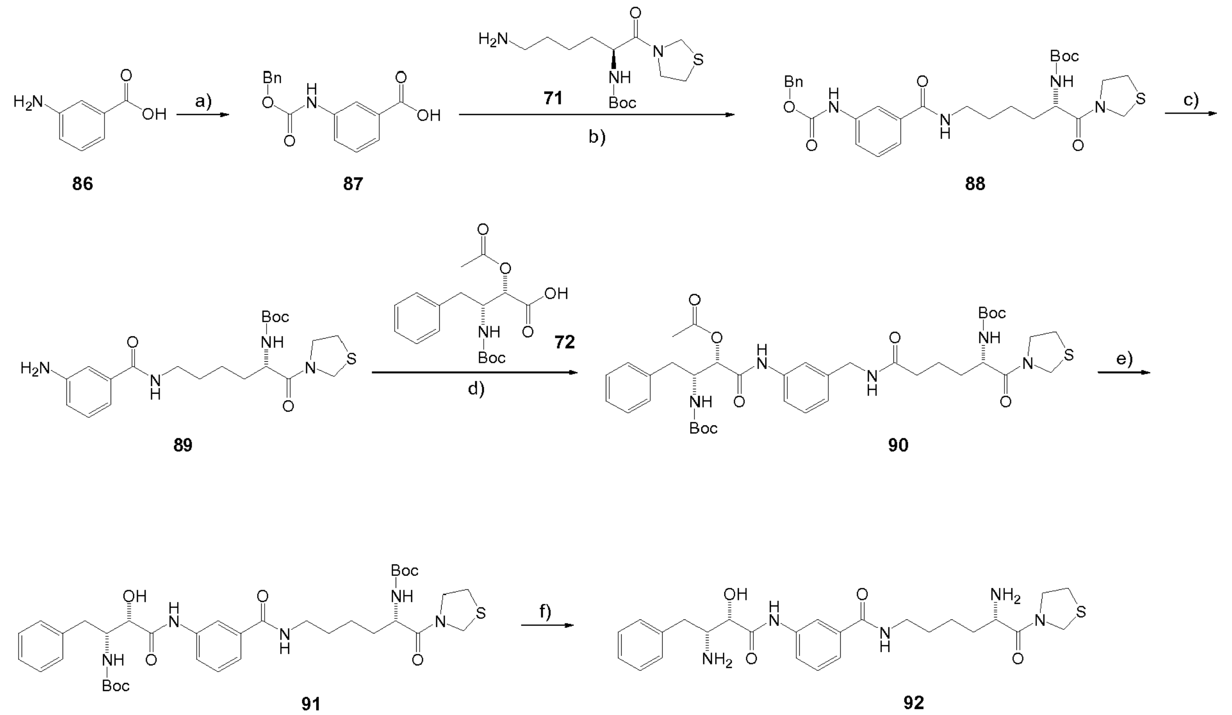


Reagents and conditions: a) BnOCOCl, $NaHCO_3$, $H_2O$, 1,4-dioxane. b) DCC, HOBt, **71,** DCM, 0˚C -> RT c) $H_2$, Pd/C, MeOH. d) DCC, HOBt, **72,** DCM, 0˚C - > RT e) LiOH, MeOH, $H_2O$. f) aq. HCl (37 %), EtOH.

Scheme 13:

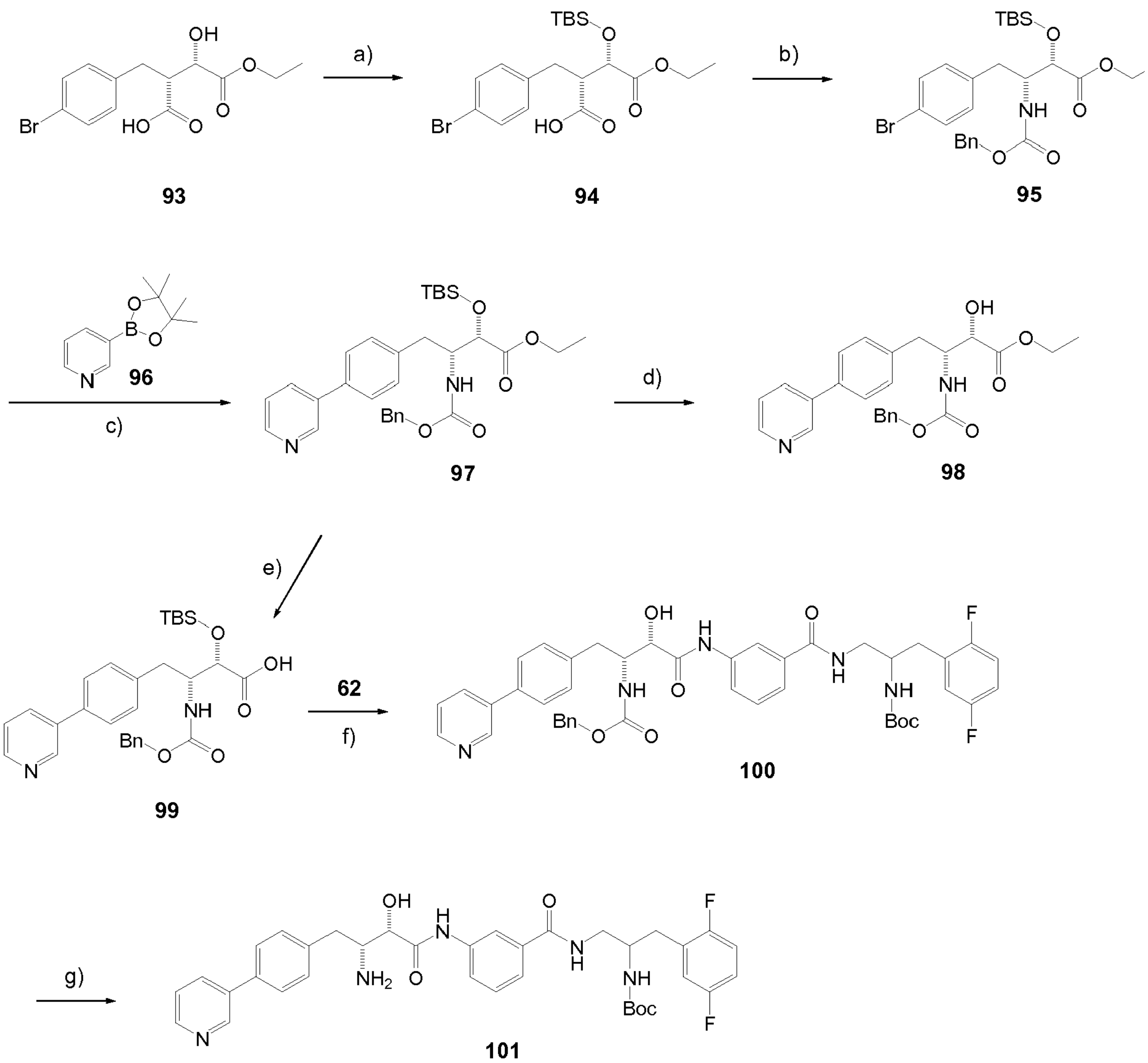


93 94 95 96 97 98 99 100 101

Reagents and conditions: a) (1) TBS-Cl, imidazole, DMF. (2) $Na_2CO_3$, $H_2O$, methanol. b) DPPA, TEA, toluene, benzyl alcohol c) **96,** $Pd(PPh_3)_4$, $CsCO_3$, DME. d) aq. HCl (37 %), EtOH.e) LiOH, MeOH, $H_2O$. f) **62,** DCC, HOBt, DCM. g) $H_2$, Pd/C, MeOH.

Scheme 14:

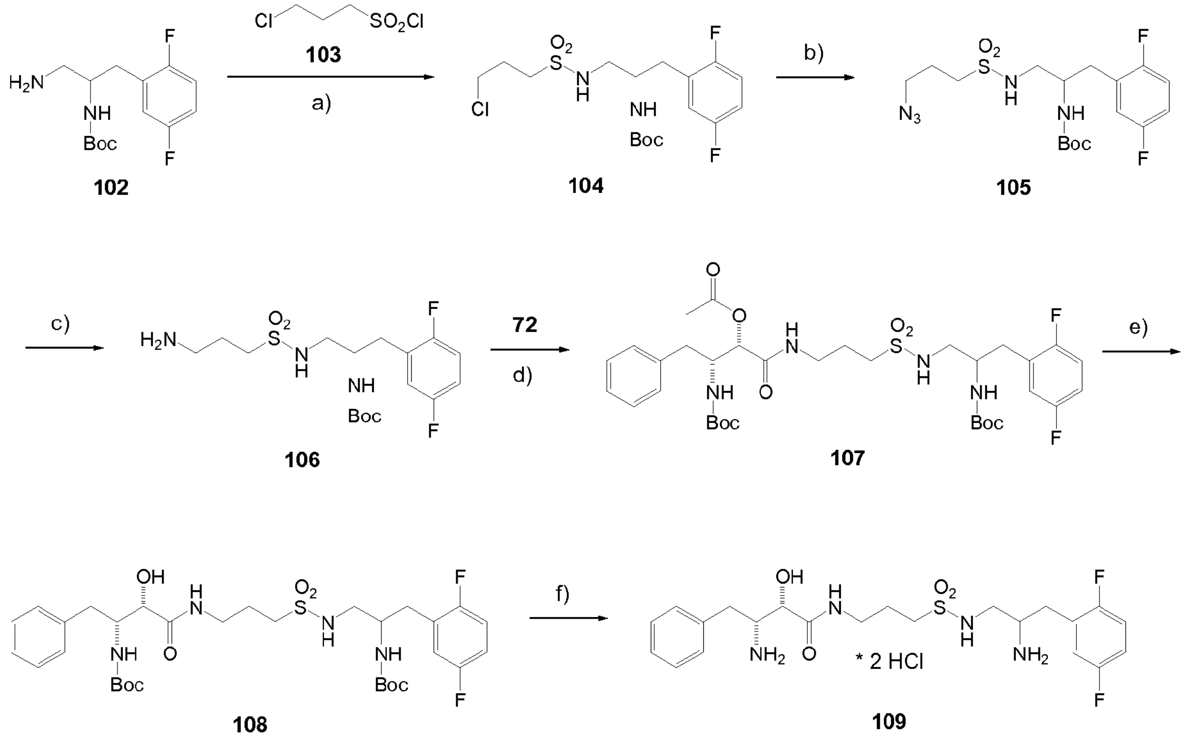


102 104 105 106 107 108 109

Reagents and conditions: a) TEA, DCM. b) $NaN_3$, DMF, 60˚C c) $H_2$, Pd/C, MeOH. d) DCC, HOBt, **72,** DCM, 0˚C -> RT e) LiOH, MeOH, $H_2O$. f) aq. HCl (37 %), EtOH.

Scheme 15:

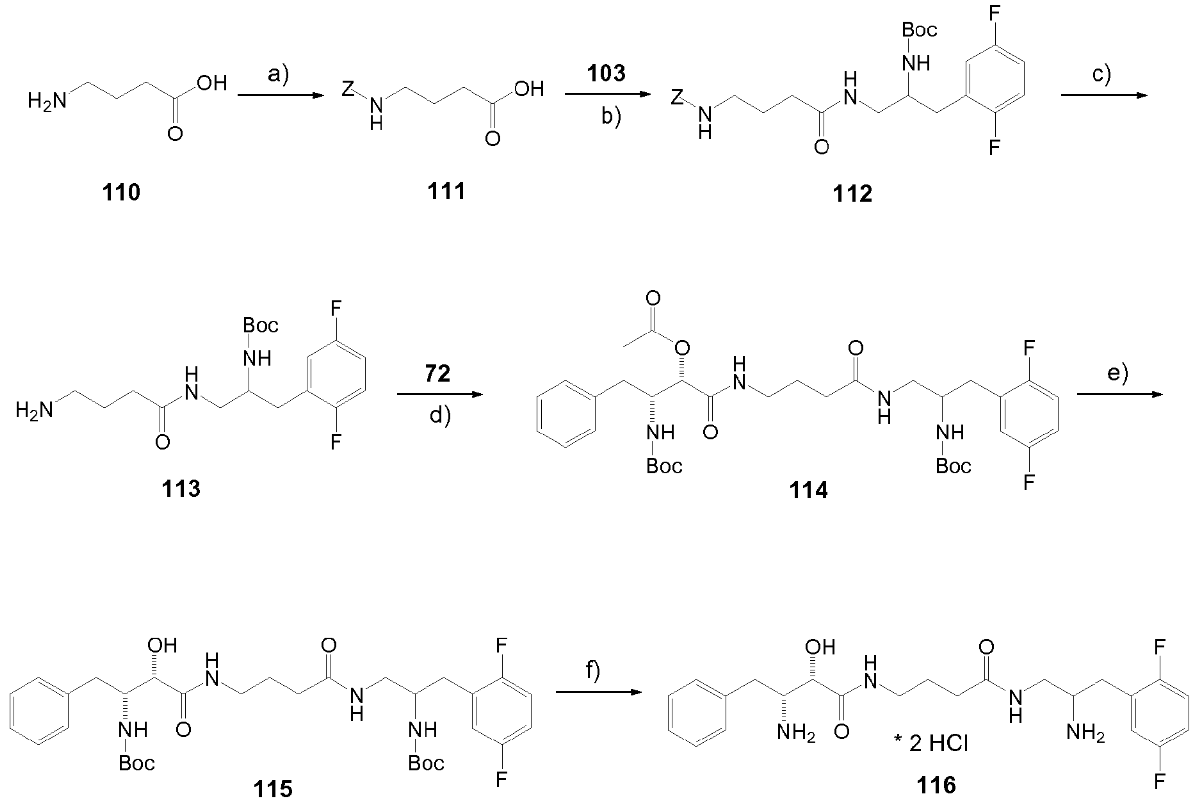


110 111 112 113 114 115 116

Reagents and conditions: a) Z-Cl, $NaHCO_3$, $H_2O$, 1,4-dioxane. b) DCC, HOBt, **103,** DCM, 0˚C -> RT c) $H_2$, Pd/C, MeOH. d) DCC, HOBt, **72,** DCM, 0˚C -> RT e) LiOH, MeOH, $H_2O$. f) aq. HCl (37 %), EtOH.

Scheme 16:

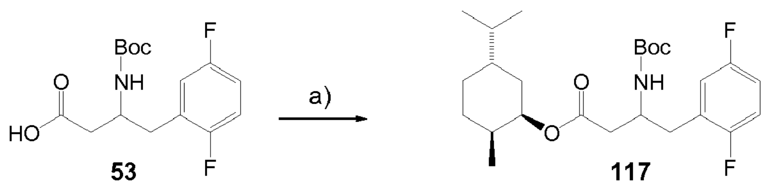


53 117

Reagents and conditions: a) DCC, DMAP, L-menthol, DCM, 0˚C -> RT.

Scheme 17:

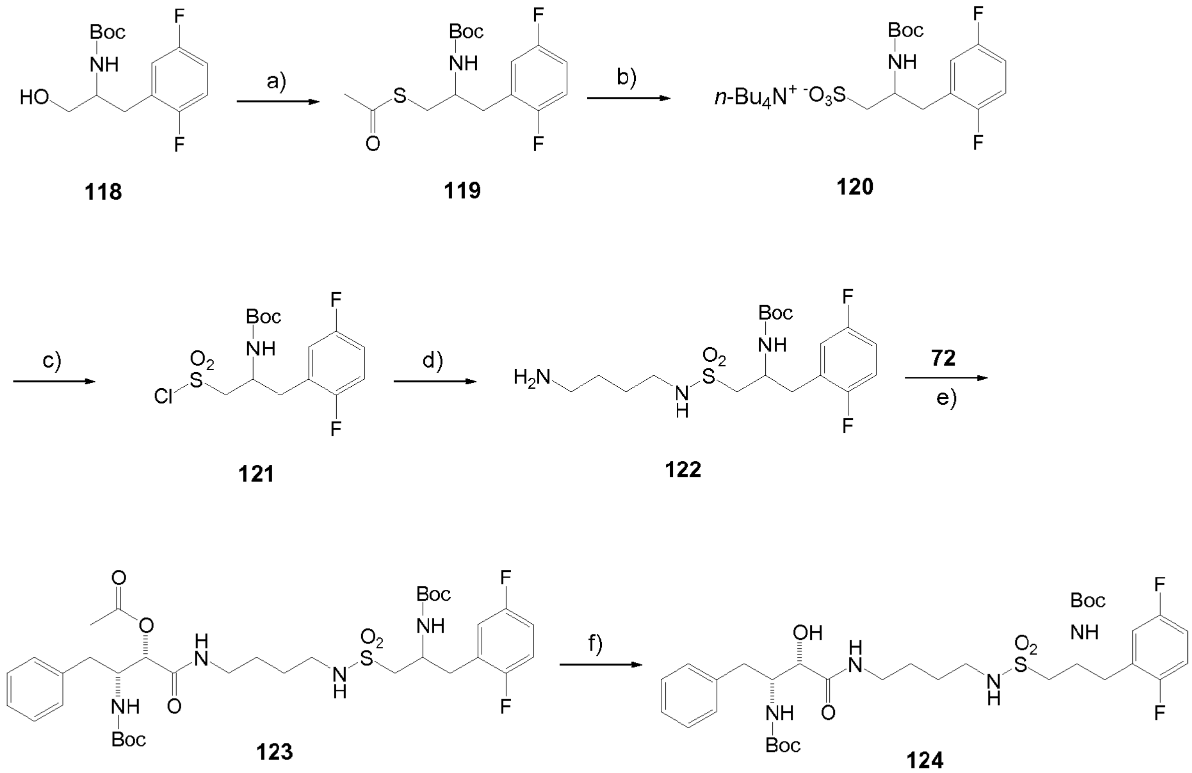


118 119 120 121 122 123 124

Reagents and conditions: a) 1. $CH_3SO_2Cl$, TEA, DCM. 2. KSAc, DMF b) Oxone, *n*-$Bu_4NOH$, $H_2O$, methanol. c) $SOCl_2$, DMF, DCM. d) 1,4-diaminobutane, TEA, DCM. e) DCC, HOBt, **72,** DCM, 0˚C -> RT. f) LiOH, MeOH, $H_2O$.

**General procedure A: Peptide coupling using DCC and HOBt**

A solution of the carboxylic acid (1.0 eq.) in dichloromethane (10 ml per mmol) was cooled to 0˚C, and 1-hydroxybenzotriazole (1.3 eq.) and N,N'-dicyclohexyl-carbodiimid (1.5 eq.) were added. After 1 hour at this temperature, the amine (1.0 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases were dried ($MgSO_4$) and evaporated. The crude product was purified by flash chromatography on silica (eluent: dichloromethane/diethylether or dichloromethane/ethyl acetate).

**General procedure B: Ester cleavage using LiOH**

A solution of the ester (1.0 eq.) in methanol/water (9 : 1, 10 ml per mmol) was treated with LiOH*$H_2O$ (5.0 eq.) and stirred at ambient temperature until TLC control showed the absence of the starting material. Methanol was evaporated and the residue was distributed between ethyl acetate and brine. The layers were separated and the organic phase was dried ($MgSO_4$). If necessary the crude product was purified by flash chromatography on silica with an appropriate eluent.

**General procedure C: Boc deprotection using aq.HCl in EtOH**

The Boc-protected compound (1.0 eq.) was treated with an 8 : 1 mixture of ethanol and hydrochloric acid (37 %) (1.5 ml per mmol). The resulting solution was stirred at ambient temperature until TLC control showed the absence of the starting material. The solvents were evaporated and the residue was dried in high vacuum. The resulting amine was obtained as its hydrochlorid.

**General procedure D: N- and O-Acylation with acid chlorides**

The amino and/or hydroxyl compound (1.0 eq.) was dissolved in dichloromethane (2,5 ml per mmol) and triethylamine (2.5 eq. per acylatable functionality), 4-N,N-dimethylaminopyridine (0.2 eq.) and acid chloride (2.0 eq. per acylatable functionality) were added. The mixture was stirred overnight and then quenched with hydrochloric acid (1 M, 2 ml per mmol triethylamine). The phases were separated and the the aqueous phase was extracted three times with dichloromethane. The combined organic phases were dried ($MgSO_4$) and evaporated. The crude product was purified by flash chromatography on silica (eluent: dichloromethane/diethylether).

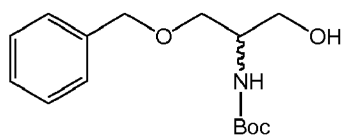

**3** and **4:** (Scheme 1): The corresponding O-benzyl protected D- or L-serine **1** or **2** (5.00 g, 25.6 mmol) was added slowly to a suspension of $LiAlH_4$ (1.46 g, 38.5 mmol) in THF (100 ml). The resulting mixture was heated under reflux for 5 hours and then cooled to 0 ˚C. Excess $LiAlH_4$ was subsequently quenched with 10% NaOH aq. (6 ml) and water (6 ml). The slurry was stirred at ambient temperature for 30 minutes and $Boc_2O$ (6.15 g, 28.2 mmol) in dichloromethane (20 ml) was added. The reaction mixture was stirred over night and filtered over a short path of silica. Evaporation of the solvents and recrystallization from MTBE and pentane provided the products **3** or **4** (8.18 g).

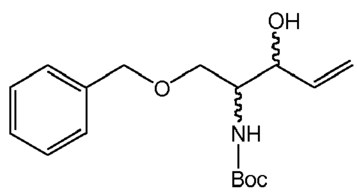

**5, 6, 7, and 8** (Scheme 1): A solution of oxalylchloride (1.1 eq.) in dichloromethane (3 ml per mmol) was cooled with dry ice/acetone to -78 ˚C, and a solution of dimethylsulfoxide (2.4 eq.) in dichloromethane (3 ml per mmol) was added over a period of 10 minutes. The mixture was stirred for 15 minutes before a solution of **3** or **4** (1.0 eq.) in dichloromethane (3 ml per mmol) was added over a period of 20 minutes. The mixture was stirred for 30 minutes before ethyl-diisopropylamine (4.0 eq.) was added. The temperature was slowly raised to -10 ˚C before the reaction mixture was cooled again to -78 ˚C. The cold mixture was transferred by the use of a double-ended needle to a mixture of vinylmagnesiumbromide (5.0 eq., 0.5 M solution in tetrahydrofurane/dichloromethane 1:1). The resulting mixture was stirred for one hour at ambient temperature and then quenched with $KHSO_4$ (1 M solution in water). The phases were separated and the the aquaous phase was extracted three times with dichloromethane. The combined organic phases were dried ($MgSO_4$) and evaporated. The epimeric products **5** and **6** from starting material **3** (or **7** and **8** from **4)** were isolated in a total yield of 57 % (ratio **5 : 6** respectively **7 : 8** = 3 : 1, by flash-chromatography on silica (eluent: pentane/diethylether). The epimers **5** and **6,** respectively **7** and **8,** were partially separated by flash-chromatography on silica (eluent: pentane/ diethylether).

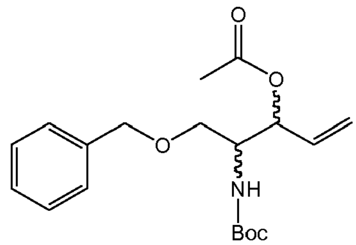

**9, 10, 11,** and **12** (Scheme 1): A solution of **5, 6, 7,** or **8** (1.0 eq.) in tetrahydrofurane (2.5 ml per mmol) was cooled to 0 ˚C and pyridine (4.0 eq.), acetic acid anhydride (2.0 eq.) and N,N-dimethylaminopyridine (0.2 eq.) were added slowly. The reaction mixture was stirred over night at ambient temperature. The solution was poured in aq. hydrochloric acid (10 ml per mmol) and the phases were separated. The aqueous phase was extracted twice with ethyl acetate. The combined organic layers were washed with saturated aq. $NaHCO_3$ and dried ($MgSO_4$). Evaporation of the solvents and flash-chromatography on silica (eluent: pentane/diethylether) provided the product.

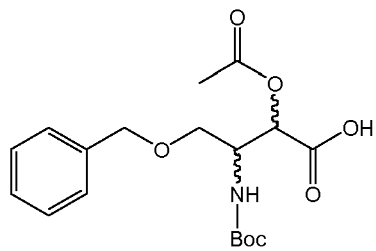

**13, 14, 15,** and **16** (Scheme 1): A solution of **9, 10, 11,** or **12** (1.0 eq.) in acetonitril/ethyl acetate/water (2 : 2 : 3, 10 ml per mmol) was treated with $NaIO_4$ (4.0 eq.) and $RuCl_3$ hydrate (0.02 eq.). The resulting slurry was stirred for 18 hours at ambient temperature. Saturated aq. NaCl (10 ml per mmol) was added and the mixture was extracted thrice with ethyl acetate. The combined organic layers were dried ($MgSO_4$) and the solvents were evaporated. The obtained products were used without further purification.

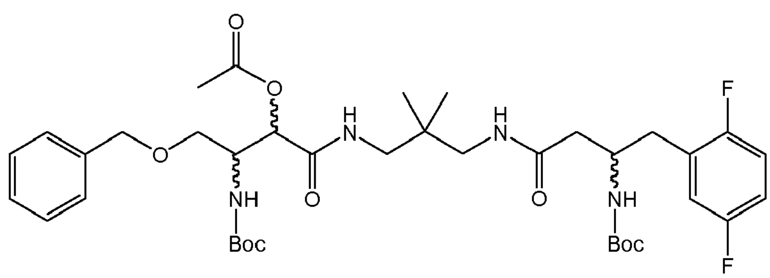

**18, 19, 20,** and **21** (Scheme 1) were obtained from **13, 14, 15,** or **16** using general procedure A.

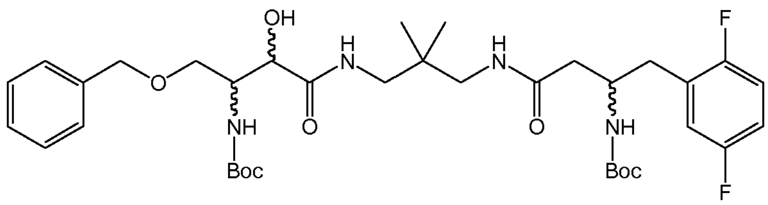

**22, 23, 24,** and **25** (Scheme 1) were obtained from **18, 19, 20** or **21** using general procedure B.

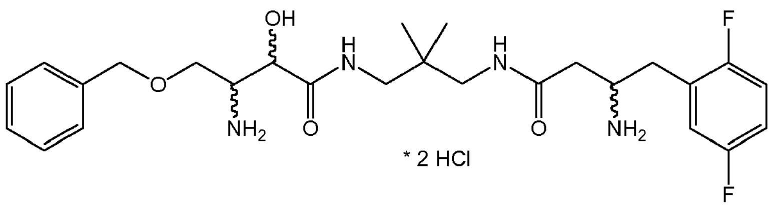

**26, 27, 28,** and **29** (Scheme 1) were obtained from **22, 23, 24** or **25** using general procedure C.

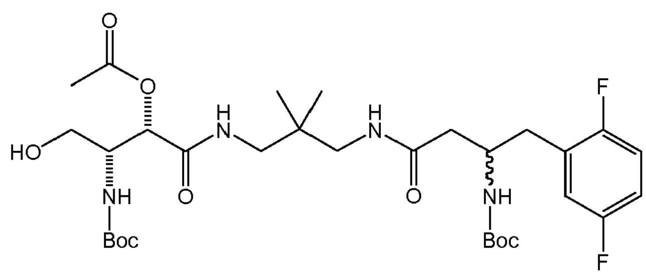

**30** (Scheme 2): Palladium on charcoal (10%, 0.2 eq.) was added to a solution of **18** (1.0 eq.) in methanol (10 ml per mmol). The resulting suspension was stirred for 18 h under a hydrogen atmosphere. The solids were filtered of through a short path of celite and the filtrate was evaporated to obtain **30.**

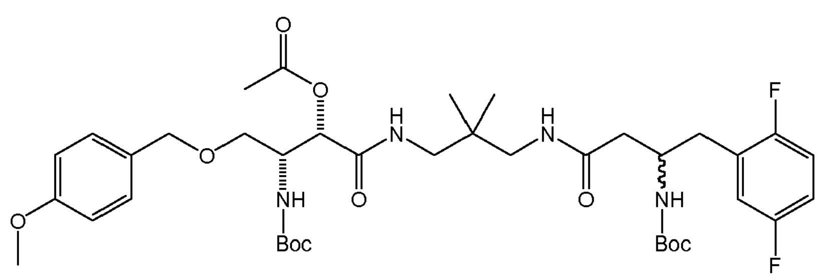

**31** (Scheme 2): To a cooled (0˚C) solution of **30** (1.0 eq.) in THF (3 ml per mmol) were added subsequently sodium hydride (60 %, 1.5 eq.), sodium iodide (2.0 eq.) and p-methoxybenzyl chloride (4.0 eq.). The reaction mixture was stirred at ambient temperature for 19 h. The reaction was quenched with saturated aq. $NH_4Cl$ (5 ml per ml THF) and the layers were separated. The aqueous phase was extracted twice with ethylacetate, and the combined organic phases were washed with brine. After drying ($MgSO_4$) and evaporation of the solvents, **31** was purified by flash-chromatography on

silica (eluent: dichloromethan/diethylether).

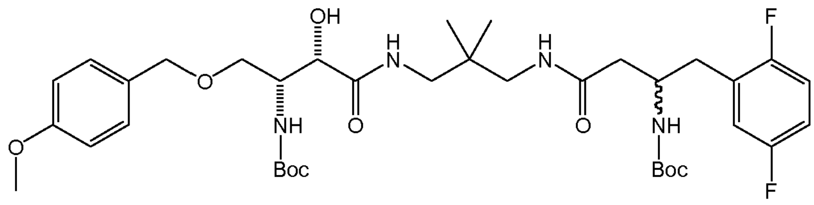

**32** (Scheme 2) was obtained from **31** using general procedure B.

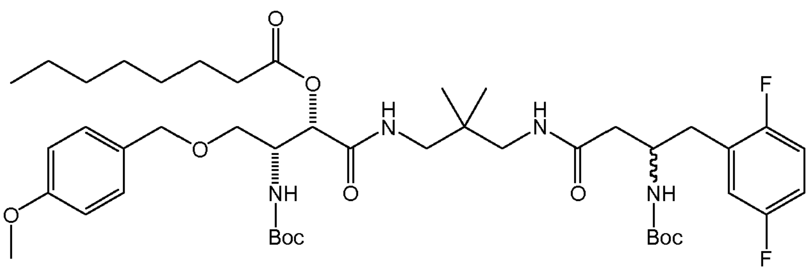

**33** (Scheme 2) was obtained from **32** and octanoylchloride using general procedure D.

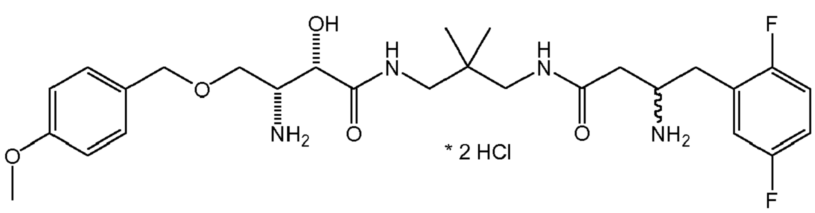

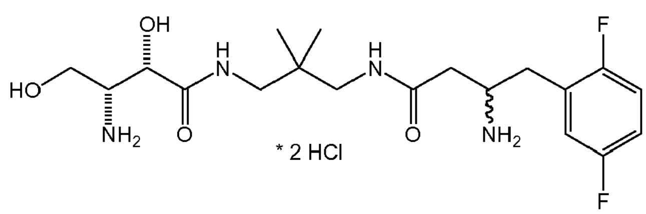

A mixture of **34** and **35** (1 : 1) was obtained from **32** using general procedure C.

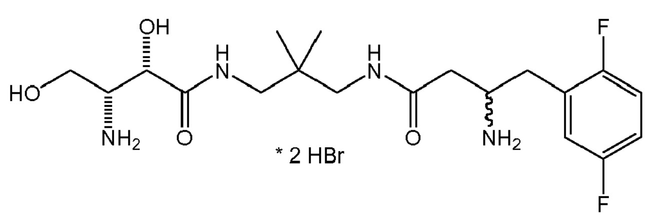

**35** (Scheme 2): A solution of **32** (26.7 mg) in dichloromethane (1.5 ml) was treated with $BBr_3$ (1 M solution in dichloromethane, 0.5 ml). After three hours at ambient temperature, methanol (10 ml) was added and volatiles were distilled off. This procedure was repeated three times to yield **35** (21.9 mg).

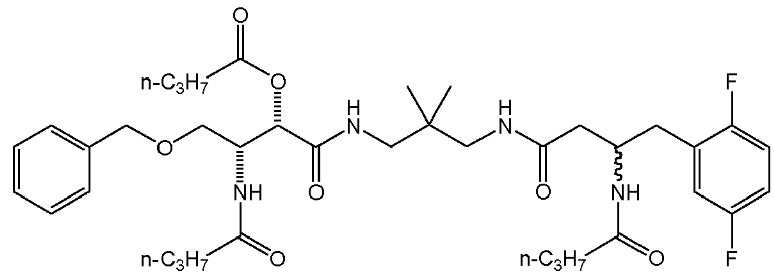

**36** (Scheme 3) was obtained from **26** and butyryl chloride using general procedure D.

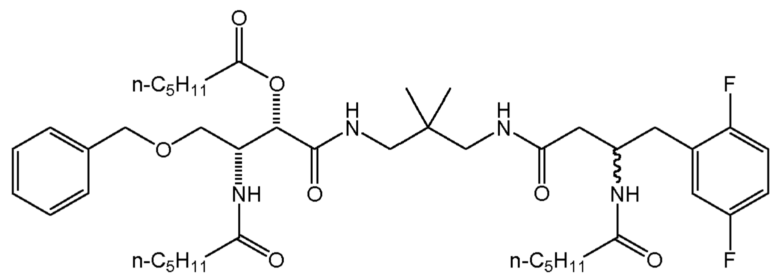

**37** (Scheme 3) was obtained from **26** and hexanoyl chloride using general procedure D.

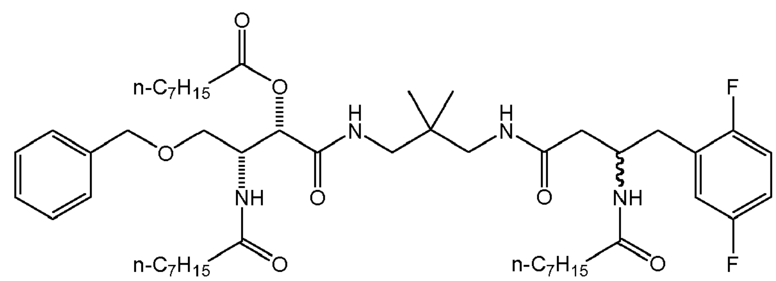

**38** (Scheme 3) was obtained from **26** and octanoyl chloride using general procedure D.

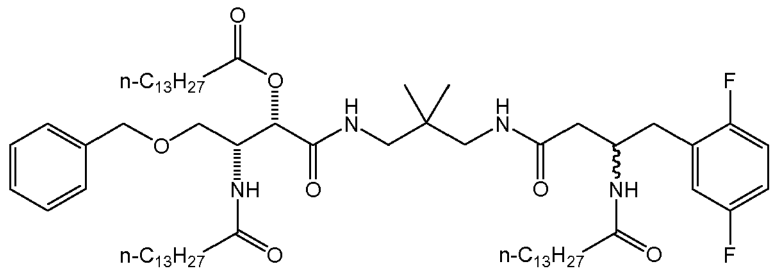

**39** (Scheme 3) was obtained from **26** and tetradecanoyl chloride using general procedure D.

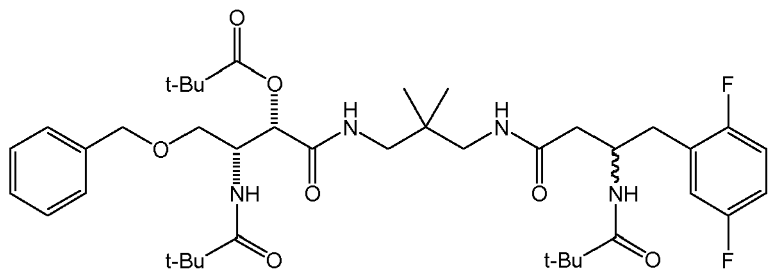

**40** (Scheme 3) was obtained from **26** and pivaloyl chloride using general procedure D.

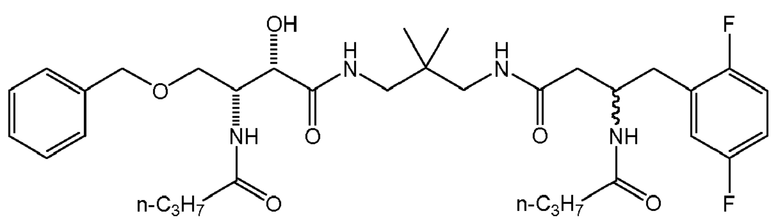

**41** (Scheme 3) was obtained from **36** using general procedure B.

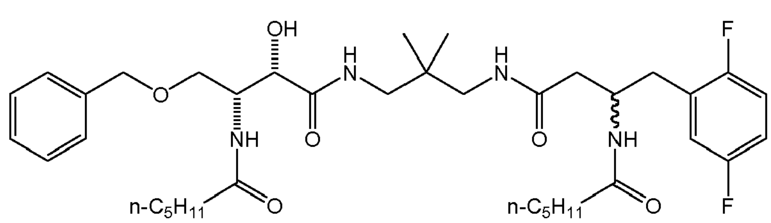

**42** (Scheme 3) was obtained from **37** and hexanoyl chloride using general procedure B.

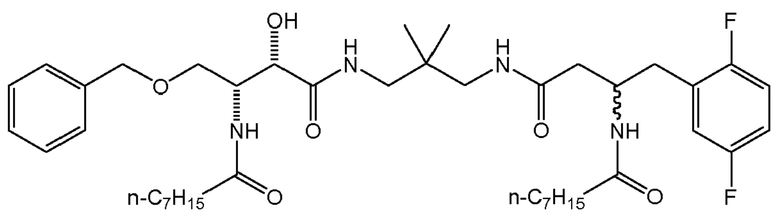

**43** (Scheme 3) was obtained from **38** using general procedure B.

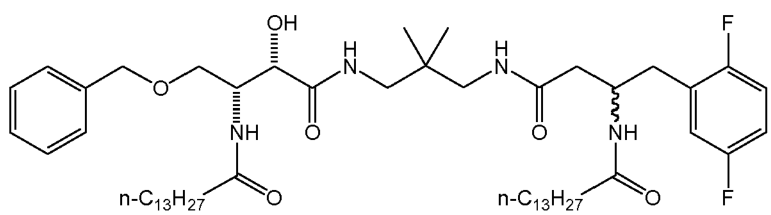

**44** (Scheme 3) was obtained from **39** using general procedure B.

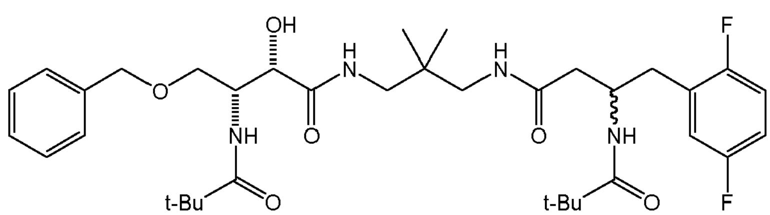

**45** (Scheme 3) was obtained from **40** using general procedure B.

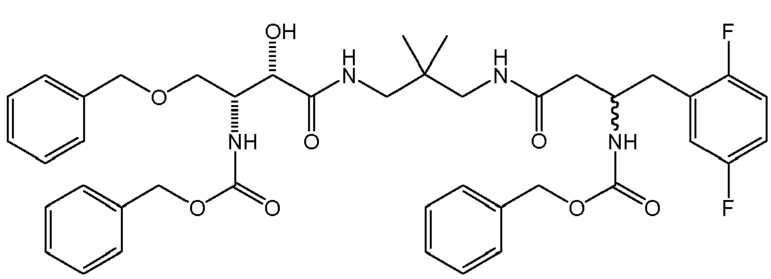

**46** (Scheme 3): To a solution of **26** (1.0 eq.) an $NaHCO_3$ (5.0 eq) in water (5 ml per mmol) was added a solution of benzyl chloroformate (2.5 eq.) in 1,4-dioxane (5 ml per mmol). The reaction mixture was stirred for seven hours at ambient temperature before the volatiles were evaporated. The residue was distributed between ethyl acetate and water and the layers were separated. The aqueous phase was extracted twice with ethyl acetate and the combined organic phases were washed with brine. After drying ($MgSO_4$) and evaporation of the solvents, **46** was purified by flash-chromatography on silica (eluent: dichloromethan/diethylether).

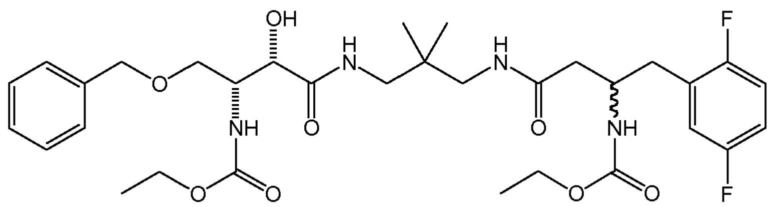

**47** (Scheme 3): To a solution of **26** (1.0 eq.) an $NaHCO_3$ (5.0 eq) in water (5 ml per mmol) was added a solution of ethyl chloroformate (2.5 eq.) in 1,4-dioxane (5 ml per mmol). The reaction mixture was stirred for seven hours at ambient temperature before the volatiles were evaporated. The residue was distributed between ethyl acetate and water and the layers were separated. The aqueous phase was extracted twice with ethyl acetate and the combined organic phases were washed with brine. After drying ($MgSO_4$) and evaporation of the solvents, **47** was purified by flash-chromatography on silica (eluent: dichloromethan/diethylether).

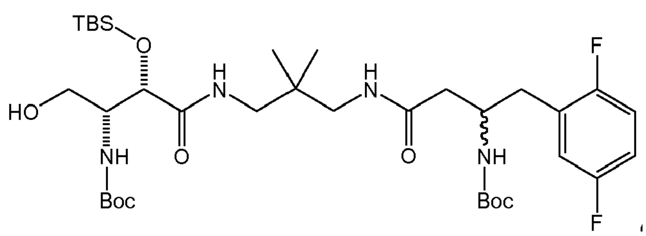

**48** (Scheme 4): A solution of **22** (1.0 eq.), imidazole (1.4 eq.) and t-butyldimethylchlorosilane (1.2 eq.) in N,N-dimethylformamid (1 ml per mmol) was stirred overnight and the filtered over a short path of silica (eluent: pentane/diethylether). The filtrate was evaporated and the residue was taken up in methanol. Palladium on charcoal (10 %, 0.05 eq.) was added and the suspension was stirred for 18 h und a hydrogen atmosphere. The reaction mixture was filtered and evaporated. **48** was purified by flash-chromatography on silica (eluent: dichloromethan/diethylether).

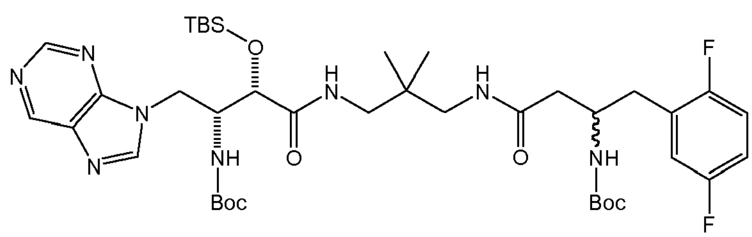

**49** (Scheme 4): A solution of **48** (1.0 eq.), purine (1.5 eq.) and triphenylphosphine (3 eq.) in THF (8 ml per mmol) was cooled to 0˚C and diisopropylazodicarboxylate (2.5 eq.) was added. The mixture was stirred for 24 h and tributylphosphine (3 eq.) and diisopropylazodicarboxylate (2.5 eq.) were added. After another 24 h, the solution was poured in brine and extracted thrice with ethyl acetate. The product was isolated by flash-chromatography on silica (eluent: ethyl acetate/methanol).

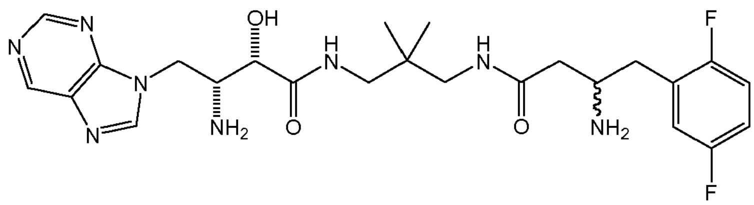

**50** (Scheme 4) was obtained from **49** using general procedure C.

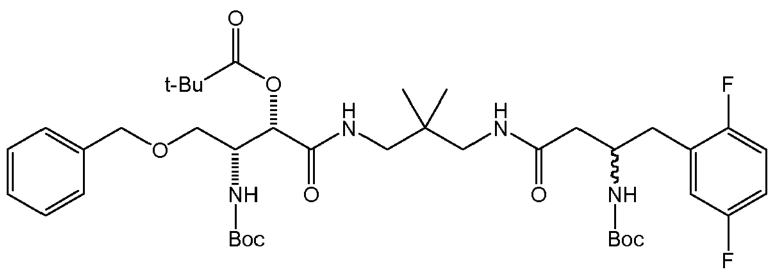

**51** (Scheme 5) was obtained from **22** and pivaloyl chloride using general procedure D.

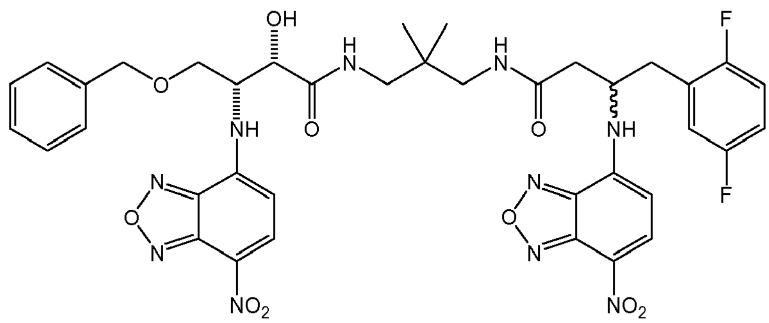

**52** (Scheme ): A solution of **26** (1.0 eq.), 4-fluoro-7-nitrobenzofurazane (2.2 eq.) and N-ethyldiisopropylamine (4.2 eq.) in ethanol (20 ml per mmol) was heated to reflux for three minutes. The volatiles were evaporated and residue subjected to flash chromatography (eluent: dichloromethane/ethyl acetate).

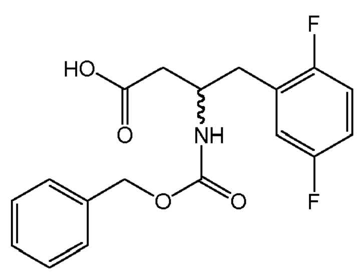

**54** (Scheme 7): **53** (1.0 eq.) was stirred at ambient temperature with trifluoroacetic acid/dichloromethane (1 : 1; 2 ml per mmol) for four hours before the volatiles were evaporated. The residue was dissolved in water (5 ml per mmol) and

$NaHCO_3$ (2.5 eq) and a solution of benzyl chloroformate (1.2 eq.) in 1,4-dioxane (5 ml per mmol). The reaction mixture was stirred for 17 h before the solvents were distilled off at reduced pressure. The residue was distributed between ethyl acetate and water and the layers were separated. The aqueous phase was extracted twice with ethyl acetate and the combined organic phases were washed with brine. After drying ($MgSO_4$) and evaporation of the solvents, **54** was purified by flash-chromatography on silica (eluent: dichloromethan/diethylether).

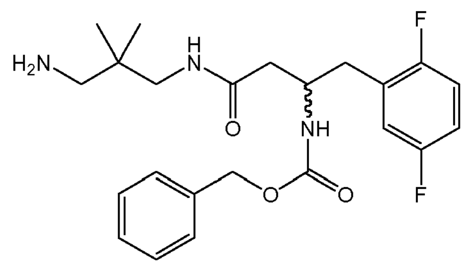

**55** (Scheme 7): A solution of **54** (1.0 eq.) in dichloromethane (10 ml per mmol) was cooled to 0˚C and 1-hydroxybenzotriazole (1.3 eq.) and N,N'-dicyclohexylcarbodiimid (1.5 eq.) were added. After 1 hour at this temperature, 1,2-diamino-2,2-dimethylpropane (5 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. Na-$HCO_3$ and saturated aq. NaCl. The organic phase was dried ($MgSO_4$) and evaporated. **55** was isolated by flash-chromatography on silica (eluent: dichloromethane/methanol/triethylamine).

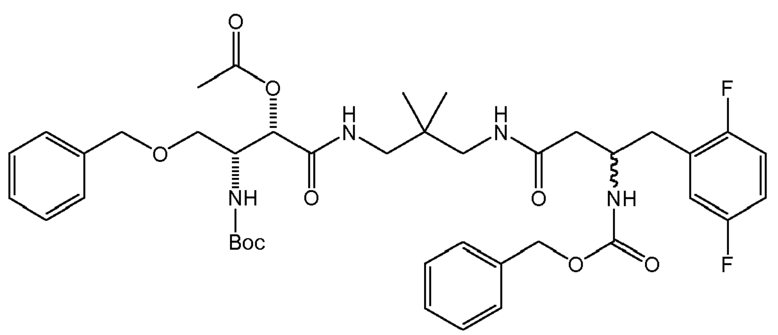

**56** (Scheme 7) was obtained from **55** and **13** using general procedure A.

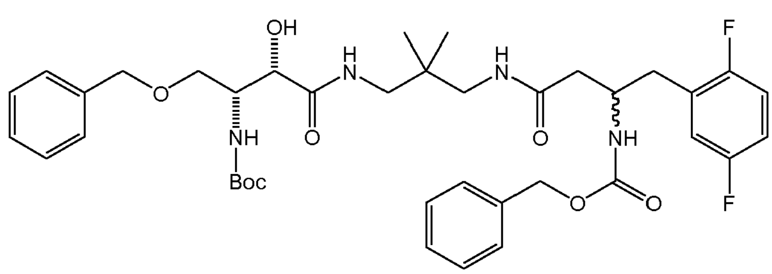

**57** (Scheme 7) was obtained from **56** using general procedure B.

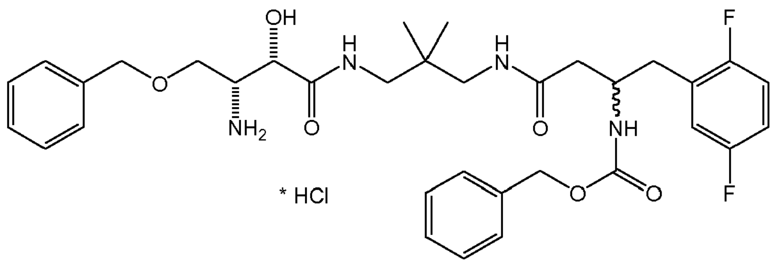

**58** (Scheme 7) was obtained from **57** using general procedure C.

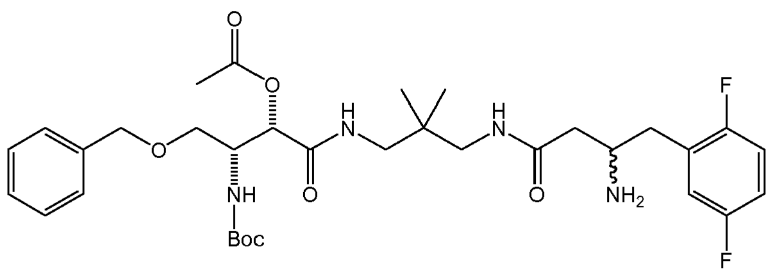

**59** (Scheme 7): Palladium on charcoal (10%, 0.05 eq.) was added to a solution of **56** (1.0 eq.) in methanol (10 ml per mmol). The resulting suspension was stirred for one hour under a hydrogen atmosphere. The solids were filtered of through a short path of celite and the filtrate was evaporated to obtain **59.**

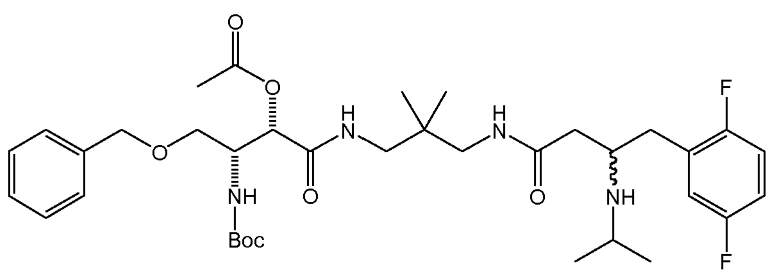

**60** (Scheme 7): A solution of **59** (1.0 eq.) and acetone (1.2 eq.) in THF (20 ml per mmol) was stirred for two hours with molecular sieves 3A (0.3 nm; 1g per mmol). Sodium cyanoborohydride (5.0 eq.) was added and the reaction was continued for 17 h. Saturated aq. $NaHCO_3$ (20 ml per mmol) was added and the mixture was extracted three times with ethyl acetate. The combined organic layers were dried ($MgSO_4$) and after evaporation **60** was isolated by by flash-chromatography on silica (eluent: dichloromethane/methanol).

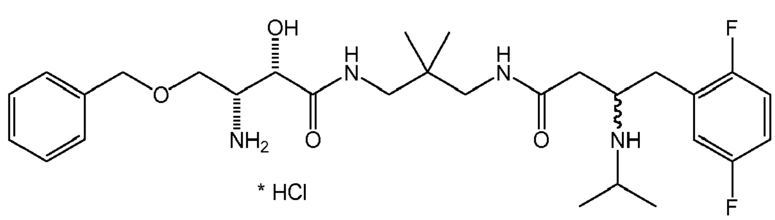

**61** (Scheme 7) was obtained from **60** through application of general procedure B, followed by the use of general procedure

C.

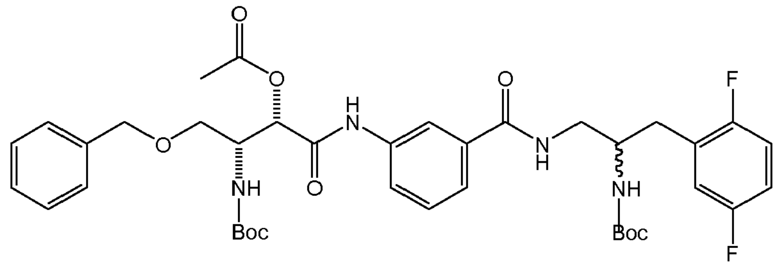

**63** (Scheme 8) was obtained from **62** and **13** using general procedure A.

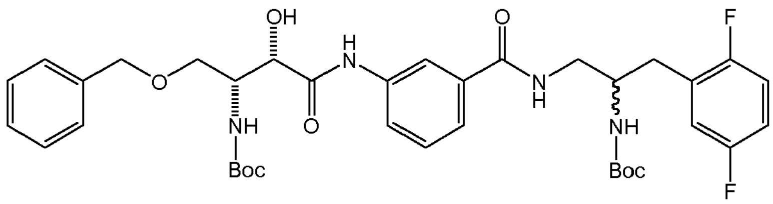

**64** (Scheme 8) was obtained from **63** using general procedure B.

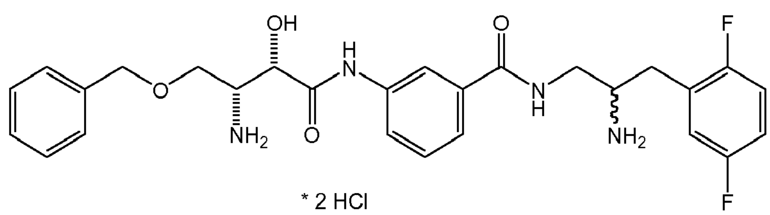

* 2 HCl

**65** (Scheme 8) was obtained from **64** using general procedure C.

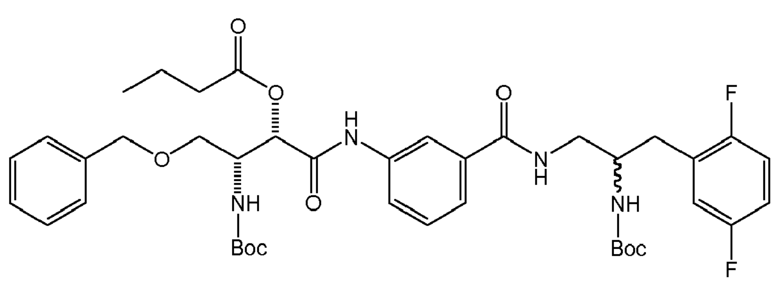

**66** (Scheme 8) was obtained from **64** and butyrylchloride using general procedure D.

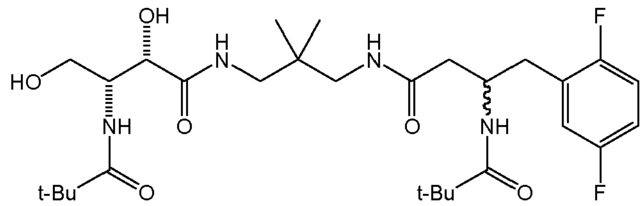

**67** (Scheme 9): Palladium on charcoal (10%, 0.2 eq.) was added to a solution of **45** (1.0 eq.) in methanol (10 ml per mmol). The resulting suspension was stirred for 18 h under a hydrogen atmosphere. The solids were filtered of through a short path of celite and the filtrate was evaporated to obtain **67.**

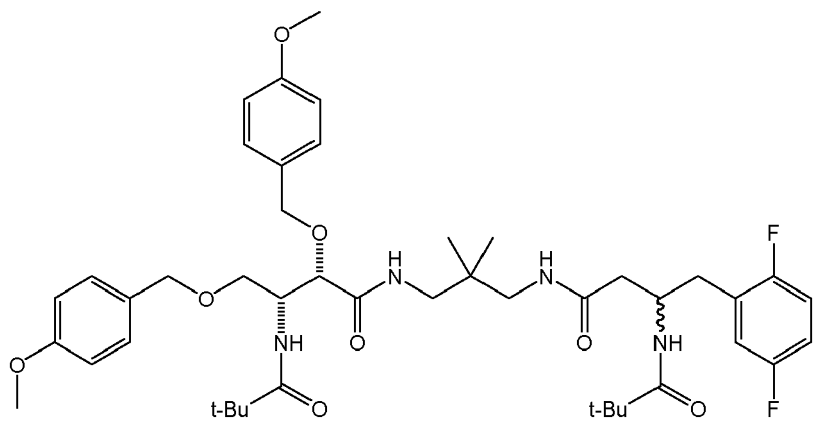

**68** (Scheme 9): To a cooled (0˚C) solution of **67** (1.0 eq.) in THF (3 ml per mmol) were added subsequently sodium hydride (60 %, 2.5 eq.), sodium iodide (4.0 eq.) and p-methoxybenzyl chloride (5.0 eq.). The reaction mixture was stirred at ambient temperature for 19 h. The reaction was quenched with saturated aq. $NH_4Cl$ (5 ml per ml THF) and the layers were separated. The aqueous phase was extracted twice with ethylacetate and the combined organic phases were washed with brine. After drying ($MgSO_4$) and evaporation of the solvents, **68** was purified by flash-chromatography on silica (eluent: dichloromethan/diethylether).

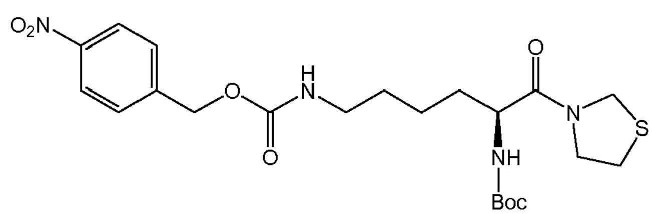

**70** (Scheme 10): A solution of **69** (1.0 eq.) in dichloromethane (10 ml per mmol) was cooled to 0 ˚C and 1-hydroxybenzotriazole (1.0 eq.) and N-(3-Dimethylaminopropyl)-N'-ethylcarbodiimide (1.2 eq.) were added. After 1 hour at this temperature, the thiazolidine (1.2 eq.) was added and the temperature was raised to ambient temperature. The suspension was stirred for 18 hours and prior to the addition of hydrochloric acid (2 ml per mmol). The phases were separated and the aqueous layer was extracted twice with dichloromethane. The combined organic phases were dried ($MgSO_4$) and evaporated. The crude product was purified by flash chromatography on silica (eluent: diethylether).

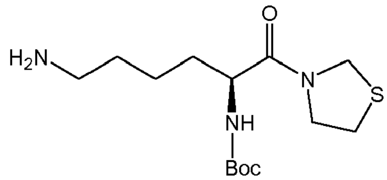

**71** (Scheme 10): Palladium on charcoal (10%, 0.05 eq.) was added to a solution of **70** (1.0 eq.) in methanol (10 ml per mmol). The resulting suspension was stirred for 18 h under a hydrogen atmosphere. The solids were filtered of through a short path of celite and the filtrate was evaporated. The crude **71** was purified by flash chromatography on silica (eluent: dichloromethane/methanol/triethylamine).

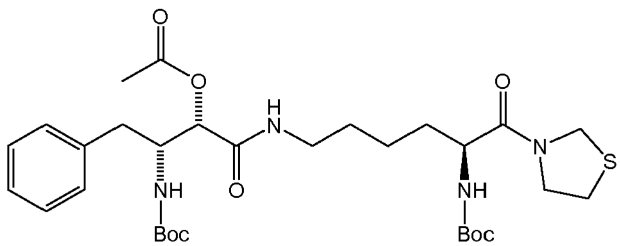

**73** (Scheme 10) was obtained from **71** and **72** using general procedure A.

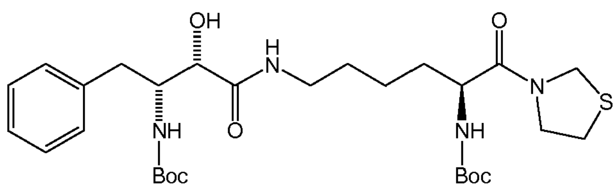

**74** (Scheme 10) was obtained from **73** using general procedure B.

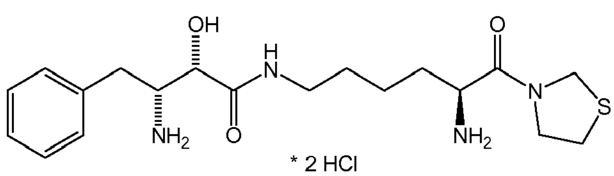

**75** (Scheme 10) was obtained from **74** using general procedure C.

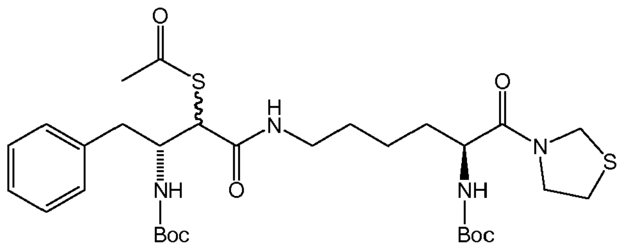

**76** (Scheme 10): A solution of **74** (1.0 eq.), triethylamine (3.0 eq.), mesylchloride (2.4 eq.) and 4-N,N-dimethylaminopyridine (0.2 eq.) in dichloromethane (15 ml per mmol) was stirred overnight at ambient temperature. The reaction was quenched with hydrochloric acid (1 M, 15 ml per mmol), the layers were separated and the aqueous layer was extracted twice with dichloromethane. The combined organic phases were dried ($MgSO_4$) and evaporated. The crude product was dissolved in DMF (2 ml per mmol) and potassium thioacetate (5 eq.) was added. The reaction mixture was stirred at 60 ˚C under an inert atmosphere for 17 h. Water and dichloromethane were added and the layers were separated. The aqueous layer was extracted twice with dichloromethane and the combined organic phases were dried ($MgSO_4$) and evaporated. **76** purified by flash chromatography on silica (eluent: dichloromethane/diethylether).

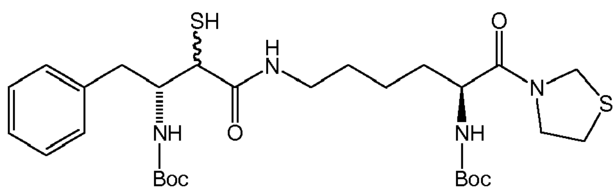

**77** (Scheme 10) was obtained from **76** using general procedure B.

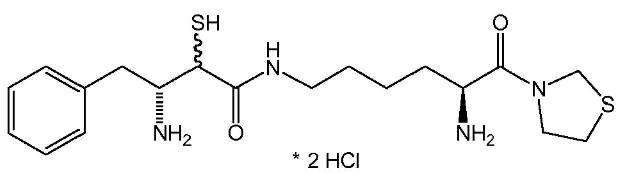

**78** (Scheme 10) was obtained from **77** using general procedure C.

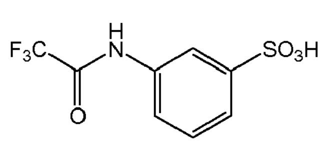

**80** (Scheme 11): A cooled (0 ˚C) suspension of **79** (1.0 eq.) in dichloromethane (1.5 ml per mmol) was treated with pyridine (1.2 eq.) and trifluoroacetic acid anhydride (1.1 eq.). The reaction mixture was stirred for four hours at ambient temperature. Hydrochloric acid (1 M, 3 ml per mmol) was added and the layers were separated. The aqueous layer was extracted three times with ethyl acetate and the combined organic phases were dried ($MgSO_4$) and evaporated. The crude product showed to be pure enough for further transformations.

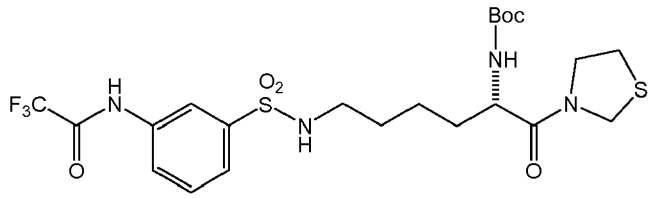

**81** (Scheme 11): A solution of **80** (1.5 eq.) in DMF (1 ml per mmol) was treated with thionylchloride (2.25 eq.) and stirred for three hours at ambient temperature. The rection was quenched with saturated aq. $NaHCO_3$ and extracted three times with ethylacetate. The combined organic layers were dried ($MgSO_4$) and evaporated under reduced pressure. The residue was taken up in dichloromethane (10 ml per mmol), **71** (1.0 eq.) and triethylamine (2.5 eq.) were added, and the mixture was stirred overnight. Hydrochloric acid (1 M, 3 ml per mmol) was added and the layers were separated. The aqueous layer was extracted twice with dichloromethane and the combined organic phases were dried ($MgSO_4$) and evaporated. **81** purified by flash chromatography on silica (eluent: dichloromethane/diethylether).

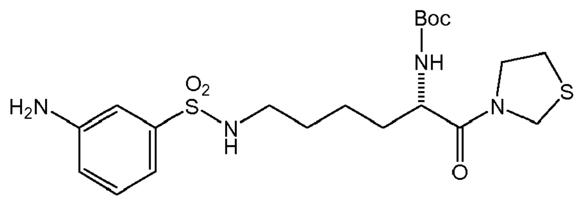

**82** (Scheme 11) was obtained from **81** by adapting general procedure B.

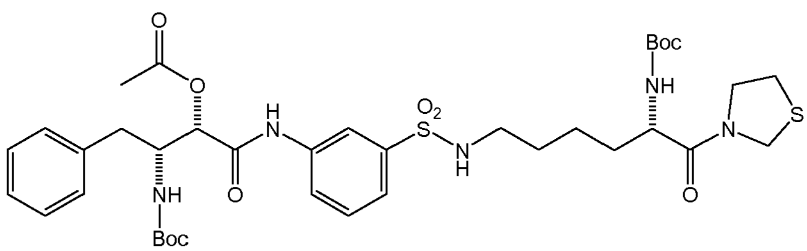

**83** (Scheme 11) was obtained from **82** and **72** using general procedure A.

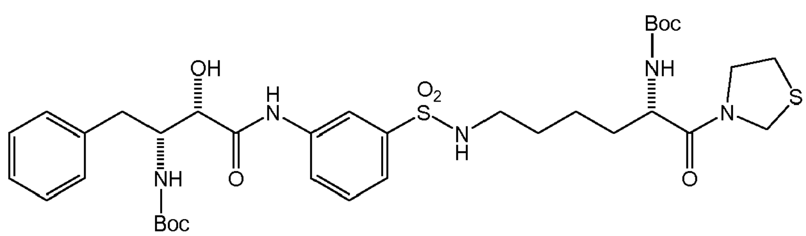

**84** (Scheme 11) was obtained from **83** using general procedure B.

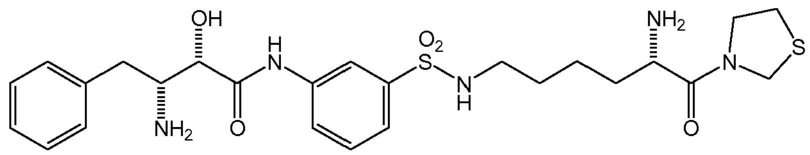

* 2 HCl

**85** (Scheme 11) was obtained from **84** using general procedure C.

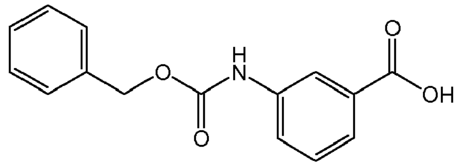

**87** (Scheme 12): To a solution of **86** (1.1 eq.) an $NaHCO_3$ (2.0 eq) in water (5 ml per mmol) was added a solution of benzyl chloroformate (1.0 eq.) in 1,4-dioxane (5 ml per mmol). The reaction mixture was stirred for seven hours at ambient temperature before the volatiles were evaporated. The residue was distributed between ethyl acetate and water and the layers were separated. The aqueous phase was extracted twice with ethyl acetate and the combined organic phases were washed with brine. After drying ($MgSO_4$) and evaporation of the solvents, the crude product was recrystallized from MTBE/pentane.

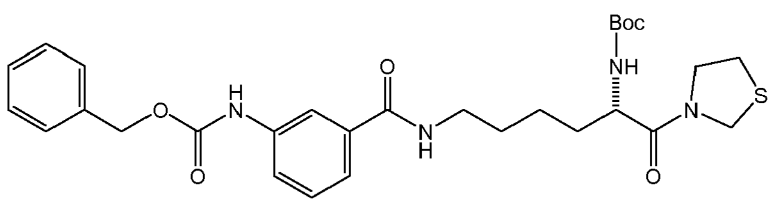

**88** (Scheme 12) was obtained from **87** and **71** using general procedure A.

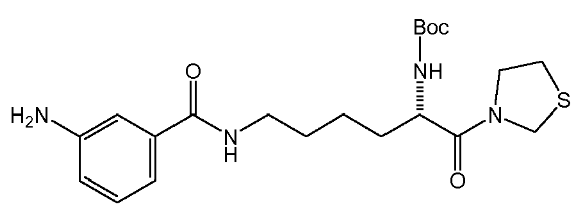

**82** (Scheme 12): Palladium on charcoal (10%, 0.05 eq.) was added to a solution of **70** (1.0 eq.) in methanol (10 ml per mmol). The resulting suspension was stirred for 18 h under a hydrogen atmosphere. The solids were filtered off through a short path of celite, the filtrate was evaporated, and the obtained product **82** was used without further purification.

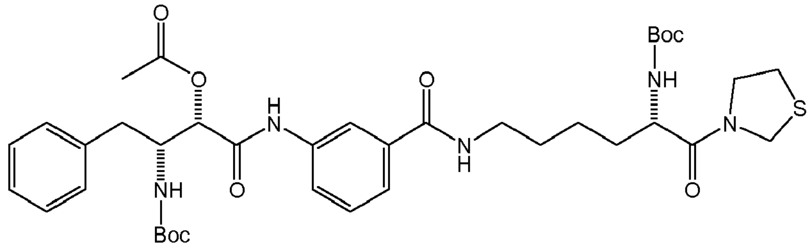

**90** (Scheme 12) was obtained from **89** and **72** using general procedure A.

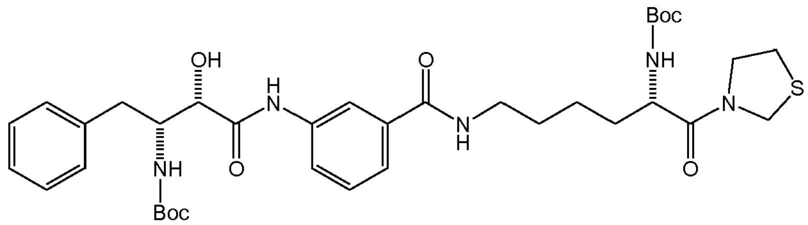

**91** (Scheme 12) was obtained from **90** using general procedure B.

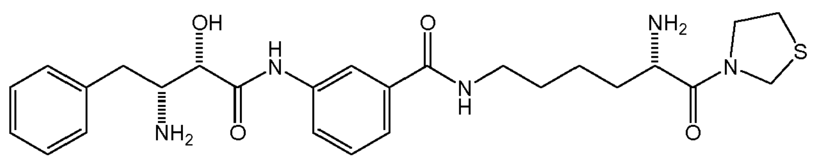

* 2 HCl

**92** (Scheme 12) was obtained from **91** using general procedure C.

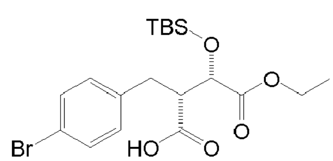

**94** (Scheme 13): A solution of **93** (3,00 g, 9.06 mmol) in DMF (9 ml) was treated with *tert*-butyl-dimethyl-silylchloride (3.28 g, 21.8 mmol) and imidazole (2.68 g, 39.8 mmol) for three hours. The reaction was quenched with 1 M hydrochloric acid and extracted twice with diethylether. The combined organic phases were evaporated and the residue was stirred with 0.5 M $K_2CO_3$ (MeOH/water 3 : 1; 100 ml). After two hours the pH was adjusted to 2 with conc. hydrochloric acid and the mixture was extracted into diethylether. The combined organic phases were dried ($MgSO_4$) and the solvent was evaporated under reduced pressure. **94** (2.75 g, 68 %) was obtained by flash chromatography on silica (eluent: pentane/ diethylether).

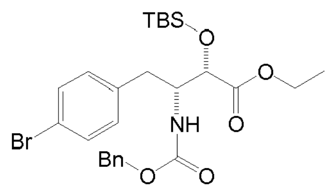

**95** (Scheme 13): A solution of **94** (2.51 g, 5.64 mmol). triethylamine (934 μl, 6.77 mmol) and DPPA (1.34 ml, 6.20 mmol) in toluene (20 ml) was heated to 80 ˚C. After two hours benzyl alcohol (1.75 ml, 16.9 mmol) was added and heating was continued for three hours. After cooling to ambient temperature water (100 ml) was added and the mixture was extracted with ethyl acetate. The combined organic phases were dried ($MgSO_4$) and the solvent was evaporated under reduced pressure. **95** (2.44 g, 78 %) was obtained by flash chromatography on silica (eluent: pentane/diethylether).

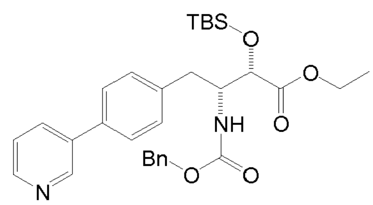

**97** (Scheme 13): A solution of **65** (909 mg, 1.65 mmol), **96** (510 mg, 2.47 mmol), and $CsCO_3$ (809 mg, 2.47 mmol) in DME (10 ml) was degassed by purging with nitrogen for 15 min.. $Pd(PPh_3)_4$ (95 mg, 83 μmol) was added and the resulting mixture was heated for four hours to 120 ˚C in a sealed tube. After cooling to ambient temperature 1 M hydrochloric acid (50 ml) was added and the mixture was extracted with ethyl acetate. The combined organic phases were dried ($MgSO_4$) and the solvent was evaporated under reduced pressure. 97 (865 mg, 96%) was obtained by flash chromatography on silica (eluent: diethylether).

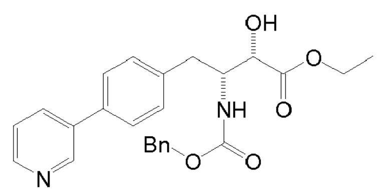

**98** (Scheme 13) was obtained from **97** using general procedure C.

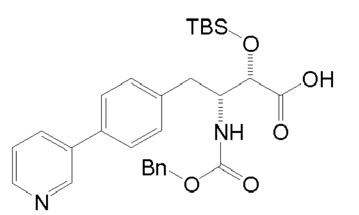

**99** (Scheme 13) was obtained from **97** using general procedure B.

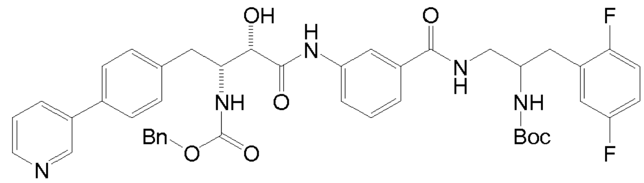

**100** (Scheme 13) was obtained from **99** and **62** using general procedure A.

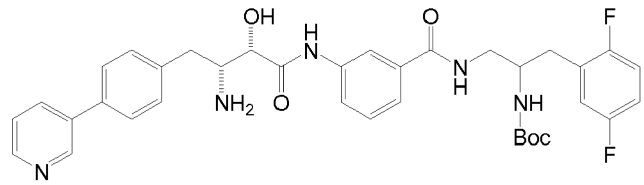

**101** (Scheme 13): Palladium on charcoal (10%, 0.2 eq.) was added to a solution of **100** (1.0 eq.) in methanol (10 ml per mmol). The resulting suspension was stirred for 18 h under a hydrogen atmosphere. The solids were filtered of through a short path of celite and the filtrate was evaporated to obtain **101.**

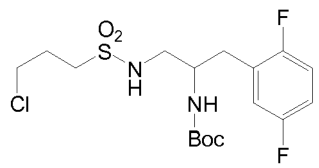

**104** (Scheme 14): A solution of **102** (282 mg, 0.986 mmol), **103** (144 μl, 1.18 mmol) and triethylamine (206 μl, 1.50 mmol) in dichloromethane (5 ml) was stirred at ambient temperature over night. 1 M hydrochloric acid (10 ml) was added and the mixture was extracted with dichloromethane. The combined organic phases were dried ($MgSO_4$) and the solvent was evaporated under reduced pressure. **104** (300 mg, 71 %) was obtained by flash chromatography on silica (eluent: pentane/diethylether).

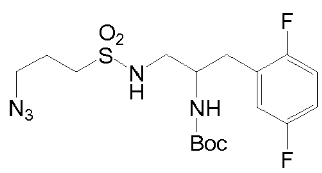

**105** (Scheme 14): A solution of **104** (299 mg, 0.7 mmol) and $NaN_3$ (91 mg, 1.4 mmol) in DMF (7 ml) was stirred over night at 50 ˚C. After cooling to ambient temperature water (50 ml) was added and the mixture was extracted with dichloromethane. The combined organic phases were dried ($MgSO_4$) and the solvent was evaporated under reduced pressure to give pure **105** (300 mg, quant.).

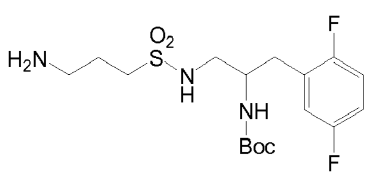

**106** (Scheme 14): Palladium on charcoal (10%, 0.2 eq.) was added to a solution of **105** (1.0 eq.) in methanol (10 ml per mmol). The resulting suspension was stirred for 18 h under a hydrogen atmosphere. The solids were filtered of through a short path of celite and the filtrate was evaporated to obtain **105.**

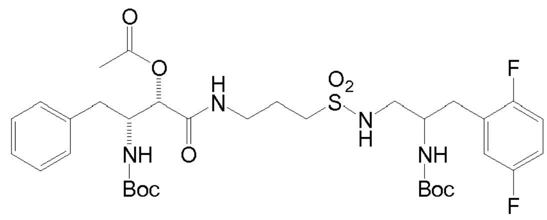

**107** (Scheme 14) was obtained from **106** and **72** using general procedure A.

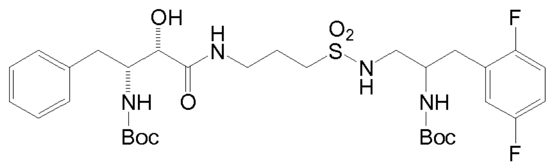

**108** (Scheme 14) was obtained from **107** using general procedure B.

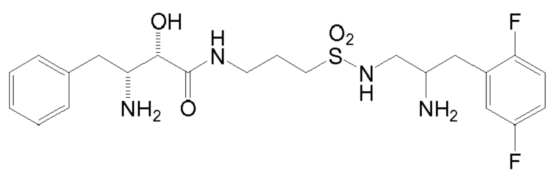

**109** (Scheme 14) was obtained from **108** using general procedure C.

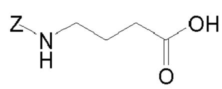

**111** (Scheme 15): **110** (6.19 g, 60.1 mmol) was dissolved in water (25 ml) and $NaHCO_3$ (5.55 g, 66.1 mmol) and a solution of benzyl chloroformate (8.12 ml, 57.1 mmol) in 1.4-dioxane (25 ml) was added. The reaction mixture was stirred for 17 h before the solvents were distilled off at reduced pressure. The residue was distributed between ethyl acetate and 1 M hydrochloric acid and the layers were separated. The aqueous phase was extracted twice with ethyl acetate, the combined organic phases were washed with brine and dried ($MgSO_4$). Evaporation of the solvent delivered pure **111** (12.8 g, 95 %).

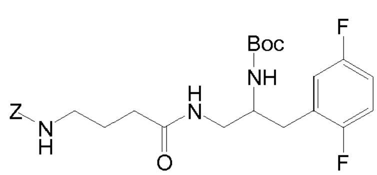

**112** (Scheme 15) was obtained from **111** and **103** using general procedure A.

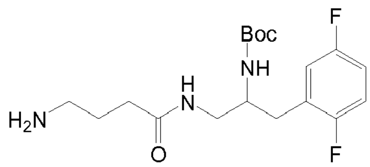

**113** (Scheme 15): Palladium on charcoal (10%, 0.2 eq.) was added to a solution of **112** (1.0 eq.) in methanol (10 ml per mmol). The resulting suspension was stirred for 18 h under a hydrogen atmosphere. The solids were filtered of through a short path of celite and the filtrate was evaporated to obtain **113.**

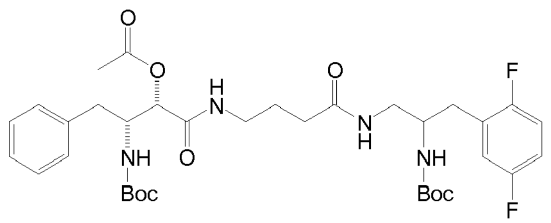

**114** (Scheme 15) was obtained from **113** and **72** using general procedure A.

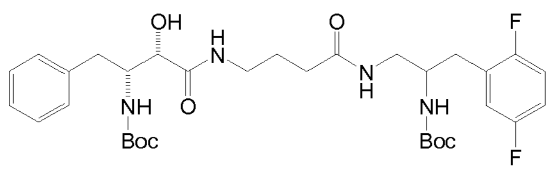

**115** (Scheme 15) was obtained from **114** using general procedure B.

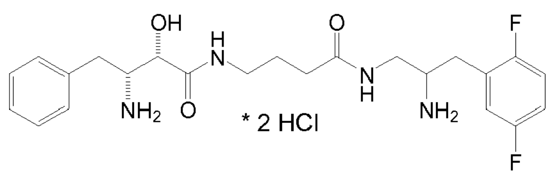

**116** (Scheme 15) was obtained from **115** using general procedure C.

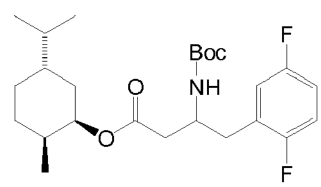

**117** (Scheme 16): A solution of **53** (104 mg, 330 μmol) in dichloromethane (5 ml), L-menthol (103 mg, 660 μmol) and

DMAP (44.3 mg, 363 μmol) was cooled to 0 °C and N,N'-dicyclohexylcarbodiimid (88.6 mg, 429 μmol) was added. After 1 hour at this temperature the temperature was raised to ambient temperatur. The suspension was stirred for 18 hours and prior to filtration diluted with ethyl acetate (25 ml). The filtrate was subsequently washed with 1 M hydrochloric acid, saturated aq. $NaHCO_3$ and saturated aq. NaCl. The organic phases was dried ($MgSO_4$) and evaporated. The crude product was purified by flash chromatography on silica (eluent: pentane/diethylether) to give **117** (122 mg, 82 %).

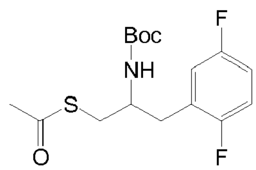

**119** (Scheme 17): A solution of **118** (2.05 g, 7.14 mmol) in dichloromethane (20 ml) was cooled to 0 °C. Triethylamine (1.19 ml, 8.57 mmol) and methanesulfonyl chloride (608 μl, 7.86 mmol) were added. The resulting mixture was stirred for two hours at ambient temperature. 1 M hydrochloric acid (20 ml) was added and the mixture was extracted with dichloromethane. The combined organic phases were dried ($MgSO_4$) and the solvent was evaporated under reduced. The residue was dissolved in DMF (6 ml) and KSAc (979 mg, 8.57 mmol) was added. The reaction mixture was stirred over night at ambient temperature. The mixture was distributed between diethylether and water and the layers were separated. The aqueous phase was extracted twice with diethylether, the combined organic phases were washed with brine and dried ($MgSO_4$). The crude product was purified by flash chromatography on silica (eluent: pentane/diethylether) and **119 (1.84** g, 75 %) was obtained.

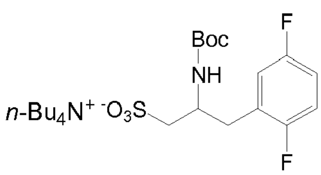

**120** (Scheme 17): Oxone (8.2 g, 13.3 mmol) in water (160 ml) was added to a solution of **119** (1.84 g, 5.33 mmol) in methanol (160 ml) and stirred for 30 min at ambient temperature. *n*-$Bu_4NOH$ (8.2 ml 40% in water) in water (40 ml) was added and the resulting solution was stirred over night. The methanol was almost complete evaporated and the aqueous residue was extracted four times with dichloromethane. The combined organic phases were washed with brine and dried ($MgSO_4$). Evaporation of the solvent delivered crude **120** (3.75 g), which was used without further purification.

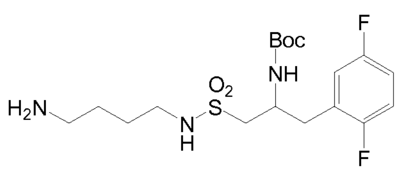

**122** (Scheme 17): A solution of **120** (200 mg, 337 μmol), DMF (98 μl, 1.35 mmol) and $SOCl_2$ (52.2 μl, 675 μmol) in dichloromethane was stirred over night at ambient temperature. All volatiles were evaporated to obtain crude **121,** which was taken up in dichloromethane (10 ml). 1.4-Diaminobutane (339 μl, 3.37 mmol) was added and the resulting mixture was stirred for 17 hours at ambient temperature. The reaction was quenched with saturated aq. $NaHCO_3$, the layers were separated and the aqueous layer was extracted three times with dichloromethane. The combined organic phases were dried ($MgSO_4$) and the evaporation of the solvents delivered crude **122** (81 mg), which was used without further purification.

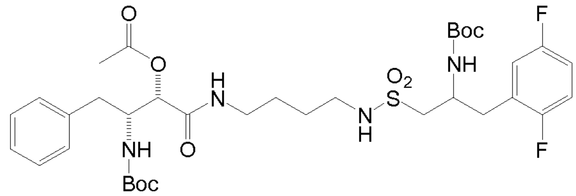

**123** (Scheme 17) was obtained from **122** and **72** using general procedure A.

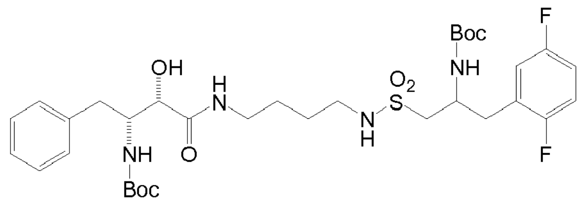

1

**124** (Scheme 17) was obtained from **123** using general procedure B.

## Claims

**1.** Compounds of the general formula (1)

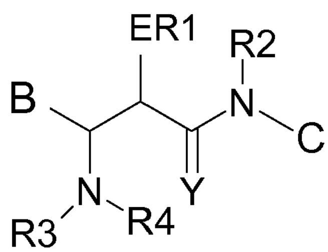

(I)

wherein the residues R1, R2, R3 and R4 may be the same or different and are independently selected from the group consisting of -H; -halogen (i. e. -F, -Cl, -Br, -I); alkyl having 1 to 25 carbon atoms, which alkyl may be straight chain or branched, saturated or once, twice or more times unsaturated (-C=C- double bonds and/or -C≡C- triple bonds), unsubstituted or substituted with any of the residues R1, R2, R3 and/or R4, and/or uninterrupted or interrupted by any of the residues -O-, -NH-, -NR5-, -S-; >C(=O), -C(=O)O-, -O-C(=O)-, -C(=O)NH-, -C(=O)NR5-, -NHC(=O)-, -NR5(C=O)-, >C(=S), -C(=S)O-, -O-C(=S)-, -C(=S)NH-, -C(=S)NR5-, -NHC(=S)-, -NR5(C=S)-, -PH-, -PR5-, >P(=O)$H_2$, >P(=O)H, >P(=O)R5, >P(=O)(OH), >P(=O)OR5; cycloalkyl having 3 to 9 ring members, which cycloalkyl may be saturated or once, twice or more times unsaturated (-C=C- double bonds and/or -C≡C- triple bonds), unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; aryl having 3 to 9 ring members, which aryl may be unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<,- S- and -P<; which cycloalkyl and/or aryl groups may form non-condensed ring systems or ring systems comprising one, two or more condensed rings selected from cycloalkyl, heretocycloalkyl, aryl or heteroaryl rings; -OH, -OR5, -$NH_2$, -NHR5, -NR5R6, -C(=O)H, -C(=O)R5, -C(=O)OH, -C(=O)OR5, -C(=O)$NH_2$, -C(=O)NHR5, -C(=O)NR5R6, -NH-C(=O)H, -NR5(C=O)H, -NH-C(=O)R5, -NR5(C=O)R5, -C(=S)OH, -C(=S)OR5, -C(=S)$NH_2$, -C(=S)NHR5, -C(=S)NR5R6, -O-C(=O)H, -OC(=O)R5, -NH(C=O)R5, -NR5(C=O)R6, -C(=O)(NHOH), -C(C=O)(NR5OH) -C(C=O)(NR5OR6) -C(C=O)NHOR5, -$PH_2$, -PHR5, -PR5R6, -P(=O)$H_2$, -P(=O)R5H, -P(=O)R5R6, -P(=O)$(OH)_2$, -P(=O)R5OH -P(=O)OR5OR6;

wherein R5 and R6 may be the same or different and may be selected from the group of residues defined above by R1, R2, R3 and R4;

E may represent a group selected from -O-, -S-, -NH- or -NR7-, wherein R7 is a group which may be selected from the group of residues defined above by R1,R2, R3 and R4;

Y may represent a group selected from -O-, -NH-, -NR8-, -S-, -$CH_2$-, -CHR8-and -CR8R9-, wherein R8 and R9 may be the same or different and may be selected from the group of residues defined above by R1, R2, R3 and R4;

B may represent a group having the general formula (II)

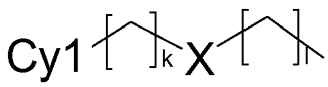

**or**

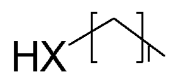

(IIa) (IIb)

wherein

Cy1 may represent condensed or non condensed, aromatic or non aromatic homo- or heterocyclic systems having 3 to 9 ring members, and, in the case of a condensed system, 3 to 9 ring members in each partial ring which, in the case of non aromatic moieties, may be saturated or once, twice or more times unsaturated (-C=C- double bonds and/or -C≡C- triple bonds), and Cy1 may be unsubstituted or substituted with any of the residues R1, R2, R3 and/or R4, and/or may comprise one or several hetero-atoms within the ring structure, which heretoatoms may be selected from the group consisting of -O-, unsubstituted or alkyl-substituted -N<, - S- and -P<; aryl having 3 to 9 ring members, and, in the case of a condensed system, 3 to 9 ring members in each partial ring, which aryl may be unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; which cycloalkyl and/or aryl groups may form non-condensed ring systems or ring systems comprising one, two or more condensed rings selected from cycloalkyl, heretocycloalkyl, aryl or heteroaryl rings;

X may represent a single bond, -O-, -S-, -NH-, -NR10-, -$CH_2$-, -CHR10-, -CR10R11-, >C(=O), >C(=S), >C(=NH), >C(=NR10), -C(=O)O-, -C(=S)O-, -C(=NH)NH-, -C(=O)NH-, -C(=O)NR10-, -O(C=O)-, -NH(C=O)-, -NR10(C=O)-, -O(C=S)-, -NH(C=S)-, or -NR10(C=S)-, wherein R10 and R11 may be the same or different and may be selected from the group of residues defined above by R1, R2, R3 and R4;

k and l may be the same or different and represent zero (0) or may be integers in the range of from 1 to 5;

C may represent a group having the general formula (III)

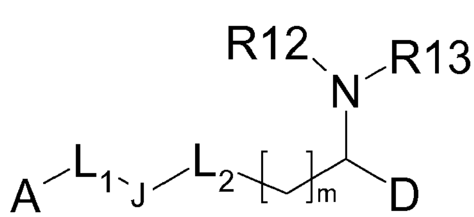

(III)

wherein

m may be the same or different and represent zero (0) or may be integers in the range of from 1 to 5;

the sequence A - L1 - J - L2 as a whole may be a single bond, or

A may be absent or may be selected from the group consisting of residues defined as for R1 above, with the proviso that the carbon chain may have 1 to 10 carbon atoms; and

J may be absent or may be selected from the group consisting of alkylene having 1 to 10 carbon atoms, which alkylene may be straight chain or branched, saturated or once, twice or more times unsaturated (-C=C- double bonds and/or -C≡C- triple bonds), unsubstituted or substituted with any of the residues R1, R2, R3 and/or R4, and/or uninterrupted or interrupted by any of the residues -O-, -NH-, -NR5-, -S-; >C(=O), -C(=O)O-, -O-C(=O)-, -C(=O)NH-, -C(=O)NR5-, -NHC(=O)-, -NR5(C=O)-, >C(=S), -C(=S)O-, -O-C(=S)-, -C(=S)NH-, -C(=S)NR5-, -NHC(=S)-, -NR5(C=O)-, -PH-, -PR5-, >P(=O)$H_2$, >P(=O)H, >P(=O)R5, >P(=O)(OH), >P(=O)OR5; cycloalkylene having 3 to 9 ring members, which cycloalkylene may be saturated or once, twice or more times unsaturated (-C=C- double bonds and/or -C≡C- triple bonds), unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; arylene having 3 to 9 ring members, which arylene may be unsubstituted or substituted with any of the residues R1, R2, R3 and/or R4, and/or may comprise one or several heteroatoms within the ring structure, which heretoatoms may be selected from the group consisting of -O-, unsubstituted or alkyl-substituted -N<, -S- and -P<; which cycloalkylene and/or arylene groups may form non-condensed ring systems or ring systems comprising one, two or more condensed rings selected from cycloalkyl, heretocycloalkyl, aryl or heteroaryl rings; -NH-, -NR5-, -C(=O)-, -C(=O)O-, -C(=O)R5-, -C(=O)NH-, -C(=O)NR5-, -NH-C(=O)-, -NR5-C(=O)-, -C(=S)O-, -C(=S)R5-, -C(=S)NH-, -C(=S)NHR5-, -C(=S)NR5- -NH-C(=O)-, -NR5-C(=O)-, -C(=O)(NHO)-, -C(=O)(NR5O)-, -PH-, -PR5-, -P(=O)H-, -P(=O)R5-, -P(=O)(OH)-, -P(=O)OR5-; wherein R5 and R6 may be the same or different and may be selected from the group of residues defined above by R1, R2, R3 and R4;

L1 and L2 may be the same or different and may represent a single bond or may represent moieties each independently selected from the group consisting of -$CH_2$-, -O-, >C=O, -NH-, -NR12-, -S-, >C=S, -$SO_2$-, -C(=O)-O-, -C(=S)-O-, -C(=O)-S-, -C(=S)-S-, -C(=O)NH-, -C(=O)NR14-, -C(=S)NH-, -C(=S)NR14-, -C(=NH)-, -C(=NH)-NH-, -C(=NH)-NR14-, -C(=NR1)-NR14-, wherein R12, R13 and R14 may be the same or different and may be selected from the group of residues defined above by R1, R2, R3 and R4; and

D represents any of the structures (IVa) or (IVb)

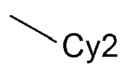 (IVa) 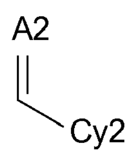 (IVb)

wherein

Cy2 is homo- or heterocyclic, non-aromatic or aromatic, uncondensed or once or twice condensed, annelated structural element and binds directly to the rest of the structure, which, in the case of heteroaromatic residues, may contain the groups -N=, -NH-, -NR1-, -S-, -O-, -S(=O)-, -S(=O)$_2$-, -P=, -PH-, -PR15-, -P(=O)-, -OP(=O)- and -P(=O)O- as the ring members, where carbon or a heteroatom moiety may be the connecting unit to structural part C (IVa) and =A2 (IVb), respectively, wherein, in the case of non-aromatic moieties Cy2, the ring structures making up Cy2 may be saturated, may be partially unsaturated, may be unsubstituted or substituted once, twice or more times at any chemically possible position by any of the substituents defined above as substituents for cycloaliphatic and aromatic residues, and Cy2 may comprise 3 to 9 ring members, and, in the case of a condensed system, 3 to 9 ring members in each partial ring; and

A2 may represent a group selected from =C, =CH, =CR16, -O-, -S-, -NH- and -NR16-, wherein R15 and R16 may be the same or different and may be selected from the group of residues defined above by R1, R2, R3 and R4.

**2.** Compounds having the general formula (1) according to claim 1, which compounds are the compounds of the following Table 1:

Table 1.

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C18.001 | 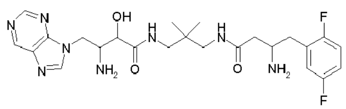 | 0.2470 | 0.9100 | 16.0000 | 1.0000 | 29.8 | | 156.37 | -10.12 | -6.46 |
| C18_A01 | 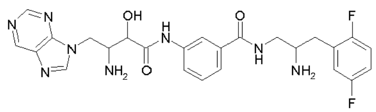 | | | | | | | | -11.32 | -9.01 |
| C18_A02 | 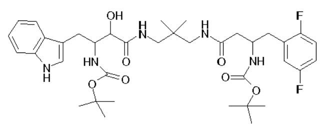 | | | | | | | | -8.99 | -6.79 |
| C18_A03 | 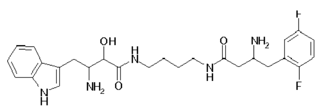 | | | | | | | | -10.28 | -7.48 |
| C18_A04 | 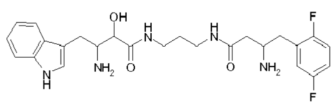 | | | | | | | | -10.37 | -7.22 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C18_A05 | 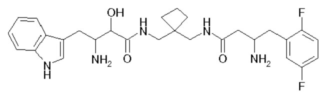 | | | | | | | | -10.53 | -7.98 |
| C18_A06 | 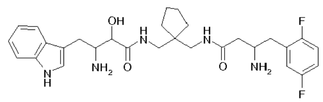 | | | | | | | | -10.15 | -7.94 |
| C18_A07 | 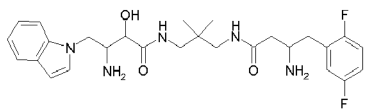 | | | | | | | | -10.04 | -8.53 |
| C18_A08 | 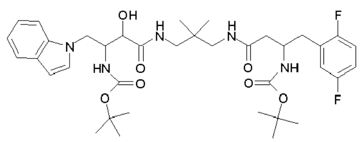 | | | | | | | | -8.63 | -9.04 |
| C18_A09 | 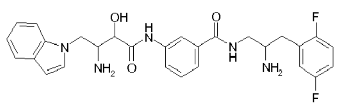 | | | | | | | | -11.32 | -8.22 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C19.001 | 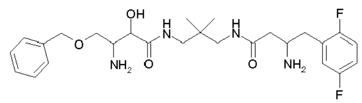 | 0.0560 | 0.0076 | 1.2500 | 0.0310 | 28.1 | 90.70 | 77.43 | -9.80 | -8.56 |
| C19_A01 | 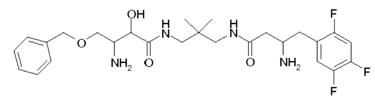 | | | | | | | | -10.30 | -8.57 |
| C19_A02 | 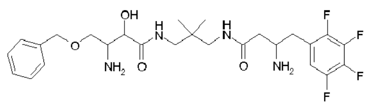 | | | | | | | | -9.75 | -8.56 |
| C19_A03 | 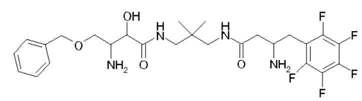 | | | | | | | | -10.15 | -8.07 |
| C19_A04 | 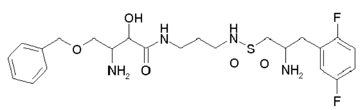 | | | | | | | | -11.63 | -9.09 |
| C19_A05 | 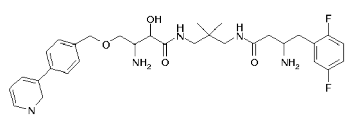 | | | | | | | | -11.05 | -9.04 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C19_A06 | 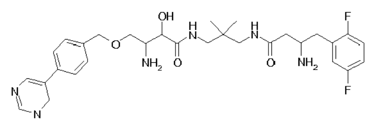 | | | | | | | | -10.91 | -7.91 |
| C19_A07 | 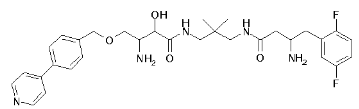 | | | | | | | | -11.05 | -8.82 |
| C19_A08 | 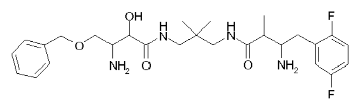 | | | | | | | | -9.94 | -7.83 |
| C19_A09 | 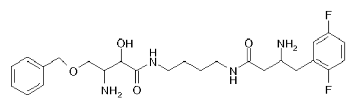 | | | | | | | | -9.80 | -7.12 |
| C19_A10 | 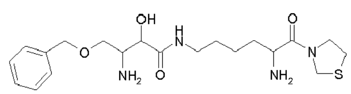 | | | | | | | | -10.60 | -8.93 |
| C19_A11 | 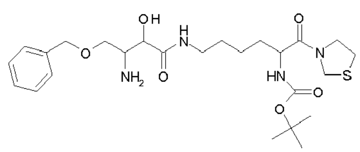 | | | | | | | | -11.01 | -8.44 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C19_B01 | 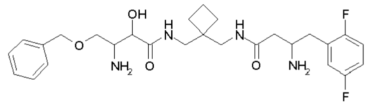 | | | | | | | | -9.81 | -7.42 |
| C19_B02 | 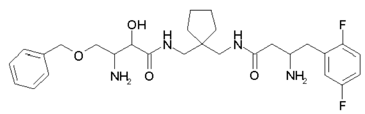 | | | | | | | | -10.67 | -8.98 |
| C19_B03 | 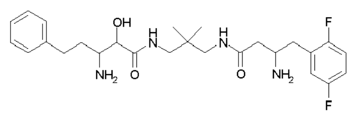 | | | | | | | | -10.71 | -8.27 |
| C19_B04 | 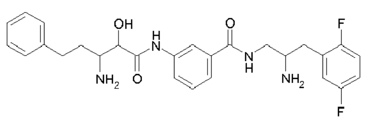 | | | | | | | | -10.91 | -8.37 |
| C19_B05 | 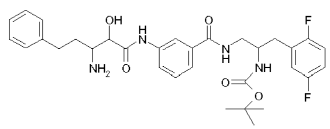 | | | | | | | | -10.39 | -8.90 |

(continued)

| Compond | Structure | meas. IC50 (DPIV) /µM | meas. IC50 (APN) /µM | meas. IC50 (DP8/9) /µM | meas. IC50 (cAAP) /µM | Supp. DNA Synt. T-cell IC50 (µM) | Supp. DNA Synt. NHEK IC50 (µM) | Supp. DNA Synt. SZ95 IC50 (µM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C19_B06 | 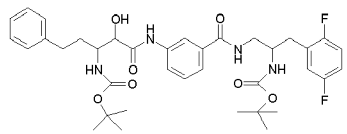 | | | | | | | | -10.80 | -8.04 |
| C19_B07 | 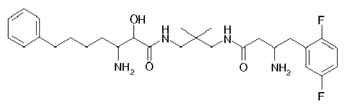 | | | | | | | | -9.87 | -8.22 |
| C19.002 mixture) | 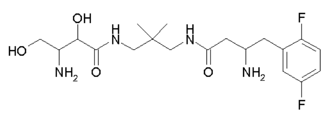 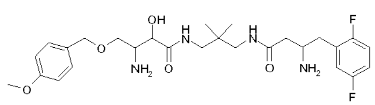 | 0.0470 | 0.1200 | 1.3000 | 0.3100 | 174.0 | | 200.00 | -9.79 | |
| C19.003 | 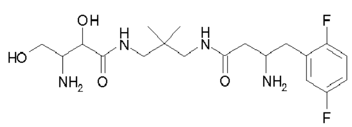 | 0.3300 | 0.9200 | 3.8000 | 0.3700 | 81.2 | | 81.43 | -9.31 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol1) | $\Delta E_{CPBS}$ (APN)/ kcal/mol12) |
|---|---|---|---|---|---|---|---|---|---|---|
| C19.004 | 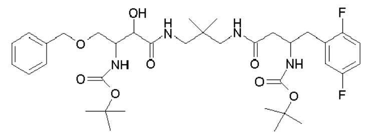 | no inhibition | no inhibition | no inhibition | no inhibition | 9.7 | 19.75 | 18.33 | -8.19 | -0.48 |
| C19_C01 | 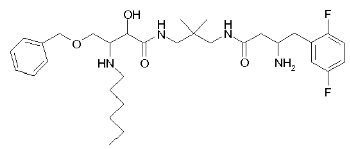 | | | | | | | | -11.12 | -7.38 |
| C19_C02 | 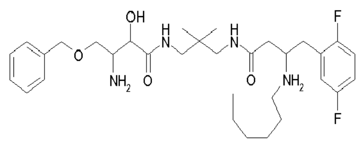 | | | | | | | | -11.05 | -7.13 |
| 7C19_C03 | 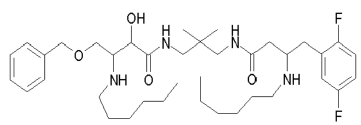 | | | | | | | | -9.83 | -6.49 |
| C19.005 | 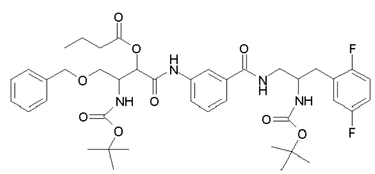 | no inhibition | no inhibition | no inhibition | 5.7000 | 11.5 | 3.65 | 10.67 | -7.33 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| **C19.006** | 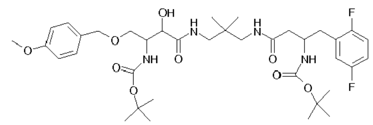 | no inhibition | no inhibition | no inhibition | 10.2000 | 6.2 | 24.13 | 18.53 | -8.43 | |
| **C19.007** | 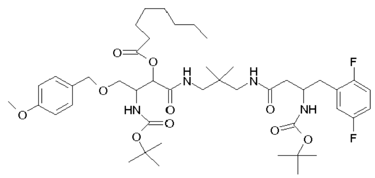 | no inhibition | no inhibition | no inhibition | no inhibition | 78.6 | 120.00 | 56.83 | -7.29 | |
| **C19.008** | 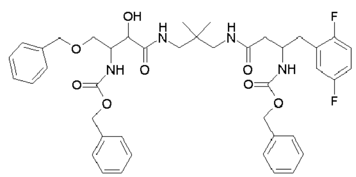 | no inhibition | no inhibition | no inhibition | 5.8000 | 5.7 | 6.77 | 8.60 | -8.69 | -6.29 |
| **C19.009** | 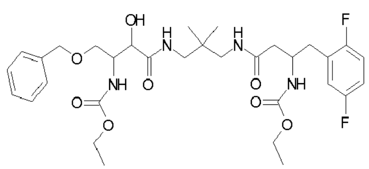 | no inhibitition | 5.9000 | no inhibitition | 3.4000 | 33.9 | 100.00 | 59.13 | -7.72 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| **C19.010** | 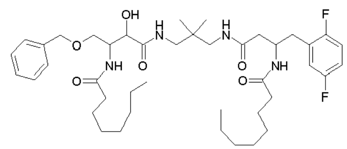 | no inhibitition | no inhibitition | no inhibitition | 3.5000 | 7.6 | 48.10 | 12.20 | -8.18 | |
| **C19.011** C19.011 | 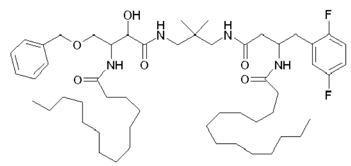 | no inhibitition | no inhibitition | no inhibitition | no inhibitition | 167.8 | 110.00 | 154.67 | n. d. | |
| **C19.012** | 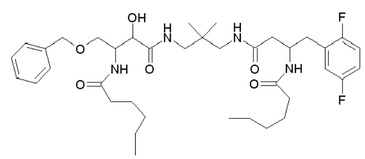 | no inhibitition | no inhibitition | no inhibitition | 0.5100 | 12.2 | 17.47 | 28.58 | -8.49 | |
| **C19.013** | 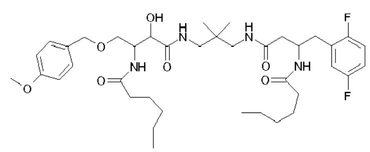 | no inhibitition | no inhibitition | no inhibitition | no inhibitition | 6.5 | 15.30 | 17.40 | -8.25 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C19.014 | 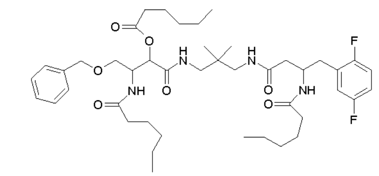 | no inhibitition | no inhibitition | no inhibitition | no inhibitition | 19.8 | 2.97 | 148.90 | -5.89 | |
| C19.015 | 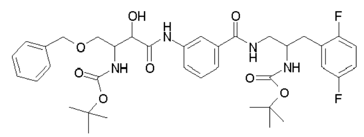 | no inhibitition | no inhibitition | no inhibitition | no inhibitition | 8.0 | 15.90 | 5.75 | -10.12 | |
| C19.016 | 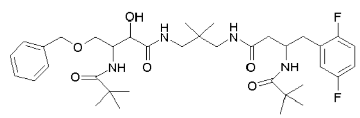 | no inhibitition | no inhibitition | no inhibitition | no inhibitition | 15.7 | 37.90 | 49.20 | -8.55 | |
| C19.017 | 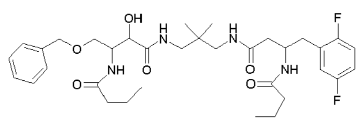 | no inhibitition | no inhibitition | no inhibitition | no inhibitition | 30.5 | 64.63 | 172.68 | -7.41 | |
| C19.018 | 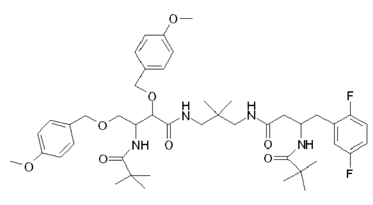 | no inhibitition | no inhibitition | no inhibitition | no inhibitition | 3.3 | 9.90 no inhibitition 3.3 9.90 9.10 -8.33 | 9.10 | -8.33 | -3.48 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| **C19.019** | 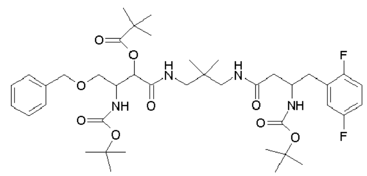 | no inhibitition | no inhibitition | no inhibitition | no inhibitition | 12.24 | 130.68 | 70.64 | -6.71 | |
| **C19.020** | 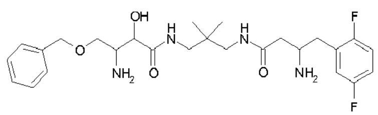 | no inhibitition | 0.1300 | 1.2000 | 0.0280 | 47.9 | 92.77 | 123.84 | -10.14 | |
| **C19.021** | 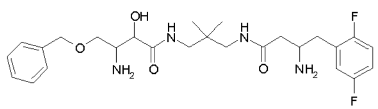 | no inhibition | 0.260 | 40210.0000 | 0.1400 | 58.8 | 68.60 | 105.65 | -9.66 | |
| **C19.022** | 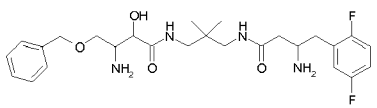 | no inhibition | 0.430 | 1.2000 | 0.3700 | 161.8 | 104.20 | 171.35 | -9.64 | |
| **C19.023** | 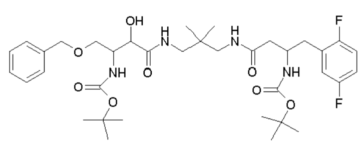 | no inhibition | no inhibition | no inhibition | no inhibition | 11.5 | 40.97 | 15.90 | n.d. | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol1) | $\Delta E_{CPBS}$ (APN)/ kcal/mol12) |
|---|---|---|---|---|---|---|---|---|---|---|
| C19.024 | 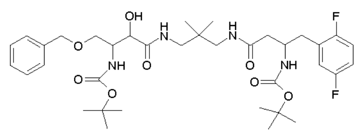 | no inhibition | no inhibition | no inhibition | no inhibition | 11.4 | 21.10 | 13.28 | n.d. | |
| C19.025 | 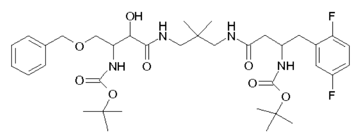 | no inhibition | no inhibition | no inhibition | no inhibition | 12.3 | 17.33 | 15.38 | n.d. | |
| C19.026 | 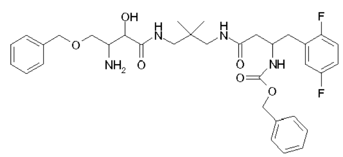 | no inhibition | 1.0000 | 7.3000 | 0.0500 | 12.2 | 8.90 | 22.73 | -10.76 | |
| C19.027 | 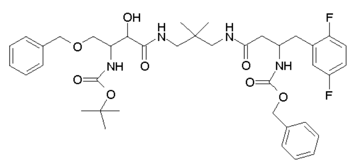 | no inhibition | no inhibition | no inhibition | no inhibition | 5.2 | 42.05 | 8.53 | -9.32 | |
| C19.028 | 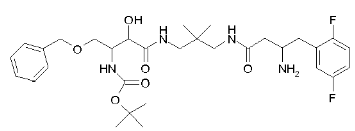 | 0.1700 | no inhibition | 1.4000 | no inhibition | 9.0 | 31.40 | 49.40 | -10.63 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| **C19.029** | 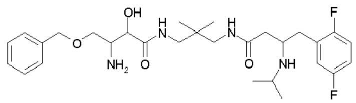 | 14.5000 | 0.5900 | 24.3000 | 0.3300 | 29.4 | 34.80 | 92.65 | -13.64 | |
| **C19.030** | 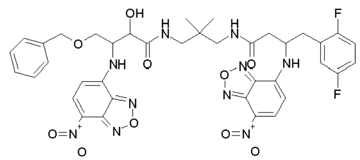 | no inhibition | no inhibition | no inhibition | no inhibition | 18.2 | 7.55 | 29.73 | -9.23 | |
| **C19.031** | 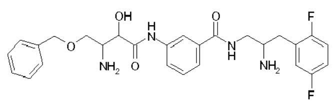 | 2.3000 | 0.0120 | 13.3000 | 0.1800 | 58.2 | 20.10 | 119.23 | -11.32 | |
| **C19_D01** | 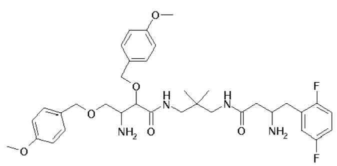 | | | | | | | | -11.87 | -8.11 |
| **C19_D02** | 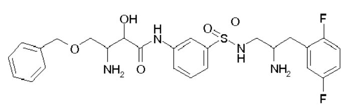 | | | | | | | | -13.64 | -8.46 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C20_A01 | 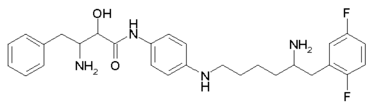 | | | | | | | | -9.03 | -8.58 |
| C20_A02 | 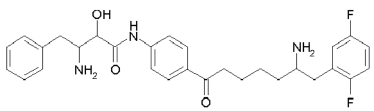 | | | | | | | | -10.08 | -8.50 |
| C20_A03 | 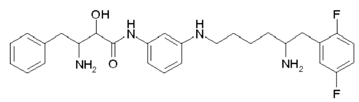 | | | | | | | | -10.53 | -8.45 |
| C20_A04 | 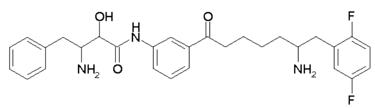 | | | | | | | | -10.67 | -9.62 |
| C20_A05 | 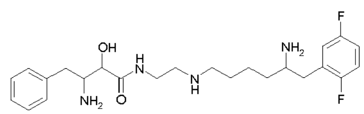 | | | | | | | | -11.67 | -7.63 |
| C20_A06 | 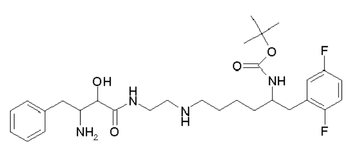 | | | | | | | | -11.34 | 7.64 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C20_A07 | 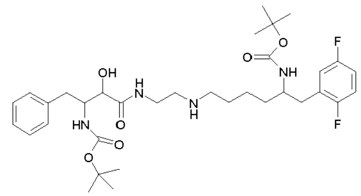 | | | | | | | | -9.16 | -6.84 |
| C21.001 | 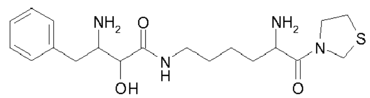 | 0.11 | 0.178 | 0.02 | 0.066 | 90.1 | 77.00 | 65.50 | -10.32 | |
| C21_A01 | 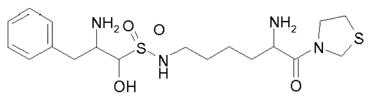 | | | | | | | | -15.72 | -7.37 |
| C21.002 | 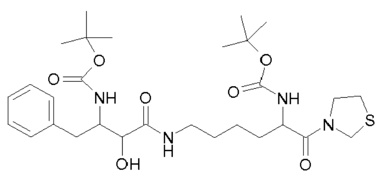 | no inhibition | no inhibition | no inhibition | no inhibition | 55.1 | 49.30 | 61.60 | -11.32 | |
| C21.003 | 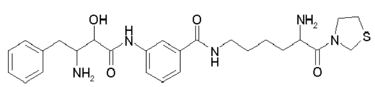 | 0.07 | 0.62 | 0.054 | 0.13 | 156.7 | 62.00 | 200.00 | -11.32 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C21.004 | 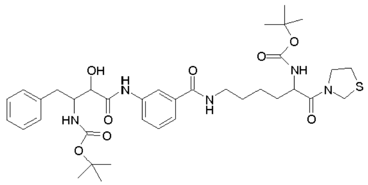 | no inhibition | no inhibition | no inhibition | no inhibition | 12.0 | 13.90 | 19.40 | n.d. | |
| C21.005 | 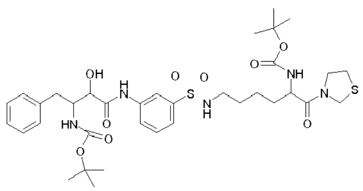 | no inhibition | no inhibition | no inhibition | no inhibition | 11.4 | 15.30 | | n.d. | |
| C21.006 | 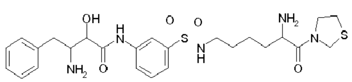 | 0.05 | 0.89 | 0.11 | 0.12 | 156.7 | 66.15 | | -16.45 | |
| C21_B1 | 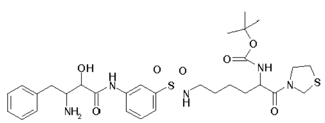 | | | | | | | | -8.62 | -9.76 |
| C21_B2 | 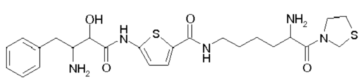 | | | | | | | | -10.28 | -9.29 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C21_B3 | 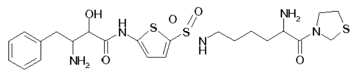 | | | | | | | | -11.77 | -10.35 |
| C22.001 | 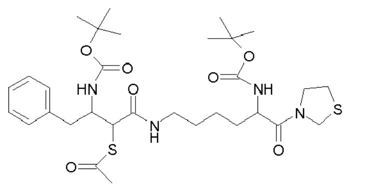 | no inhibition | no inhibition | no inhibition | 0.57 | 136.3 | 200.00 | 57.10 | n.d. | |
| C22.002 | 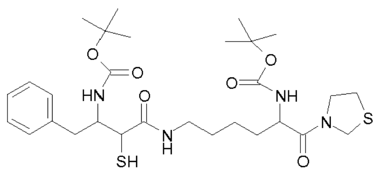 | no inhibition | no inhibition | no inhibition | 1 | 54.1 | 85.20 | 200.00 | n.d. | |
| C22.003 | 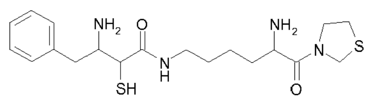 | 0.032 | 3.79 | 0.34 | 3.5 | 30.2 | 38.90 | 75.20 | -10.21 | |
| C22_B01 | 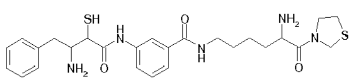 | | | | | | | | -10.00 | -10.67 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C22_B02 | 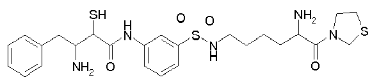 | | | | | | | | -11.63 | -10.90 |
| C26.001 | 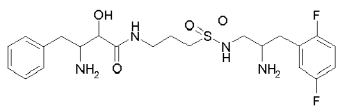 | 0.58 | 0.07 | 7.37 | 0.09 | 200.0 | 135.43 | | -10.91 | |
| C26_A01 | 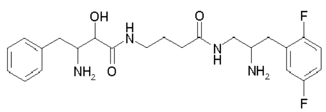 | | | | | | | | -10.50 | -7.77 |
| C26_B01 | 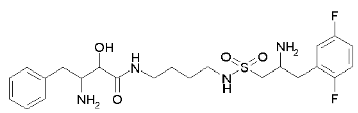 | | | | | | | | -10.50 | -9.77 |
| C26_B02 | 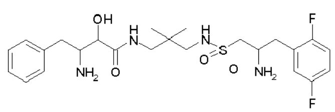 | | | | | | | | -14.14 | -9.74 |
| C26_B03 | 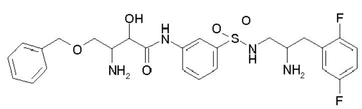 | | | | | | | | -14.08 | -9.92 |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| **C26.002** | 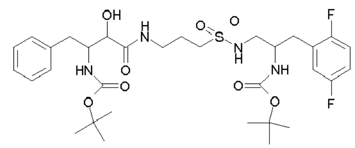 | 6.52 | no inhibition | no inhibition | no inhibition | 200.0 | 18.07 | | -8.57 | |
| **C26_C01** | 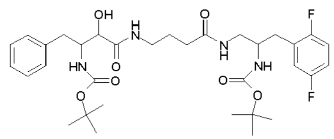 | | | | | | | | -9.68 | -6.74 |
| **C26.003** | 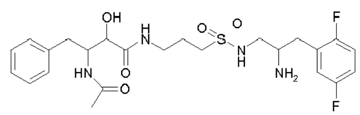 | 0.36 | 0.19 | no inhibition | 0.25 | 70.0 | 167.33 | | -10.37 | |
| **C26_C02** | 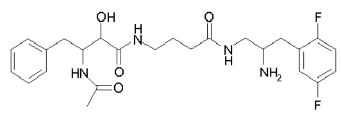 | | | | | | | | -10.91 | -6.22 |
| **C26.004** | 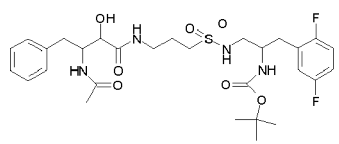 | no inhibition | no inhibition | no inhibition | no inhibition | 70.0 | 105.80 | | -9.57 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| **C26.005** | 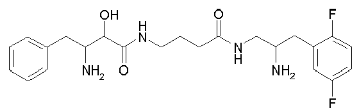 | no inhibition | 0.09 | not determined | 0.13 | 27.3 | 28.90 | | -10.50 | -7.45 |
| **C26.006** | 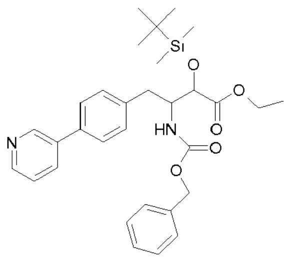 | no inhibition | no inhibition | not determined | no inhibition | 3.8 | 1.90 | 7.90 | -9.68 | -7.33 |
| **C26.007** | 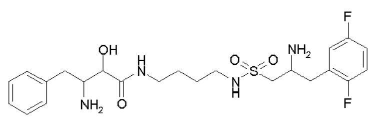 | no inhibition | no inhibition | | | 3.5 | | 7.3 | -10.50 | -5.13 |
| **C26.008** | 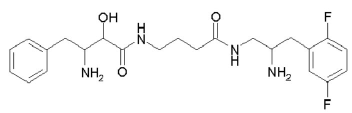 | 0.90 | 0.01 | not determined | not determined | | | | -10.50 | |

(continued)

| Compond | Structure | meas. IC50 (DPIV) /μM | meas. IC50 (APN) /μM | meas. IC50 (DP8/9) /μM | meas. IC50 (cAAP) /μM | Supp. DNA Synt. T-cell IC50 (μM) | Supp. DNA Synt. NHEK IC50 (μM) | Supp. DNA Synt. SZ95 IC50 (μM) | $\Delta E_{CPBS}$ (DPIV)/ kcal/mol[1)] | $\Delta E_{CPBS}$ (APN)/ kcal/mol[12)] |
|---|---|---|---|---|---|---|---|---|---|---|
| C26.009 | 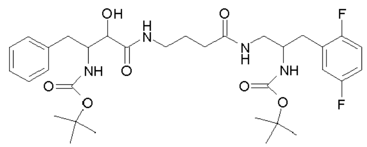 | no inhibition | no inhibition | not determined | not determined | | | | -9.68 | |
| C-122 | 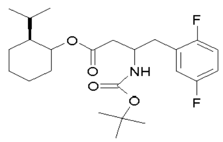 | no inhibition | no inhibition | no inhibition | no inhibition | 5.83 | 69.4 | 65.2 | -9.21 | -5.63 |
| C-125 | 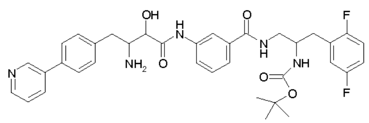 | no inhibition | 0.094 | not determined | 0.005 | 12.90 | 4.4 | 13.73 | -16.37 | -8.79 |

1) Free Energy of the interaction between the ligand and the respective central pore binding site

2) In case of missing data for the APN central pore binding site experimental values characterize the interaction.

**3.** The compounds as claimed in any of the claims 1 and 2 for use in the medical field.

**4.** The compounds as claimed in any of the claims 1 and 2 for use as dual inhibitors or central pore binding ligands of dipeptidyl peptidase IV and of peptidases with analogous enzymatic effect and of alanyl aminopeptidase N (APN) and of peptidases with analogous enzymatic effect, or for use as solitary inhibitors or central pore binding ligands of dipeptidyl peptidase IV and of peptidases with analogous enzymatic effect or of alanyl aminopeptidase N (APN) and of peptidases with analogous enzymatic effect.

**5.** The compounds as claimed in any of the claims 1 and 2 for use in the suppression of DNA synthesis and inflammatory cytokine production as well as in the stimulation of anti-inflammatory cytokine production in vitro and in vivo.

**6.** The compounds as claimed in any of the claims 1 and 2 for use for the prophylaxis and therapy of autoimmune diseases, of diseases with exceeding immune response and/or inflammatory genesis, including arteriosclerosis, neuronal diseases, cerebral damages, skin diseases, tumour diseases, transplant rejection, Graft-versus-Host Diseases (GvHD) and virus- or bacteria-caused diseases.

**7.** The compounds as claimed in claim 6, wherein the diseases or conditions are multiple sclerosis, morbus Crohn, colitis ulcerosa, diabetes mellitus Typ 1, rheumatoide arthritis, arteriosclerosis, arterial inflammation, stent-restenosis and other autoimmune diseases as well as inflammatory diseases.

**8.** The compounds as claimed in claim 6, wherein the diseases or conditions are tumours as well as metastases.

**9.** The compounds as claimed in claim 6, wherein the diseases or conditions are skin- and mucosa-related diseases, psoriasis, acne as well as dermatological diseases with hyper-proliferation and modified conditions of differentiation of fibroblasts, benign fibrosing and sclerosing skin diseases and malign fibroblastic conditions of hyper-proliferation.

**10.** The compounds as claimed in claim 6, wherein the diseases or conditions are asthma bronchiale and other allergic diseases as well as chronic obstructive pulmonary disease (COPD).

**11.** The compounds as claimed in claim 6, wherein the diseases or conditions are acute neuronal diseases, ischemia-caused cerebral damages after an ischemia- or haemorrhagic apoplexia, cranio-cerebral injury, cardiac arrest, heart attack or as a consequence of cardio surgical intervention, of chronic neuronal diseases for example of Morbus Alzheimer, of the Pick-disease, a progressive supra-nuclear palsy, the corticobasal degeneration, the frontotemporal dementia, of Morbus Parkinson, especially parkinsonism coupled to chromosome number 17, of Morbus Huntington, of prion-caused conditions or diseases and amyotrophic lateral sclerosis.

**12.** The compounds as claimed in claim 6, wherein the diseases or conditions are rejection of allogene or xenogene transplanted organs, tissues and cells such as bone marrow, kidney-, heart-, liver- pancreas-, skin- or stem cells, and stents, of joint implants (knee joint implants, hip joint implants). bone implants, cardiac pace makers or other implants, vessel balloons, as well as Graft-versus-Host Diseases (GvHD).

**13.** The compounds as claimed in claim 6, wherein the diseases or conditions are inflammatory infectious diseases such as malaria, severe acute respiratory syndrome (SARS), and of sepsis and sepsis-like conditions.

**14.** The compounds of the general formula (1) as claimed in any of claims 1 to 13, in combination with one or more pharmaceutically acceptable carrier(s), auxiliary substance(s) and/or adjuvant(s).

**15.** A pharmaceutical preparation comprising at least one of the compounds of the general formula (I) according to claim 1 or claim 2, optionally in combination with one or more pharmaceutically acceptable carrier(s), auxiliary substance(s) and/or adjuvant(s).

**16.** The pharmaceutical preparation as claimed in claim 15, further comprising at least one further pharmaceutically effective compound.

**17.** A cosmetic preparation comprising at least one of the compounds of the general formula (I) according to claim 1 or claim 2, optionally in combination with one or more cosmetically acceptable carrier(s), auxiliary substance(s) and/or adjuvant(s).

Figure 1

DPIV

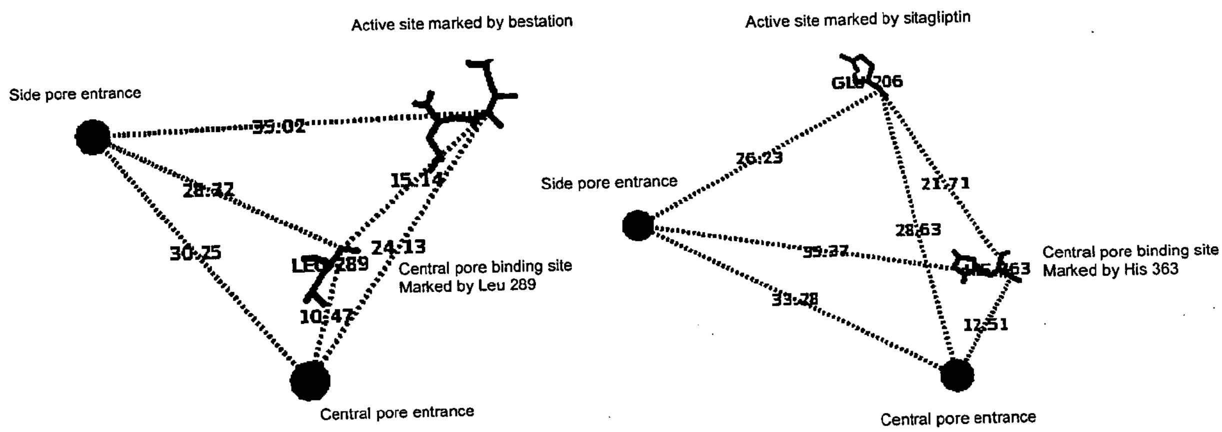


Essential distances (Ångström) in the monomers of APN and DPIV

## Figure 2

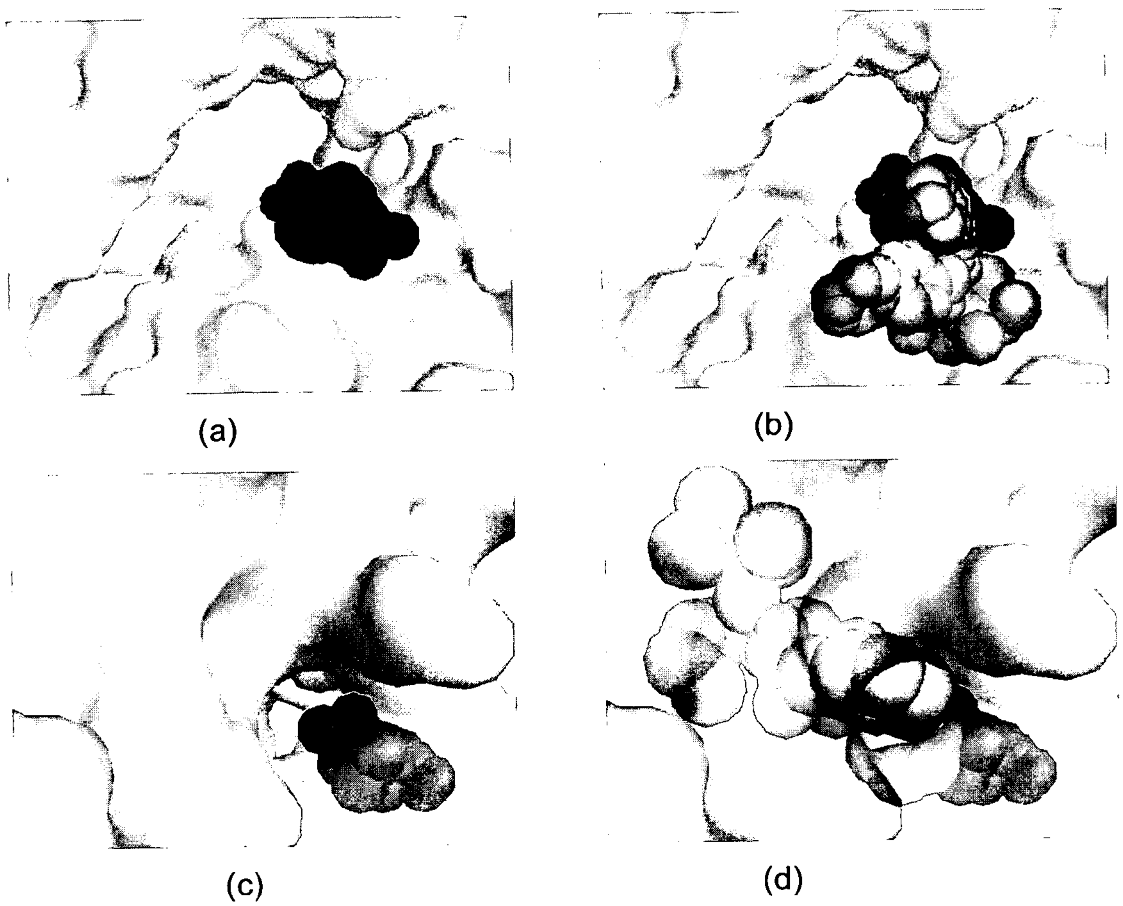

Europäisches Patentamt
European Patent Office
Office européen des brevets

# EUROPEAN SEARCH REPORT

Application Number
EP 10 17 1196

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | WO 2005/034940 A2 (IMTM GMBH [DE]; KEYNEUROTEK AG [DE] IMTM GMBH [DE]; KEYNEUROTEK AG [DE) 21 April 2005 (2005-04-21) * the whole document * ----- | 1 | INV. C07C237/08 C07D209/04 C07D277/04 C07D401/02 A61K31/16 A61K31/505 A61K31/404 A61K31/4164 A61P29/00 A61P37/00 A61P25/00 A61P35/00 |
| A,D | WO 2007/057128 A1 (IMTM GMBH [DE]; ANSORGE SIEGFRIED [DE]; NEUBERT KLAUS [DE]; BANK UTE [] 24 May 2007 (2007-05-24) * the whole document * ----- | 1 | |
| | | | TECHNICAL FIELDS SEARCHED (IPC) |
| | | | C07C C07D |

The present search report has been drawn up for all claims

2

| Place of search | Date of completion of the search | Examiner |
|---|---|---|
| Munich | 17 January 2011 | Götz, Gerhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 10 17 1196

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2011

| Patent document cited in search report | Publication date | Patent family member(s) | Publication date |
|---|---|---|---|
| WO 2005034940 A2 | 21-04-2005 | AU 2004280090 A1 | 21-04-2005 |
| | | CA 2542592 A1 | 21-04-2005 |
| | | CN 1882332 A | 20-12-2006 |
| | | DE 10348044 A1 | 19-05-2005 |
| | | EP 1673082 A2 | 28-06-2006 |
| | | EP 2105441 A1 | 30-09-2009 |
| | | JP 2007508350 T | 05-04-2007 |
| | | US 2007078130 A1 | 05-04-2007 |
| WO 2007057128 A1 | 24-05-2007 | AU 2006314813 A1 | 24-05-2007 |
| | | CA 2628145 A1 | 24-05-2007 |
| | | CN 101326171 A | 17-12-2008 |
| | | DE 102005054700 A1 | 05-07-2007 |
| | | EA 200801331 A1 | 27-02-2009 |
| | | EP 1948627 A1 | 30-07-2008 |
| | | JP 2009515915 T | 16-04-2009 |
| | | US 2009124667 A1 | 14-05-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 10156805 A
- WO 0189569 A
- WO 02053170 A
- EP 0307199 W
- WO 02053169 A
- DE 10337074
- DE 10330842
- EP 0302356 W
- DE 10102392
- DE 102006703942
- EP 1948627 A
- WO 2007057128 A
- EP 09169269 A


### Non-patent literature cited in the description


- **T. CHEN et al.** *Adv. Exp. Med. Biol.,* 2003, vol. 524, 79
- **J. S. DUKE-COHAN et al.** *J. Immunol.,* 1996, vol. 156, 1714
- **K. LTO et al.** *J. Biol. Chem.,* 2006, vol. 281, 33664-33676
- **A. J. BARRETT et al.** Handbook of Proteolytic Enzymes. Academic Press, 1998
- **H. B. RASMUSSEN et al.** *Nat. Struct. Biol.,* 2003, vol. 10, 3-5
- **LTO et al.** *J. Biol. Chem.,* 2006, vol. 281, 33664-33676
- **S. ANSORGE et al.** *Clin. Chem. Lab. Med.,* 2009, vol. 47, 253-261
- **U. LENDECKEL et al.** *Intern. J. Mol. Med.,* 1999, vol. 4, 17
- **T. KÄHNE et al.** *Intern. J. Mol. Med.,* 1999, vol. 4, 3
- **I. DE MEESTER et al.** *Advanc. Exp. Med. Biol.,* 2002, vol. 524, 3
- **D. P. KONTOYIANNIS et al.** *Lancet,* 2003, vol. 361, 1558
- **D. M. EVANS.** *Drugs,* 2002, vol. 5, 577
- **M.-C. FOURNIE-ZALUSKI ; B. P. ROQUES.** Ectopeptidases. Kluwer Academic/Plenum Publishers, 2002, 51
- **P. ANDERSON ; E. GONZALEZ-REY.** *Mol. Cell. Biol.,* 2010, vol. 30, 2537-2551
- **E. GONZALEZ-REY et al.** *Ann. Rheum. Dis.,* 2007, vol. 66, 70-76
- **J. HOLLER et al.** *J. Immunol.,* 2008, vol. 181, 6909-6912
- **J.-R. ZHOU et al.** *Neurosci. Bull.,* 2008, vol. 24, 155-159
- **K. ITO et al.** *J. Biol. Chem.,* 2006, vol. 281, 33664-33676
- Immunobiology. **C. JANAWAY et al.** Immunobiology. Garland Publishing, 2001, 565-566
- **S. ANSORGE et al.** *Clin. Chem.,* 2009, vol. 47, 253-261
- **T. KÄHNE et al.** *Int. J. Mol. Med.,* 1999, 3-15
- **U. LENDECKEL et al.** *Int. J. Mol. Med.,* 1999, 17-27
- **D. M. T. YU et al.** *FEBS J.,* 2010, 1-19
- **N. PETROVIC et al.** Aminopeptidases in biology and diseases. Kluwer Academic/Plenum Publishers, 2004, 179-200
- **R. PASQUALINI et al.** *Cancer Res.,* 2000, vol. 60, 722-727
- **R. RANGEL et al.** *Proc. Nat. Acad. Sci.,* 2007, vol. 104, 4588-4593
- **CC ZOUBOULIS ; H SELTMANN ; H NEITZEL ; CE ORFANOS.** Establishment and characterization of an immortalized human sebaceous gland cell line (SZ95. *J. Invest. Dermatol.,* 1999, vol. 113 (6), 1011-1020
- **A. KLAMT et al.** *Journal of the Chemical Society, Perkin Transaction,* 1993, vol. 2, 799
- Remington, The Science and Practice of Pharmacy. 2000
- **G.A. NOWAK.** Die kosmetischen Präparate, Band 2: Die kosmetischen Präparate - Rezepturen, Rohstoffe, wissenschaftliche Grundlagen. Verlag für Chemische Industrie H. Ziolkowsky KG, vol. 2